# EUROPEAN PATENT SPECIFICATION

(11) **EP 2 866 788 B1**
(45) Date of publication and mention of the grant of the patent: **16.12.2020**
(21) Application number: 13737879.0
(22) Date of filing: 26.06.2013
(51) Int. Cl.: A61K 9/00, A61K 47/02, A61K 47/12, A61K 9/08, A61K 31/4439, A61P 1/04

(54) **LIQUID PREPARATIONS OF AMINES AND ORGANIC ACIDS STABILIZED BY SALTS**
FLÜSSIGFORMULIERUNGEN VON SALZSTABILISIERTEN AMINEN UND ORGANISCHEN SÄUREN
PRÉPARATIONS LIQUIDES D'AMINES ET D'ACIDES ORGANIQUES STABILISÉS PAR DES SELS

(30) Priority: 27.06.2012 JP 2012144750
(43) Date of publication of application: 06.05.2015
(73) Proprietor: Takeda Pharmaceutical Company Limited, Osaka-shi, Osaka 541-0045 (JP)
(72) Inventor: IKEDA, Megumi, Yaizu-shi Shizuoka 425-0072 (JP); HORIUCHI, Shohei, Osaka-shi, Osaka 532-0024 (JP); SATO, Tomomi, Osaka-shi Osaka 532-0024 (JP); NAKAI, Shinichiro, Osaka-shi Osaka 532-0024 (JP); KIYOSHIMA, Kenichiro, Osaka-shi Osaka 532-0024 (JP)
(74) Representative: Jones, Nicholas Andrew
(86) International application number: PCT/JP2013/068192
(87) International publication number: WO 2014/003199

(56) References cited:
- WO-A1-00/04902
- WO-A1-02/41919
- WO-A1-2007/026916
- WO-A2-2007/022165
- WO-A2-2010/013823
- CA-A1- 2 473 412
- US-A1- 2009 042 932
- US-B1- 6 310 094
- HARUYUKI NISHIDA ET AL: "Discovery, synthesis, and biological evaluation of novel pyrrole derivatives as highly selective potassium-competitive acid blockers", BIOORGANIC & MEDICINAL CHEMISTRY, vol. 20, no. 12, 1 June 2012 (2012-06-01), pages 3925-3938, XP055080139, ISSN: 0968-0896, DOI: 10.1016/j.bmc.2012.04.014

## Description

### Technical field of the invention

The present invention relates to a stabilized liquid preparation comprising a pharmaceutically active ingredient as defined in claim 1 having a primary or secondary amino group, an organic acid and a salt as defined here, a freeze-dried preparation obtained by freeze-drying the liquid preparation and a stabilizing method.

### (Background of the Invention)

A "pharmaceutically active ingredient having a primary or secondary amino group" is widely used as a pharmaceutically active ingredient for various diseases. For example, patent document 1 describes a compound represented by the following formula or a salt thereof as an agent for the treatment or prophylaxis of peptic ulcer, gastritis, erosive esophagitis and the like. 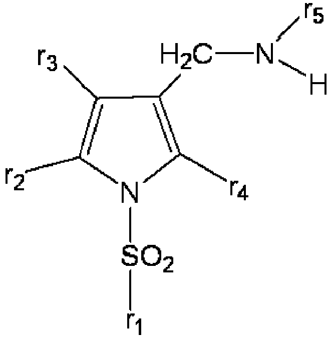 wherein r₁ is a monocyclic nitrogen-containing heterocyclic group optionally condensed with a benzene ring or a heterocycle, the monocyclic nitrogen-containing heterocyclic group optionally condensed with a benzene ring or a heterocycle optionally has substituent(s), r₂ is an optionally substituted C₆₋₁₄ aryl group, an optionally substituted thienyl group or an optionally substituted pyridyl group, r₃ and r₄ are each a hydrogen atom, or one of r₃ and r₄ is a hydrogen atom and the other is an optionally substituted lower alkyl group, an acyl group, a halogen atom, a cyano group or a nitro group, and r₅ is an alkyl group.

Patent document 2 describes a stabilized pharmaceutical composition comprising a nonpeptidic pharmaceutically active ingredient having a primary or secondary amino group, an excipient and an acidic compound, and the like.
patent document 1: WO2007-026916
patent document 2: WO2010-013823

### Disclosure of the Invention

### Problems to be Solved by the Invention

An object of the present invention is to provide a stabilized pharmaceutical composition to use a pharmaceutically active ingredient having a primary or secondary amino group particularly as an active ingredient of a liquid preparation, a stabilizing method and the like.

### Means of Solving the Problems

When a compound showing a superior pharmacological activity is examined as a pharmaceutically active ingredient, an organic acid salt compound that forms a salt with an organic acid is sometimes selected in consideration of the stability, solubility, crystallization and the like of the compound as a solid (powder, crystal etc.). It is known that a pharmaceutically active ingredient having a primary or secondary amino group, for example, the below-mentioned compound A, is converted to an organic acid salt (e.g., fumarate) to stabilize the solid. However, the present inventors have found that a liquid preparation containing the salt as a material is associated with a problem of impaired stability of compound A due to an organic acid liberated into the liquid.

When a liquid preparation is produced from an organic acid salt compound of a pharmaceutically active ingredient having a primary or secondary amino group as a material, the organic acid liberated from the organic acid salt compound of the pharmaceutically active ingredient into the liquid and the primary or secondary amino group of the pharmaceutically active ingredient undergo a covalent bond reaction and produce an adduct as an analogue. Therefore, the present inventors have conducted intensive studies in an attempt to stabilize a liquid preparation containing a pharmaceutically active ingredient having a primary or secondary amino group and an organic acid, and found for the first time that a liquid preparation showing more excellent stability can be obtained by further adding a salt to a pharmaceutical composition containing a pharmaceutically active ingredient having a primary or secondary amino group and an organic acid, which resulted in the completion of the present invention.

That is, the present invention relates to the following.
[1] A liquid preparation comprising a pharmaceutically active ingredient, an organic acid and a salt,
   wherein a content of a reaction product of the pharmaceutically active ingredient and the organic acid in the preparation is not more than 5%,
   wherein the pharmaceutically active ingredient is 1-{5-(2-fluorophenyl)-1-[(6-methylpyridin-3-yl)sulfonyl]-1H-pyrrol-3-yl}-N-methylmethanamine, 1-[4-fluoro-5-phenyl-1-(pyridin-3-ylsulfonyl)-1H-pyrrol-3-yl]-N-methylmethanamine, N-methyl-1-[5-(4-methyl-3-thienyl)-1-(pyridin-3-ylsulfonyl)-1H-pyrrol-3-yl]methanamine, 1-[5-(2-fluorophenyl)-1-(pyridin-3-ylsulfonyl)-1H-pyrrol-3-yl]-N-methylmethanamine,
   N-methyl-1-[5-(2-methylphenyl)-1-(pyridin-3-ylsulfonyl)-1H-pyrrol-3-yl]methanamine,
   1-{4-fluoro-5-(2-fluoropyridin-3-yl)-1-[(4-methylpyridin-2-yl)sulfonyl]-1H-pyrrol-3-yl}-N-methylmethanamine, or 1-[4-fluoro-5-(2-fluoropyridin-3-yl)-1-(pyridin-3-ylsulfonyl)-1H-pyrrol-3-yl]-N-methylmethanamine,
   wherein the organic acid is a compound represented by the formula (IV): 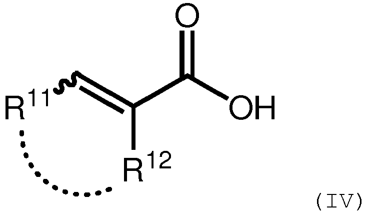 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
   wherein the salt is one or more kinds selected from the group consisting of sodium chloride, calcium chloride, magnesium chloride, sodium bromide and calcium bromide,
   wherein the reaction product is a compound represented by the formula (V) or (V') : 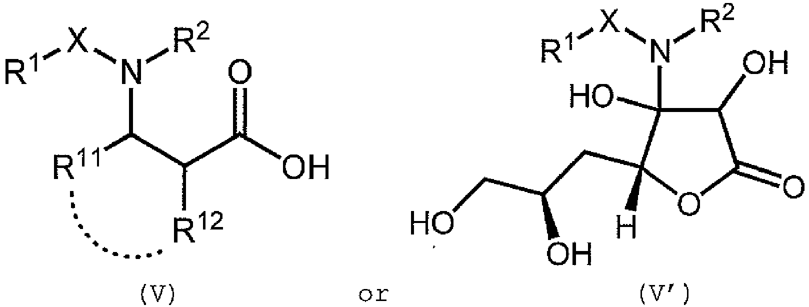 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.
[2] The liquid preparation according to the above mentioned [1], which is a solution for injection.
[3] The liquid preparation according to the above mentioned [1], comprising the reaction product of the pharmaceutically active ingredient and the organic acid at not more than 1.8-fold % after storage at 70°C for 1 week than before the storage.
[4] The liquid preparation according to the above mentioned [1], comprising the reaction product of the pharmaceutically active ingredient and the organic acid at not more than 1.3-fold % after storage at 60°C for 1 week than before the storage.
[5] A method of producing the liquid preparation according to the above mentioned [1], comprising a step of dissolving or suspending an organic acid salt of the pharmaceutically active ingredient, and the salt in a solvent.
[6] The liquid preparation according to the above mentioned [1], wherein the organic acid is one or more kinds selected from the group consisting of ascorbic acid, benzoic acid, sorbic acid, fumaric acid and maleic acid.
[7] The liquid preparation according to the above mentioned [1], wherein the pH is a physiologically acceptable pH.
[8] The liquid preparation according to the above mentioned [1], wherein the pH is about 3.0 to about 5.0.
[9] The liquid preparation according to the above mentioned [1], wherein the pharmaceutically active ingredient and the organic acid are contained at a molar ratio of 1:0.001 - 1:1000.
[10] The liquid preparation according to the above mentioned [1], wherein the pharmaceutically active ingredient and the salt are contained at a molar ratio of 1:0.001 - 1:10000.
[11] The liquid preparation according to the above mentioned [1], wherein the pharmaceutically active ingredient has a concentration of 0.1 - 100 mg/mL.
[12] The liquid preparation according to the above mentioned [1], for use in the prophylaxis or treatment of gastric ulcer accompanied by bleeding, duodenal ulcer, acute stress ulcer or acute stomach mucosal lesion.
[13] A freeze-dried preparation obtained by freeze-drying the liquid preparation according to the above mentioned [1].
[14] An injection kit comprising the solution for injection according to the above mentioned [2] and an infusion in combination.
[15] An injection kit comprising the freeze-dry preparation according to the above mentioned [13] and an infusion in combination.
[16] A method of stabilizing a liquid preparation, comprising adding a salt to a composition containing a pharmaceutically active ingredient and an organic acid,
   wherein the pharmaceutically active ingredient is 1-{5-(2-fluorophenyl)-1-[(6-methylpyridin-3-yl)sulfonyl]-1H-pyrrol-3-yl}-N-methylmethanamine,
   1-[4-fluoro-5-phenyl-1-(pyridin-3-ylsulfonyl)-1H-pyrrol-3-yl]-N-methylmethanamine,
   N-methyl-1-[5-(4-methyl-3-thienyl)-1-(pyridin-3-ylsulfonyl)-1H-pyrrol-3-yl]methanamine,
   1-[5-(2-fluorophenyl)-1-(pyridin-3-ylsulfonyl)-1H-pyrrol-3-yl]-N-methylmethanamine,
   N-methyl-1-[5-(2-methylphenyl)-1-(pyridin-3-ylsulfonyl)-1H-pyrrol-3-yl]methanamine,
   1-{4-fluoro-5-(2-fluoropyridin-3-yl)-1-[(4-methylpyridin-2-yl)sulfonyl]-1H-pyrrol-3-yl}-N-methylmethanamine, or
   1-[4-fluoro-5-(2-fluoropyridin-3-yl)-1-(pyridin-3-ylsulfonyl)-1H-pyrrol-3-yl]-N-methylmethanamine,
   wherein the organic acid is a compound represented by the formula (IV): 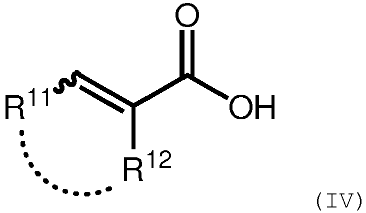 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
   wherein the salt is one or more kinds selected from the group consisting of sodium chloride, calcium chloride, magnesium chloride, sodium bromide and calcium bromide.
[17] A method of suppressing the production of a reaction product of a pharmaceutically active ingredient and an organic acid, comprising adding a salt to a composition containing the pharmaceutically active ingredient and the organic acid,
   wherein the pharmaceutically active ingredient is
   1-{5-(2-fluorophenyl)-1-[(6-methylpyridin-3-yl)sulfonyl]-1H-pyrrol-3-yl}-N-methylmethanamine,
   1-[4-fluoro-5-phenyl-1-(pyridin-3-ylsulfonyl)-1H-pyrrol-3-yl]-N-methylmethanamine,
   N-methyl-1-[5-(4-methyl-3-thienyl)-1-(pyridin-3-ylsulfonyl)-1H-pyrrol-3-yl]methanamine,
   1-[5-(2-fluorophenyl)-1-(pyridin-3-ylsulfonyl)-1H-pyrrol-3-yl]-N-methylmethanamine,
   N-methyl-1-[5-(2-methylphenyl)-1-(pyridin-3-ylsulfonyl)-1H-pyrrol-3-yl]methanamine,
   1-{4-fluoro-5-(2-fluoropyridin-3-yl)-1-[(4-methylpyridin-2-yl)sulfonyl]-1H-pyrrol-3-yl}-N-methylmethanamine, or
   1-[4-fluoro-5-(2-fluoropyridin-3-yl)-1-(pyridin-3-ylsulfonyl)-1H-pyrrol-3-yl]-N-methylmethanamine,
   wherein the organic acid is a compound represented by the formula (IV): 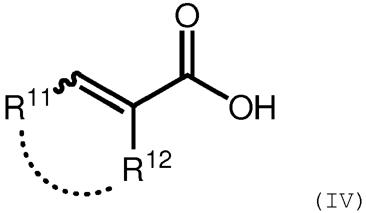 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
   wherein the salt is one or more kinds selected from the group consisting of sodium chloride, calcium chloride, magnesium chloride, sodium bromide and calcium bromide,
   wherein the reaction product is a compound represented by the formula (V) or (V'): 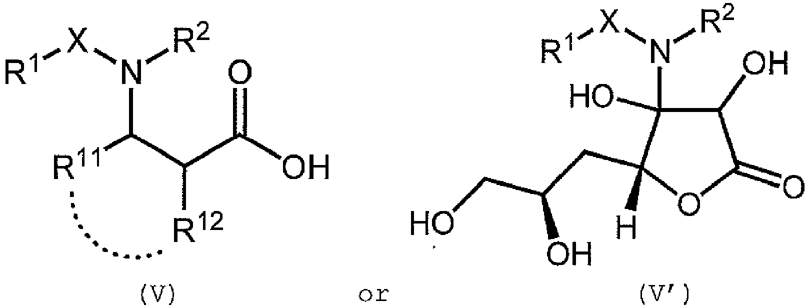 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.
[18] Use of a salt as a stabilizer in a liquid preparation comprising a pharmaceutically active ingredient and an organic acid,
   wherein a content of a reaction product of the pharmaceutically active ingredient and the organic acid in the preparation is not more than 5%,
   wherein the pharmaceutically active ingredient is 1-{5-(2-fluorophenyl)-1-[(6-methylpyridin-3-yl)sulfonyl]-1H-pyrrol-3-yl}-N-methylmethanamine,
   1-[4-fluoro-5-phenyl-1-(pyridin-3-ylsulfonyl)-1H-pyrrol-3-yl]-N-methylmethanamine,
   N-methyl-1-[5-(4-methyl-3-thienyl)-1-(pyridin-3-ylsulfonyl)-1H-pyrrol-3-yl]methanamine,
   1-[5-(2-fluorophenyl)-1-(pyridin-3-ylsulfonyl)-1H-pyrrol-3-yl]-N-methylmethanamine,
   N-methyl-1-[5-(2-methylphenyl)-1-(pyridin-3-ylsulfonyl)-1H-pyrrol-3-yl]methanamine,
   1-{4-fluoro-5-(2-fluoropyridin-3-yl)-1-[(4-methylpyridin-2-yl)sulfonyl]-1H-pyrrol-3-yl}-N-methylmethanamine, or
   1-[4-fluoro-5-(2-fluoropyridin-3-yl)-1-(pyridin-3-ylsulfonyl)-1H-pyrrol-3-yl]-N-methylmethanamine,
   wherein the organic acid is a compound represented by the formula (IV): 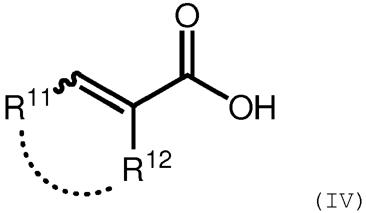 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
   wherein the salt is one or more kinds selected from the group consisting of sodium chloride, calcium chloride, magnesium chloride, sodium bromide and calcium bromide,
   wherein the reaction product is a compound represented by the formula (V) or (V'): 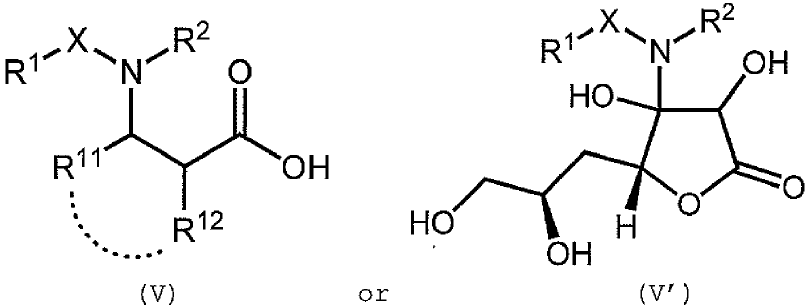 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.
[19] Use of a salt for the production of a stabilized liquid preparation comprising a pharmaceutically active ingredient and an organic acid,
   wherein a content of a reaction product of the pharmaceutically active ingredient and the organic acid in the preparation is not more than 5%,
   wherein the pharmaceutically active ingredient is 1-{5-(2-fluorophenyl)-1-[(6-methylpyridin-3-yl)sulfonyl]-1H-pyrrol-3-yl}-N-methylmethanamine,
   1-[4-fluoro-5-phenyl-1-(pyridin-3-ylsulfonyl)-1H-pyrrol-3-yl]-N-methylmethanamine,
   N-methyl-1-[5-(4-methyl-3-thienyl)-1-(pyridin-3-ylsulfonyl)-1H-pyrrol-3-yl]methanamine,
   1-[5-(2-fluorophenyl)-1-(pyridin-3-ylsulfonyl)-1H-pyrrol-3-yl]-N-methylmethanamine,
   N-methyl-1-[5-(2-methylphenyl)-1-(pyridin-3-ylsulfonyl)-1H-pyrrol-3-yl]methanamine,
   1-{4-fluoro-5-(2-fluoropyridin-3-yl)-1-[(4-methylpyridin-2-yl)sulfonyl]-1H-pyrrol-3-yl}-N-methylmethanamine, or 1-[4-fluoro-5-(2-fluoropyridin-3-yl)-1-(pyridin-3-ylsulfonyl)-1H-pyrrol-3-yl]-N-methylmethanamine,
   wherein the organic acid is a compound represented by the formula (IV): 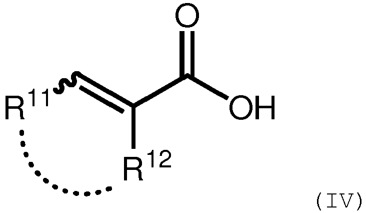 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
   wherein the salt is one or more kinds selected from the group consisting of sodium chloride, calcium chloride, magnesium chloride, sodium bromide and calcium bromide,
   wherein the reaction product is a compound represented by the formula (V) or (V'): 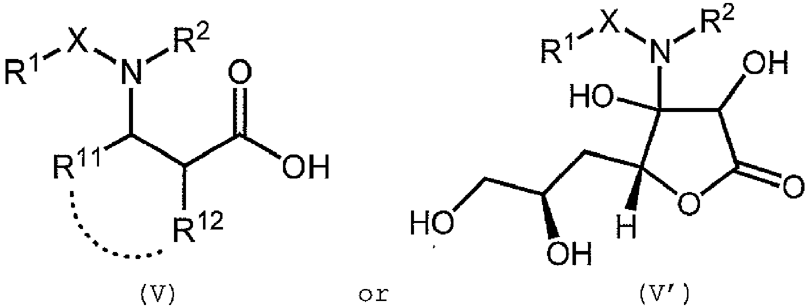 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.
[20] A liquid preparation produced from an organic acid salt compound of a pharmaceutically active ingredient, and a salt as starting materials, wherein the amount of a reaction product of the pharmaceutically active ingredient and the liberated organic acid is suppressed by the salt,
   wherein the pharmaceutically active ingredient is 1-{5-(2-fluorophenyl)-1-[(6-methylpyridin-3-yl)sulfonyl]-1H-pyrrol-3-yl}-N-methylmethanamine,
   1-[4-fluoro-5-phenyl-1-(pyridin-3-ylsulfonyl)-1H-pyrrol-3-yl]-N-methylmethanamine,
   N-methyl-1-[5-(4-methyl-3-thienyl)-1-(pyridin-3-ylsulfonyl)-1H-pyrrol-3-yl]methanamine,
   1-[5-(2-fluorophenyl)-1-(pyridin-3-ylsulfonyl)-1H-pyrrol-3-yl]-N-methylmethanamine,
   N-methyl-1-[5-(2-methylphenyl)-1-(pyridin-3-ylsulfonyl)-1H-pyrrol-3-yl]methanamine,
   1-{4-fluoro-5-(2-fluoropyridin-3-yl)-1-[(4-methylpyridin-2-yl)sulfonyl]-1H-pyrrol-3-yl}-N-methylmethanamine, or 1-[4-fluoro-5-(2-fluoropyridin-3-yl)-1-(pyridin-3-ylsulfonyl)-1H-pyrrol-3-yl]-N-methylmethanamine,
   wherein the organic acid is a compound represented by the formula (IV): 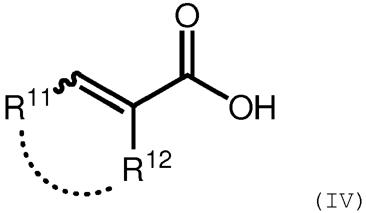 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
   wherein the salt is one or more kinds selected from the group consisting of sodium chloride, calcium chloride, magnesium chloride, sodium bromide and calcium bromide,
   wherein the reaction product is a compound represented by the formula (V) or (V'): 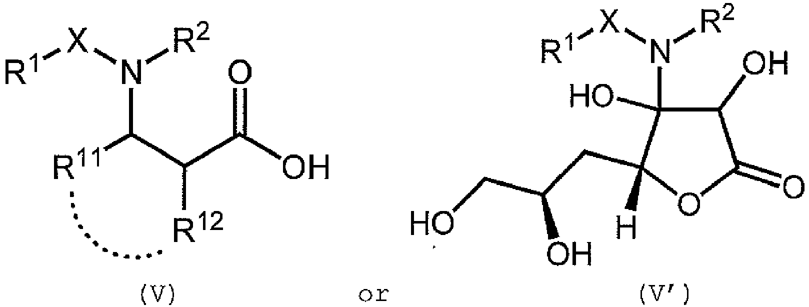 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.

### Effect of the Invention

According to the present invention, a stabilized liquid preparation containing a pharmaceutically active ingredient having a primary or secondary amino group as defined above, are provided. Specifically, since production of a reaction product of a pharmaceutically active ingredient having a primary or secondary amino group as defined above and an organic acid as defined above can be suppressed by adding a salt as defined above to a liquid preparation containing the pharmaceutically active ingredient and the organic acid, a liquid preparation showing more excellent stability and safe as a medicament can be provided. According to the present invention, moreover, since production of the reaction product can be suppressed, a liquid preparation having excellent preservation stability and the like can be provided, and a liquid preparation wherein the amount of the reaction product is suppressed by the salt as defined above can be provided. It has not been known that a salt as defined above has new use of suppression of the production of a reaction product of a pharmaceutically active ingredient having a primary or secondary amino group as defined above and an organic acid as defined above in a liquid preparation, namely, a stabilizing action on a liquid preparation containing a pharmaceutically active ingredient having a primary or secondary amino group as defined above and an organic acid as defined above. Furthermore, since the liquid preparation of the present invention is controlled to have an appropriate pH to lower the stimulation to the skin, the vein or the vicinity thereof, a pain caused to patients when administered as an injection can be suppressed.

The present invention is explained in detail in the following.

### [1. Pharmaceutically active ingredient having a primary or secondary amino group as defined above (first component)]

The "pharmaceutically active ingredient having a primary or secondary amino group", which is the first component of the liquid preparation of the present invention, is, 1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or 1-[4-fluoro-5-(2-fluoropyridin-3-yl)-1-(pyridin-3-ylsulfonyl)-1H-pyrrol-3-yl]-N-methylmethanamine.

When used herein R^{a} is an organic residue and R^{b} is a hydrogen atom or an organic residue. The "organic residue" for R^{a} or R^{b} is a monovalent group having 1 to 700 carbon atoms, and may contain, besides carbon atom, a hydrogen atom, a nitrogen atom, an oxygen atom, a sulfur atom or a halogen atom (e.g., fluorine atom, chlorine atom, bromine atom, iodine atom etc.) and the like. The "organic residue" means, for example, a hydrocarbon group optionally having substituent(s). Here, examples of the "hydrocarbon group" of the "hydrocarbon group optionally having substituent(s)" include chain or cyclic hydrocarbon groups (e.g., alkyl, alkenyl, alkynyl, cycloalkyl, aryl, aralkyl etc.). Of these, a chain or cyclic hydrocarbon group having 1 to 16 carbon atoms and the like are preferable.

Examples of the "alkyl" include C₁₋₆ alkyl (e.g., methyl, ethyl, propyl, isopropyl, butyl, isobutyl, sec-butyl, tert-butyl, pentyl, hexyl etc.) and the like.

Examples of the "alkenyl" include C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl etc.) and the like.

Examples of the "alkynyl" include C₂₋₆ alkynyl (e.g., ethynyl, propargyl, 1-butynyl, 2-butynyl, 3-butynyl, 1-hexynyl etc.) and the like.

Examples of the "cycloalkyl" include C₃₋₇ cycloalkyl (e.g., cyclopropyl, cyclobutyl, cyclopentyl, cyclohexyl, cycloheptyl etc.) and the like.

Examples of the "aryl" include C₆₋₁₄ aryl (e.g., phenyl, 1-naphthyl, 2-naphthyl, 2-biphenylyl, 3-biphenylyl, 4-biphenylyl, 2-anthryl etc.) and the like.

Examples of the "aralkyl" include C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, phenyl-C₁₋₆ alkyl such as 5-phenylpentyl etc., naphthyl-C₁₋₆ alkyl, diphenyl-C₁₋₄ alkyl etc.) and the like.

When the above-mentioned hydrocarbon group is alkyl, alkenyl or alkynyl, it is optionally substituted by 1 to 3 substituents selected from (1) a halogen atom (e.g., fluorine atom, chlorine atom, bromine atom, iodine atom etc.), (2) nitro, (3) cyano, (4) hydroxy, (5) C₁₋₆ alkoxy (e.g., methoxy, ethoxy, propoxy, isopropoxy, butoxy, isobutoxy, sec-butoxy, pentyloxy, hexyloxy, fluoromethoxy etc.) optionally having 1 to 3 halogen atoms (e.g., fluorine atom, chlorine atom, bromine atom, iodine atom), (6) C₆₋₁₄ aryloxy (e.g., phenyloxy, naphthyloxy etc.), (7) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.), (8) mercapto, (9) C₁₋₆ alkylthio (e.g., methylthio, difluoromethylthio, trifluoromethylthio, ethylthio, propylthio, isopropylthio, butylthio, 4,4,4-trifluorobutylthio, pentylthio, hexylthio etc.) optionally having 1 to 3 halogen atoms (e.g., fluorine atom, chlorine atom, bromine atom, iodine atom), (10) C₆₋₁₄ arylthio (e.g., phenylthio, naphthylthio etc.), (11) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.) (12) amino, (13) mono-C₁₋₆ alkylamino (e.g., methylamino, ethylamino etc.), (14) mono-C₆₋₁₄ arylamino (e.g., phenylamino, 1-naphthylamino, 2-naphthylamino etc.), (15) mono-C₇₋₁₆ aralkylamino (e.g., benzylamino etc.), (16) di-C₁₋₆ alkylamino (e.g., dimethylamino, diethylamino etc.), (17) di-C₆₋₁₄ arylamino (e.g., diphenylamino etc.), (18) di-C₇₋₁₆ aralkylamino (e.g., dibenzylamino etc.), (19) formyl, (20) C₁₋₆ alkyl-carbonyl (e.g., acetyl, propionyl etc.), (21) C₆₋₁₄ arylcarbonyl (e.g., benzoyl, 1-naphthoyl, 2-naphthoyl etc.), (22) carboxyl, (23) C₁₋₆ alkoxy-carbonyl (e.g., methoxycarbonyl, ethoxycarbonyl, propoxycarbonyl, tert-butoxycarbonyl etc.), (24) C₆₋₁₄ aryloxy-carbonyl (e.g., phenoxycarbonyl etc.), (25) carbamoyl, (26) thiocarbamoyl, (27) mono-C₁₋₆ alkyl-carbamoyl (e.g., methylcarbamoyl, ethylcarbamoyl etc.), (28) di-C₁₋₆ alkyl-carbamoyl (e.g., dimethylcarbamoyl, diethylcarbamoyl, ethylmethylcarbamoyl etc.), (29) C₆₋₁₄ aryl-carbamoyl (e.g., phenylcarbamoyl, 1-naphthylcarbamoyl, 2-naphthylcarbamoyl etc.), (30) C₁₋₆ alkylsulfonyl (e.g., methylsulfonyl, ethylsulfonyl etc.), (31) C₆₋₁₄ arylsulfonyl (e.g., phenylsulfonyl, 1-naphthylsulfonyl, 2-naphthylsulfonyl etc.), (32) C₁₋₆ alkylsulfinyl (e.g., methylsulfinyl, ethylsulfinyl etc.), (33) C₆₋₁₄ arylsulfinyl (e.g., phenylsulfinyl, 1-naphthylsulfinyl, 2-naphthylsulfinyl etc.), (34) formylamino, (35) C₁₋₆ alkylcarbonylamino (e.g., acetylamino etc.), (36) C₆₋₁₄ arylcarbonylamino (e.g., benzoylamino, naphthoylamino etc.), (37) C₁₋₆ alkoxy-carbonylamino (e.g., methoxycarbonylamino, ethoxycarbonylamino, propoxycarbonylamino, butoxycarbonylamino etc.), (38) C₁₋₆ alkylsulfonylamino (e.g., methylsulfonylamino, ethylsulfonylamino etc.), (39) C₆₋₁₄ arylsulfonylamino (e.g., phenylsulfonylamino, 2-naphthylsulfonylamino, 1-naphthylsulfonylamino etc.), (40) C₁₋₆ alkyl-carbonyloxy (e.g., acetoxy, propionyloxy etc.), (41) C₆₋₁₄ aryl-carbonyloxy (e.g., benzoyloxy, naphthylcarbonyloxy etc.), (42) C₁₋₆ alkoxy-carbonyloxy (e.g., methoxycarbonyloxy, ethoxycarbonyloxy, propoxycarbonyloxy, butoxycarbonyloxy etc.), (43) mono-C₁₋₆ alkyl-carbamoyloxy (e.g., methylcarbamoyloxy, ethylcarbamoyloxy etc.), (44) di-C₁₋₆ alkyl-carbamoyloxy (e.g., dimethylcarbamoyloxy, diethylcarbamoyloxy etc.), (45) C₆₋₁₄ aryl-carbamoyloxy (e.g., phenylcarbamoyloxy, naphthylcarbamoyloxy etc.), (46) a 5- to 7-membered saturated cyclic amino optionally containing, besides one nitrogen atom and carbon atom, one or two kinds of 1 to 4 hetero atoms selected from a nitrogen atom, a sulfur atom and an oxygen atom (e.g., pyrrolidin-1-yl, piperidino, piperazin-1-yl, morpholino, thiomorpholino, hexahydroazepin-1-yl etc.), (47) a 5- to 10-membered aromatic heterocyclic group containing, besides carbon atom, one or two kinds of 1 to 4 hetero atoms selected from a nitrogen atom, a sulfur atom and an oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.), (48) C₁₋₃ alkylenedioxy (e.g., methylenedioxy, ethylenedioxy etc.), and (49) C₃₋₇ cycloalkyl (e.g., cyclopropyl, cyclobutyl, cyclopentyl, cyclohexyl, cycloheptyl etc.) (hereinafter to be referred to as substituent group A) and the like. These substituents may have 1 to 4 substituents at substitutable position(s). Examples of such substituents include those similar to the substituents in substituent group A.

When the above-mentioned hydrocarbon group is cycloalkyl, aryl or aralkyl, it is optionally substituted by 1 to 5 (preferably 1 to 3) substituents selected from (1) a halogen atom (e.g., fluorine atom, chlorine atom, bromine atom, iodine atom etc.), (2) nitro, (3) cyano, (4) hydroxy, (5) C₁₋₆ alkoxy optionally having 1 to 3 halogen atoms (e.g., fluorine atom, chlorine atom, bromine atom, iodine atom) (e.g., methoxy, ethoxy, propoxy, isopropoxy, butoxy, isobutoxy, sec-butoxy, pentyloxy, hexyloxy, fluoromethoxy etc.), (6) C₆₋₁₄ aryloxy (e.g., phenyloxy, naphthyloxy etc.), (7) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.), (8) mercapto, (9) C₁₋₆ alkylthio optionally having 1 to 3 halogen atoms (e.g., fluorine atom, chlorine atom, bromine atom, iodine atom) (e.g., methylthio, difluoromethylthio, trifluoromethylthio, ethylthio, propylthio, isopropylthio, butylthio, 4,4,4-trifluorobutylthio, pentylthio, hexylthio etc.), (10) C₆₋₁₄ arylthio (e.g., phenylthio, naphthylthio etc.), (11) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.), (12) amino, (13) mono-C₁₋₆ alkylamino (e.g., methylamino, ethylamino etc.), (14) mono-C₆₋₁₄ arylamino (e.g., phenylamino, 1-naphthylamino, 2-naphthylamino etc.), (15) mono-C₇₋₁₆ aralkylamino (e.g., benzylamino etc.), (16) di-C₁₋₆ alkylamino (e.g., dimethylamino, diethylamino etc.), (17) di-C₆₋₁₄ arylamino (e.g., diphenylamino etc.), (18) di-C₇₋₁₆ aralkylamino (e.g., dibenzylamino etc.), (19) formyl, (20) C₁₋₆ alkyl-carbonyl (e.g., acetyl, propionyl etc.), (21) C₆₋₁₄ arylcarbonyl (e.g., benzoyl, 1-naphthoyl, 2-naphthoyl etc.), (22) carboxyl, (23) C₁₋₆ alkoxy-carbonyl (e.g., methoxycarbonyl, ethoxycarbonyl, propoxycarbonyl, tert-butoxycarbonyl etc.), (24) C₆₋₁₄ aryloxy-carbonyl (e.g., phenoxycarbonyl etc.), (25) carbamoyl, (26) thiocarbamoyl, (27) mono-C₁₋₆ alkyl-carbamoyl (e.g., methylcarbamoyl, ethylcarbamoyl etc.), (28) di-C₁₋₆ alkyl-carbamoyl (e.g., dimethylcarbamoyl, diethylcarbamoyl, ethylmethylcarbamoyl etc.), (29) C₆₋₁₄ aryl-carbamoyl (e.g., phenylcarbamoyl, 1-naphthylcarbamoyl, 2-naphthylcarbamoyl etc.), (30) C₁₋₆ alkylsulfonyl (e.g., methylsulfonyl, ethylsulfonyl, trifluoromethylsulfonyl etc.) optionally having 1 to 3 halogen atoms (e.g., fluorine atom, chlorine atom, bromine atom, iodine atom), (31) C₆₋₁₄ arylsulfonyl (e.g., phenylsulfonyl, 1-naphthylsulfonyl, 2-naphthylsulfonyl etc.), (32) C₁₋₆ alkylsulfinyl (e.g., methylsulfinyl, ethylsulfinyl etc.), (33) C₆₋₁₄ arylsulfinyl (e.g., phenylsulfinyl, 1-naphthylsulfinyl, 2-naphthylsulfinyl etc.), (34) formylamino, (35) C₁₋₆ alkylcarbonylamino (e.g., acetylamino etc.), (36) C₆₋₁₄ arylcarbonylamino (e.g., benzoylamino, naphthoylamino etc.), (37) C₁₋₆ alkoxy-carbonylamino (e.g., methoxycarbonylamino, ethoxycarbonylamino, propoxycarbonylamino, butoxycarbonylamino etc.), (38) C₁₋₆ alkylsulfonylamino (e.g., methylsulfonylamino, ethylsulfonylamino etc.), (39) C₆₋₁₄ arylsulfonylamino (e.g., phenylsulfonylamino, 2-naphthylsulfonylamino, 1-naphthylsulfonylamino etc.), (40) C₁₋₆ alkyl-carbonyloxy (e.g., acetoxy, propionyloxy etc.), (41) C₆₋₁₄ aryl-carbonyloxy (e.g., benzoyloxy, naphthylcarbonyloxy etc.), (42) C₁₋₆ alkoxy-carbonyloxy (e.g., methoxycarbonyloxy, ethoxycarbonyloxy, propoxycarbonyloxy, butoxycarbonyloxy etc.), (43) mono-C₁₋₆ alkyl-carbamoyloxy (e.g., methylcarbamoyloxy, ethylcarbamoyloxy etc.), (44) di-C₁₋₆ alkyl-carbamoyloxy (e.g., dimethylcarbamoyloxy, diethylcarbamoyloxy etc.), (45) C₆₋₁₄ aryl-carbamoyloxy (e.g., phenylcarbamoyloxy, naphthylcarbamoyloxy etc.), (46) 5- to 7-membered saturated cyclic amino optionally containing, besides one nitrogen atom and carbon atom, one or two kinds of 1 to 4 hetero atoms selected from a nitrogen atom, a sulfur atom and an oxygen atom (e.g., pyrrolidin-1-yl, piperidino, piperazin-1-yl, morpholino, thiomorpholino, hexahydroazepin-1-yl etc.), (47) 5- to 10-membered aromatic heterocyclic group containing, besides carbon atom, one or two kinds of 1 to 4 hetero atoms selected from a nitrogen atom, a sulfur atom and an oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.), (48) C₁₋₃ alkylenedioxy (e.g., methylenedioxy, ethylenedioxy etc.), (49) C₃₋₇ cycloalkyl (e.g., cyclopropyl, cyclobutyl, cyclopentyl, cyclohexyl, cycloheptyl etc.), (50) a C₁₋₆ alkyl group (e.g., methyl, ethyl, n-propyl, isopropyl, n-butyl, isobutyl, sec-butyl, tert-butyl, n-pentyl, sec-pentyl, isopentyl, neopentyl, n-hexyl, isohexyl etc.) optionally having 1 to 3 halogen atoms (e.g., fluorine atom, chlorine atom, bromine atom, iodine atom) or a hydroxy group, (51) a C₂₋₆ alkenyl group (e.g., allyl, isopropenyl, isobutenyl, 1-methylallyl, 2-pentenyl, 2-hexenyl etc.) optionally having 1 to 3 halogen atoms (e.g., fluorine atom, chlorine atom, bromine atom, iodine atom), (52) a C₂₋₆ alkynyl group (e.g., propargyl, 2-butynyl, 3-butynyl, 3-pentynyl, 3-hexynyl etc.), (53) mono-C₃₋₇ cycloalkyl-carbamoyl (e.g., cyclopropylcarbamoyl, cyclobutylcarbamoyl etc.), and (54) 5- to 10-membered heterocyclyl-carbonyl containing, besides carbon atom, one or two kinds of 1 to 4 hetero atoms selected from a nitrogen atom, a sulfur atom and an oxygen atom (e.g., 4-morpholinocarbonyl etc.) (hereinafter to be referred to as substituent group B), and the like.

In the present specification, the substituent of the "optionally substituted hydrocarbon group" does not include an oxo group.

When the pharmaceutical active ingredient contains an optical isomer, a stereoisomer, a regioisomer or a rotamer, both an isomer and a mixture of these are also encompassed in the active ingredient. For example, when the pharmaceutical active ingredient has an optical isomer, an optical isomer resolved from a racemate is also encompassed in the active ingredient. These isomers can be obtained as single products according to synthesis and separation methods known *per se* (concentration, solvent extraction, column chromatography, recrystallization, etc.).

The pharmaceutical active ingredient may be a crystal, and encompasses both a single form and a mixture thereof. The crystal can be produced by crystallizing according to a crystallization method known per se.

The pharmaceutical active ingredient may be a solvate (e.g., hydrate etc.) or non-solvate, and both are encompassed in the pharmaceutical active ingredient.

A compound labeled with an isotope (e.g., ³H, ¹⁴C, ³⁵S, ¹²⁵I and the like) and the like are also encompassed in the pharmaceutical active ingredient.

The above mentioned pharmaceutical active ingredient can be produced according to the methods described in, for example, WO2006/036024, WO2007/026916, WO2008/108380, WO2009/041705 and WO2010/024451.

The pharmaceutically active ingredient having a primary or secondary amino group in the liquid preparation of the present invention include the following compounds. 1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, and 1-[4-fluoro-5-(2-fluoropyridin-3-yl)-1-(pyridin-3-ylsulfonyl)-1H-pyrrol-3-yl]-N-methylmethanamine.

In the liquid preparation of the present invention, particularly preferred as the pharmaceutically active ingredient having a primary or secondary amino group includes 1-[5-(2-fluorophenyl)-1-(pyridin-3-ylsulfonyl)-1H-pyrrol-3-yl]-N-methylmethanamine (hereinafter to be referred to as compound A), N-methyl-1-[5-(2-methylphenyl)-1-(pyridin-3-ylsulfonyl)-1H-pyrrol-3-yl]methanamine (hereinafter compound B) and 1-[4-fluoro-5-(2-fluoropyridin-3-yl)-1-(pyridin-3-ylsulfonyl)-1H-pyrrol-3-yl]-N-methylmethanamine (hereinafter compound C). More preferred are compound A and compound B. Of these, 1-[5-(2-fluorophenyl)-1-(pyridin-3-ylsulfonyl)-1H-pyrrol-3-yl]-N-methylmethanamine (compound A) is preferable.

For production of the liquid preparation of the present invention, the above-mentioned "pharmaceutically active ingredient having a primary or secondary amino group" forms a salt as defined below.

In a material to be used for the production of the liquid preparation of the present invention, the pharmaceutically active ingredient preferably forms a salt with an organic acid as defined above.

As such salt of a pharmaceutically active ingredient having a primary or secondary amino group with an organic acid, a salt with α,β-unsaturated carboxylic acid, specifically, a salt with a compound represented by the formula (IV): 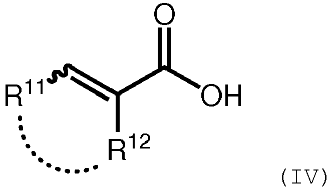 wherein R¹¹ and R¹² are the same or different and each is a hydrogen atom, an optionally substituted hydrocarbon group, a carboxyl group, a halogen atom, a C₁₋₆ alkoxy-carbonyl group, or a C₁₋₆ alkoxy group, or R¹¹ and R¹² jointly form an optionally substituted ring,
or ascorbic acid can be mentioned. Of these, a compound represented by the formula (IV) is preferable.

In the above-mentioned formula (IV), examples of the "optionally substituted hydrocarbon group" for R¹¹ or R¹² include those similar to the "optionally substituted hydrocarbon group" exemplified above as the "organic residue" for R^{a} or R^{b}.

In the above-mentioned formula (IV), examples of the "halogen atom" for R¹¹ or R¹² include a fluorine atom, a chlorine atom, a bromine atom, and an iodine atom.

In the above-mentioned formula (IV), examples of the "C₁₋₆ alkoxy-carbonyl group" for R¹¹ or R¹² include methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl and the like.

In the above-mentioned formula (IV), examples of the "C₁₋₆ alkoxy group" for R¹¹ or R¹² include methoxy, ethoxy, propoxy, isopropoxy, butoxy, isobutoxy, sec-butoxy, pentyloxy, hexyloxy and the like.

In the above-mentioned formula (IV), examples of the "optionally substituted ring jointly formed by R¹¹ and R¹²" for R¹¹ or R¹² include an optionally substituted benzene ring and the like. Examples of the substituent of the ring include C₁₋₄ alkyl group (e.g., methyl) and the like. The "optionally substituted ring jointly formed by R¹¹ and R¹²" is preferably a unsubstituted benzene ring.

Examples of the salts of a pharmaceutically active ingredient having a primary or secondary amino group as defined above with an organic acid include salts with ascorbic acid, benzoic acid, sorbic acid, fumaric acid, maleic acid and the like. Of these, a salt with benzoic acid, sorbic acid, fumaric acid or maleic acid is preferable. In addition, of the organic acid salts, a salt with unsaturated carboxylic acid is particularly preferably used. Examples of such salt with unsaturated carboxylic acid include salts with fumaric acid, sorbic acid, maleic acid and the like. Of these, a salt with fumaric acid and the like are preferable.

The concentration of the "pharmaceutically active ingredient having a primary or secondary amino group" as defined above in the liquid preparation of the present invention is desirably 0.1 - 100 mg/mL, further desirably 0.1 - 50 mg/mL, especially desirably 0.1 - 10 mg/mL.

When a salt of the "pharmaceutically active ingredient having a primary or secondary amino group" as defined above with an organic acid as defined above is used as the material of the liquid preparation of the present invention, since the organic acid is liberated into the liquid by formulating a liquid preparation, the "pharmaceutically active ingredient having a primary or secondary amino group" as defined above in the liquid preparation of the present invention may be present as a compound without forming a salt with the organic acid (free form) or a compound in the form of a salt with the organic acid, the two compounds being in an equilibrium.

### [2. Organic acid (second component)]

The "organic acid" to be used in the present invention is α,β-unsaturated carboxylic acid, specifically, a compound represented by the formula (IV):

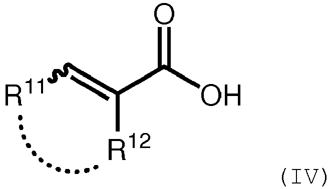

wherein R¹¹ and R¹² are the same or different and each is a hydrogen atom, an optionally substituted hydrocarbon group, a carboxyl group, a halogen atom, a C₁₋₆ alkoxy-carbonyl group, or a C₁₋₆ alkoxy group, or R¹¹ and R¹² jointly form an optionally substituted ring,
or ascorbic acid. Of these, a compound represented by the formula (IV) is preferable.

Examples of the "organic acid" to be used in the present invention include edible organic acids such as ascorbic acid, benzoic acid, sorbic acid, fumaric acid, maleic acid and the like. Of these, benzoic acid, sorbic acid, fumaric acid and maleic acid are preferable.

These organic acids may be used alone or two or more kinds thereof may be used simultaneously. The organic acid may be added separately from the "pharmaceutically active ingredient having a primary or secondary amino group" as defined above. When the material for the production of the liquid preparation is a salt of the "pharmaceutically active ingredient having a primary or secondary amino group" as defined above with an organic acid, the organic acid may be an organic acid liberated into the liquid preparation by formulation.

Here, the organic acid used when the above-mentioned "pharmaceutically active ingredient having a primary or secondary amino group" as defined above is a salt with an organic acid, and an organic acid to be separately added may be the same or different. When the above-mentioned "pharmaceutically active ingredient having a primary or secondary amino group" as defined above is a free form when producing the liquid preparation, the organic acid may be separately added.

The "organic acid" to be used in the present invention is desirably an organic acid liberated into a liquid when a salt of the "pharmaceutically active ingredient having a primary or secondary amino group" as defined above with an organic acid is formulated into a liquid preparation. In the liquid preparation of the present invention, when the organic acid is added separately from the "pharmaceutically active ingredient having a primary or secondary amino group" as defined above, it is a free form at the time of production of the liquid preparation. When a salt of the "pharmaceutically active ingredient having a primary or secondary amino group" as defined above with an organic acid is used as the material for producing a liquid preparation, the organic acid is preferably an organic acid liberated into the liquid preparation due to formulation thereof, and it is preferable to not further add other organic acid. In the liquid preparation of the present invention, the "organic acid" may be liberated into a liquid or form an organic acid salt compound of the active ingredient, which is in equilibrium with the active ingredient (free form).

In the present invention, when a salt of the "pharmaceutically active ingredient having a primary or secondary amino group" as defined above with an organic acid is used as the material and the pharmaceutically active ingredient is formulated into a liquid preparation, a decrease in the stability of the pharmaceutically active ingredient, which is due to the organic acid liberated into the liquid, can be prevented.

As the organic acid salt compound of the "pharmaceutically active ingredient having a primary or secondary amino group" to be used as a material in the present invention to produce a liquid preparation, for example, 1-[5-(2-fluorophenyl)-1-(pyridin-3-ylsulfonyl)-1H-pyrrol-3-yl]-N-methylmethanamine fumarate, N-methyl-1-[5-(2-methylphenyl)-1-(pyridin-3-ylsulfonyl)-1H-pyrrol-3-yl]methanamine fumarate, or 1-[4-fluoro-5-(2-fluoropyridin-3-yl)-1-(pyridine-3-ylsulfonyl)-1H-pyrrol-3-yl]-N-methylmethanamine 0.5 fumarate is preferable. Particularly, fumarate of compound A and fumarate of compound B are preferable, and 1-[5-(2-fluorophenyl)-1-(pyridin-3-ylsulfonyl)-1H-pyrrol-3-yl]-N-methylmethanamine fumarate (fumarate of compound A) is more preferable. These compounds may be present in the liquid preparation as an equilibrium of a compound without forming a salt with the organic acid (free form) and a compound forming a salt with the organic acid.

In the liquid preparation of the present invention, the molar ratio of the pharmaceutically active ingredient having a primary or secondary amino group as defined above, and an organic acid is 1:0.001 to 1:1000, preferably 1:0.01 to 1:100, more preferably 1:0.1 to 1:10.

### [3. Salt (tertiary component)]

The above-mentioned "pharmaceutically active ingredient having a primary or secondary amino group (first component)" as defined above has a primary or secondary amino group having high nucleophilicity.

When the highly nucleophilic pharmaceutically active ingredient having a primary or secondary amino group as defined above is dissolved or suspended in a suitable solvent (e.g., distilled water for injection, electrolyte liquid etc.), it is highly possible that the Michael addition (nucleophilic addition reaction that occurs on a carbon at the end of a conjugated system in conjugation with an electron-withdrawing substituent) occurs with an α,β-unsaturated carbonyl compound (particularly α,β-unsaturated carboxylic acid such as fumaric acid etc.).

For example, when the highly nucleophilic pharmaceutically active ingredient having a primary or secondary amino group is as defined above, it highly possibly reacts with an organic acid liberated in the liquid (e.g., a compound represented by the formula (IV):

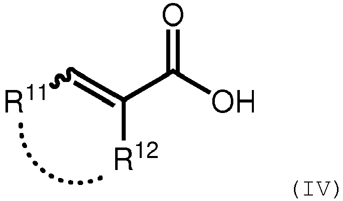

[R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring], or ascorbic acid) to produce a compound represented by the formula (V) or (V'):

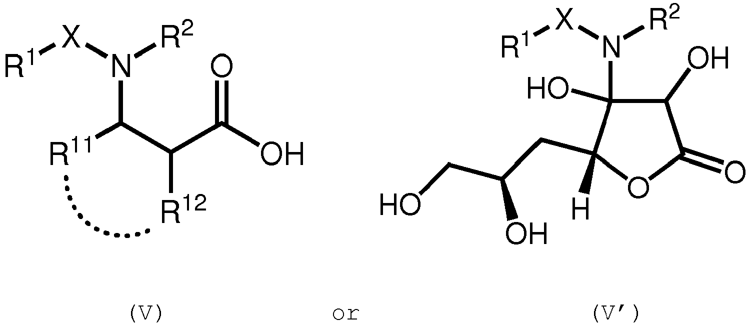

wherein R¹¹ and R¹² are as defined above, R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-, in the liquid.

When a reaction product as the one represented by the formula (V) or (V') is produced in the liquid, an inconvenient need to secure nontoxicity and the like occur and the liquid may be unsuitable as a medicament. In addition, even when the reaction product can be separated and removed from the liquid by a known means, it is industrially complicated, which is undesirable in terms of production costs.

Thus, the liquid preparation of the present invention is added with a salt as defined above as a stabilizer. Said stabilizer stabilizes the preparation by preventing a reaction of an α,β-unsaturated carbonyl compound (particularly, α,β-unsaturated carboxylic acid) with an amino group in the pharmaceutically active ingredient having a primary or secondary amino group as defined above. In other words, in the present invention, a reaction product of the pharmaceutically active ingredient having a primary or secondary amino group as defined above with an organic acid as defined above is suppressed by adding a salt as defined above to a composition containing the pharmaceutically active ingredient having a primary or secondary amino group and the organic acid. The production suppressive effect of the salt as defined above on the reaction product of the pharmaceutically active ingredient having a primary or secondary amino group as defined above and an organic acid as defined above in the liquid preparation, namely, new use of the salt for stabilizing a liquid preparation containing the pharmaceutically active ingredient having a primary or secondary amino group and an organic acid has not been known heretofore.

The salt to be used in the present invention is one or more kinds selected from the group consisting of sodium chloride, calcium chloride, magnesium chloride, sodium bromide, and calcium bromide. As the salt, a hydrate may also be used. As the salt to be used in the present invention, sodium chloride, calcium chloride, magnesium chloride or sodium bromide is preferable, and sodium chloride is particularly preferable.

The above-mentioned salt may be used alone or two or more kinds thereof may be used in combination.

The molar ratio of the pharmaceutically active ingredient having a primary or secondary amino group as defined above, and the salt in the liquid preparation of the present invention is 1:0.001 to 1:10000, preferably 1:0.01 to 1:1000, more preferably 1:0.1 to 1:500. The molar concentration of the salt in the liquid preparation of the present invention is preferably not less than 15 mmol/L (more preferably not less than 17 mmol/L, still more preferably not less than 17 mmol/L and not more than 310 mmol/L), more preferably not less than 30 mmol/L (more preferably not less than 34 mmol/L, still more preferably not less than 34 mmol/L and not more than 250 mmol/L), particularly preferably not less than 50 mmol/L (more preferably not less than 51 mmol/L, still more preferably not less than 51 mmol/L and not more than 250 mmol/L), further more preferably not less than 70 mmol/L (more preferably not less than 77 mmol/L, still more preferably not less than 77 mmol/L and not more than 200 mmol/L), especially preferably not less than 150 mmol/L (more preferably not less than 150 mmol/L, still more preferably not less than 150 mmol/L and not more than 200 mmol/L), most preferably not less than 154 mmol/L. The most preferable molar concentration of the salt in the liquid preparation of the present invention is 154 mmol/L.

The liquid preparation of the present invention is useful as a stable and safe medicament wherein the content of the reaction product in the aforementioned liquid preparation is not more than 5%, preferably not more than 3%, more preferably not more than 1%.

In the liquid preparation of the present invention, the amount of the reaction product of the pharmaceutically active ingredient as defined above and the organic acid as defined above is controlled, and therefore, it is useful as a stable and safe medicament. The liquid preparation of the present invention which does not substantially contain a reaction product of the pharmaceutically active ingredient as defined above and an organic acid as defined above contains the reaction product preferably at not more than about 1.8-fold (preferably not less than about 1-fold and not more than about 1.8-fold, more preferably not less than about 1-fold and not more than about 1.5-fold, more preferably not less than about 1-fold and not more than about 1.4-fold) % after preservation at 70°C for 1 week and preferably at not more than about 1.3-fold (preferably not less than about 1-fold and not more than about 1.3-fold, more preferably not less than about 1-fold and not more than about 1.2-fold, more preferably not less than about 1-fold and not more than about 1.1-fold) % after preservation at 60°C for 1 week, than before the preservation.

In the liquid preparation of the present invention, the amount of the reaction product of the pharmaceutically active ingredient as defined above and the organic acid as defined above is controlled, and therefore, it is useful as a stable and safe medicament. Here, that the amount of the reaction product of the pharmaceutically active ingredient as defined above and the organic acid as defined above is "controlled" means, for example, that the content of the reaction product in the aforementioned liquid preparation increases preferably at not more than about 1.8-fold (preferably not less than about 1-fold and not more than about 1.8-fold, more preferably not less than about 1-fold and not more than about 1.5-fold, more preferably not less than about 1-fold and not more than about 1.4-fold) % after preservation at 70°C for 1 week and preferably at not more than about 1.3-fold (preferably not less than about 1-fold and not more than about 1.3-fold, more preferably not less than about 1-fold and not more than about 1.2-fold, more preferably not less than about 1-fold and not more than about 1.1-fold) % after preservation at 60°C for 1 week, than before the preservation.

Specifically, when the pharmaceutically active ingredient is compound A, the content of the aforementioned reaction product in the liquid preparation after preservation at 70°C for 1 week is preferably not more than 0.24%, more preferably not less than 0.02% and not more than 0.24%, further preferably not less than 0.02% and not more than 0.20% by the measurement at Rt: about 0.79 (Rt is a relative retention time when the elution time of compound A is 1). The increase rate of the reaction product is preferably about 1-fold to about 1.8-fold, more preferably about 1-fold to about 1.6-fold, further preferably about 1-fold to about 1.5-fold, at % ratio. When the pharmaceutically active ingredient is compound A, the content of the aforementioned reaction product in the liquid preparation after preservation at 60°C for 1 week is preferably not more than 0.13% (e.g., not less than 0.02% and not more than 0.13%), more preferably not more than 0.12% (e.g., not less than 0.02% and not more than 0.12%), further preferably not more than 0.11% (e.g., not less than 0.02% and not more than 0.11%) by the measurement at Rt: about 0.79 (Rt is a relative retention time when the elution time of compound A is 1). The increase ratio of the reaction product is preferably about 1-fold to about 1.3-fold, more preferably about 1-fold to about 1.2-fold, further preferably about 1-fold to about 1.1-fold, at % ratio.

When the pharmaceutically active ingredient is compound B, the content of the aforementioned reaction product in the liquid preparation after preservation at 60°C for 1 week is preferably not more than 0.11% (e.g., not less than 0.02% and not more than 0.11%), more preferably not more than 0.10% (e.g., not less than 0.02% and not more than 0.10%), by the measurement at Rt: about 0.38 (Rt is a relative retention time when the elution time of compound B is 1). The increase ratio of the reaction product is preferably about 1-fold to about 1.5-fold, more preferably about 1-fold - about 1.3-fold, at % ratio.

When the pharmaceutically active ingredient is compound C, the content of the aforementioned reaction product in the liquid preparation is preferably not more than 0.68% (e.g., not less than 0.02% and not more than 0.68%), more preferably not more than 0.60% (e.g., not less than 0.02% and not more than 0.6%), further preferably not more than 0.58% (e.g., not less than 0.02% and not more than 0.58%), by the measurement at Rt: about 0.8. The increase ratio of the reaction product is preferably about 1-fold to about 1.5-fold, more preferably about 1-fold to about 1.4-fold, further preferably about 1-fold to about 1.38-fold, at % ratio.

When the pharmaceutically active ingredient is compound A, and fumaric acid is further added in addition to the fumarate compound of compound A as the material, the content of the reaction product in the liquid preparation of the present invention after treatment at 123°C in an autoclave is preferably not more than 1%, more preferably not more than 0.80% (e.g., not less than 0.02% and not more than 0.80%), further preferably not more than 0.70% (e.g., not less than 0.02% and not more than 0.70%), by the measurement at Rt:0.79 (Rt is a relative retention time when the elution time of compound A is 1). The increase ratio of the reaction product is preferably not more than about 6-fold, more preferably not more than about 5-fold, still more preferably not more than about 4.5-fold.

The percentage of the content (%) of the reaction product is the ratio thereof to the total peak area of the pharmaceutically active ingredient compound as described above and an analog thereof (reaction product), with the area on the chromatograph as 100%, which is detected by the HPLC method

(high performance liquid chromatography method), and the increase rate is obtained by dividing the content (%) of the reaction product in the liquid preparation calculated after preservation by the content(%) of the reaction product in the liquid preparation calculated before preservation (content (%) of reaction product after preservation/content (%) of reaction product before preservation).

The content of the reaction product can be measured according to Experimental Examples 1-5, 8 and 10 to be mentioned later under the following HPLC test conditions.

### [HPLC test conditions]

system: Waters 2690 Separation Module
detector: ultraviolet absorption spectrophotometer (measurement wavelength: 230 nm) (waters 2487 Dual λ Absorbance Detector)

### Condition (1) 70°C 1 week

column: CAPCELL PAK C18 MGII, 3 µm, 4.6 mm i.d.× 150 mm (manufactured by Shiseido Co., Ltd.)
column temperature: constant temperature near 25°C

### mobile phase A:

0.05 mol/L sodium phosphate buffer (pH 6.0)/acetonitrile mixed solution (19:1)

### mobile phase B:

acetonitrile/0.05 mol/L sodium phosphate buffer (pH 6.0) mixed solution (3:2)

### feed for the mobile phase:

**Table 1**

| time (min) | mobile phase A (%) | mobile phase B (%) |
|---|---|---|
| 0 (injection) | 100 | 0 |
| 20 | 80 | 20 |
| 60 | 70 | 30 |
| 110 | 0 | 100 |
| 110.1 | 100 | 0 |
| 120 (injection) | 100 | 0 |

Condition (2) 60°C 1 week or autoclave at 123°C
column: CAPCELL PAK C18 MGII, 3 µm, 4.6 mm i.d.× 100 mm (manufactured by Shiseido Co., Ltd.)
column temperature: constant temperature near 25°C

### mobile phase A:

0.025 mol/L sodium phosphate buffer (pH 6.8)/methanol/acetonitrile mixed solution (14:5:1)

### mobile phase B:

acetonitrile/0.025 mol/L sodium phosphate buffer (pH 6.8) mixed solution (7:3)

### feed for the mobile phase:

**Table 2**

| time (min) | mobile phase A (%) | mobile phase B (%) |
|---|---|---|
| 0 (injection) | 100 | 0 |
| 10 | 100 | 0 |
| 30 | 50 | 50 |
| 40 | 0 | 100 |
| 45 | 0 | 100 |
| 45.1 | 100 | 0 |
| 55 (injection) | 100 | 0 |

### Condition (3) 60°C 2 weeks

column: Zorbax Eclipse XDB-C18, 5 µm, 4.6 mm i.d.×150 mm (manufactured by Agilent)
column temperature: constant temperature near 25°C
mobile phase A: 0.02 mol/L sodium phosphate buffer (pH 7.0)/acetonitrile mixed solution (19:1)
mobile phase B: acetonitrile/0.02 mol/L sodium phosphate buffer (pH 7.0) mixed solution (3:2)

### feed for the mobile phase:

**Table 3**

| time (min) | mobile phase A (%) | mobile phase B (%) |
|---|---|---|
| 0 (injection) | 100 | 0 |
| 5 | 100 | 0 |
| 80 | 0 | 100 |
| 81 | 100 | 0 |
| 90 | 100 | 0 |

The preservation environment after a pharmaceutical product left the manufacturer to be placed on the market is difficult to control. To maintain the quality of the pharmaceutical product, therefore, the content of the reaction product of the pharmaceutically active ingredient as defined above and an organic acid as defined above or a decomposed product (of the pharmaceutically active ingredient) in the whole pharmaceutical composition is preferably low under any temperature and humidity conditions (e.g., 2 - 8°C, 25°C, 40°C) and in any package form (open state, sealed state etc.).

The liquid preparation of the present invention shows a small content of the reaction product of the pharmaceutically active ingredient as defined above and an organic acid as defined above or a decomposed product (of the pharmaceutically active ingredient) in the whole pharmaceutical composition under any conditions (e.g., 2 - 8°C, 25°C/60% RH, 40°C/75% RH, 60°C, 70°C, 123°C etc.). Therefore, the liquid preparation of the present invention can retain preservation stability under any conditions and can maintain high quality.

The liquid preparation of the present invention has low toxicity and can be safely administered orally or parenterally (e.g., topical, intravenous administration etc.) as, for example, a pharmaceutical preparation such as injection (e.g., solution for injection, suspend injection etc.); liquid (e.g., drink, syrup) and the like. In addition, it is also possible to freeze-dry the liquid preparation of the present invention according to a method known per se after production to give a freeze-dry preparation, and use by dissolving or suspending in an aqueous solvent (e.g., distilled water for injection, electrolyte liquid etc.) when in use.

The liquid preparation of the present invention is preferably administered as an intravenous injection such as a solution for injection and the like.

The liquid preparation of the present invention may contain, besides a pharmaceutically active ingredient having a primary or secondary amino group as defined above, an organic acid as defined above and a salt as defined above, additives such as conventionally-used solvent, solubilizing agent, suspending agent, isotonicity agent, pH adjusting agent, buffering agent, soothing agent and the like as preparation materials. Where necessary, general preservative, antioxidant and the like can also be used.

Examples of the "solvent" include water for injection (distilled water for injection), alcohol, propylene glycol, macrogol, sesame oil, corn oil, olive oil and the like.

The content of the "solvent" in the whole liquid preparation is 0.1 mg - 100 g, preferably 0.1 mg - 20 g.

Examples of the "solubilizing agent" include polyethylene glycol, propylene glycol, benzyl benzoate, ethanol, trisaminomethane, cholesterol, triethanolamine, sodium carbonate, sodium citrate and the like.

The content of the "solubilizing agent" in the whole liquid preparation is 0.1 mg - 100 g, preferably 0.1 mg - 10 g.

Examples of the "suspending agent" include surfactants such as stearyltriethanolamine, sodium lauryl sulfate, laurylaminopropionic acid, lecithin, benzalkonium chloride, benzethonium chloride, glyceryl monostearate etc; hydrophilic polymers such as polyvinyl alcohol, polyvinylpyrrolidone, carboxymethyl cellulose sodium, methyl cellulose, hydroxymethyl cellulose, hydroxyethyl cellulose, hydroxypropyl cellulose etc., and the like.

The content of the "suspending agent" in the whole liquid preparation is 0.1 mg - 100 g, preferably 0.1 mg - 10 g.

Examples of the "isotonicity agent" include glucose, D-sorbitol, glycerol, D-mannitol and the like.

The content of the "isotonicity agent" in the whole liquid preparation is 0.1 mg - 100 g, preferably 0.1 mg - 10 g.

Examples of the "pH adjusting agent" include basic inorganic salt (e.g., sodium hydroxide, potassium hydroxide), inorganic acid (e.g., phosphoric acid, carbonic acid and the like), alkali metal salt with inorganic acid (e.g., potassium chloride etc.), alkaline earth metal salt with inorganic acid (e.g., calcium chloride, magnesium chloride etc.), alkali metal salt with organic acid (e.g., sodium citrate, sodium tartrate etc.), alkaline earth metal salt with organic acid (e.g., calcium citrate, calcium lactate, magnesium gluconate etc.), neutral amino acid (e.g., glycine, alanine etc.), acidic amino acid (aspartic acid, glutamic acid etc.), salt with acidic amino acid (e.g., sodium aspartate, potassium glutamate etc.), salt with basic amino acid (e.g., lysine hydrochloride, arginine hydrochloride etc.) and the like.

The content of the "pH adjusting agent" in the whole liquid preparation is 0.1 mg - 100 g, preferably 0.1 mg - 10 g.

Examples of the "buffering agent" include buffer solutions of phosphates, acetates, carbonates, citrates etc, and the like.

The content of the "buffering agent" in the whole liquid preparation is 0.1 mg - 100 g, preferably 0.1 mg - 10 g.

Examples of the "soothing agent" include glucose, benzyl alcohol, mepivacaine hydrochloride, xylocaine hydrochloride, procaine hydrochloride, carbocain hydrochloride and the like.

The content of the "soothing agent" in the whole liquid preparation is 0.01 mg - 4000 mg, preferably 0.01 mg - 100 mg.

Examples of the "preservative" include p-oxybenzoic acid esters, chlorobutanol, benzyl alcohol, phenethyl alcohol, dehydroacetic acid, sorbic acid and the like.

The content of the "preservative" in the whole liquid preparation is 1 mg - 4000 mg, preferably 1 mg - 500 mg.

Examples of the "antioxidants" include sulfites, ascorbic acid, α-tocopherol and the like.

The content of the "antioxidant" in the whole liquid preparation is 10 mg - 3000 mg, preferably 10 mg - 100 mg.

The liquid preparation of the present invention may be in the form of a liquid (e.g., solution for injection) or a semisolid (e.g., thick aqueous injection and the like). In addition, the liquid preparation of the present invention may be freeze-dried to give a freeze-dry preparation (freeze-dry injection). Moreover, the liquid preparation of the present invention includes a solution preparation and a suspend preparation.

The liquid preparation of the present invention also includes an injection obtained by dissolving in or diluting with a dissolution liquid or dilution liquid when in use.

The liquid injection of the present invention is adjusted to a physiologically acceptable pH. In the present specification, the "physiologically acceptable pH" means a pH of about 3.0 to about 9.0, preferably about 3.0 to about 5.0, particularly preferably about 3.3 to about 4.3.

When the pH is not more than 3.0, disadvantages occur for patients since administration as an injection causes a pain and the like. On the other hand, when the pH is not less than 9.0, the safety as a medicament may not be maintained, since decomposition of the preparation components may be promoted, the development of the reaction product may not be suppressed and the like.

The liquid injection of the present invention is preferably a solution preparation (solution for injection) wherein the injection adjusted to a physiologically acceptable pH in advance is filled in a container such as vial, ampoule and the like, so that it can be rapidly administered to patients.

In addition, it can be formulated as an injection to be adjusted (dissolution, dilution) to a physiologically acceptable pH when in use.

The pH can be adjusted to, for example, the aforementioned "physiologically acceptable pH" when 5 mg of a pharmaceutically active ingredient as defined above is dissolved in 5 ml of saline or distilled water for injection.

When the liquid preparation of the present invention is an injection of a freeze-dry preparation and the like, the injection can be easily prepared by dissolving in or diluting with a dissolution liquid or dilution liquid (water for injection such as distilled water for injection and the like, infusion (electrolyte liquid such as saline and the like, etc.) and the like).

A freeze-dry preparation can be produced by dissolving a pharmaceutically active ingredient as defined above, an organic acid as defined above, a salt as defined above, and, where necessary, various additives in an aqueous solvent such as distilled water for injection and the like, adjusting pH, where necessary, with a pH adjusting agent such as aqueous sodium hydroxide solution and the like, and lyophilizing the solution.

The "freeze-drying" can be performed by a method known per se, and a method including freezing at a temperature of generally -25°C or below, and drying while raising the shelf temperature to 25°C to 40°C and maintaining the drying chamber vacuum at about 13.3 Pa or below is desirable.

A freeze-dry preparation may contain saccharides (e.g., sugar alcohol such as mannitol and the like, etc.) to stabilize the shape and the like.

When sodium hydroxide is used as a pH adjusting agent, for example, the concentration of "aqueous sodium hydroxide solution" is about 0.15 - about 10 mol/L. When alkali other than sodium hydroxide is used, it can be produced according to the aforementioned method.

As a container for liquid preparation, various containers such as glass container, plastic container and the like can be used irrespective of the material thereof. For the plastic container, polyethylene, polypropylene, polyethylene polypropylene copolymer, polyvinyl chloride, ethylenevinyl acetate·copolymer, ethylene·propylene copolymer, silicone, polybutadiene, thermoplastic elastomer, Teflon (registered trade mark), polyurethane, cyclic polyolefin and polyolefin can be used.

The "glass container (vial)" is preferably made from a glass material usable for injection. Preferable "vial" is USP TYPE I, II, III and the like, particularly TYPE I. In addition, a glass vial showing a reduced level of alkaline elution than the general level and the like can also be used.

As a plastic container (vial), cyclic polyolefin [e.g., CZ vial (Daikyo Seiko, Ltd.)] and the like can also be used.

The shape and size of the vial are not particularly limited. The volume of the vial is preferably not more than 100 ml, more preferably not more than 40 ml, particularly preferably not more than 20 ml. Specific examples of the vial include 17P vial, 9P vial, 5P vial and 3.5P vial.

When an "ampoule" is used, a glass ampoule is preferably made from a glass material usable for injection, and a plastic ampoule can be made from polyethylene, polypropylene, polyethylene polypropylene copolymer, polyvinyl chloride, ethylenevinyl acetate·copolymer, ethylene·propylene copolymer, silicone, polybutadiene, thermoplastic elastomer, Teflon (registered trade mark), polyurethane, cyclic polyolefin or polyolefin. The shape and size thereof are not particularly limited. The volume of the ampoule is preferably not more than 30 ml, more preferably not more than 20 ml, particularly preferably not more than 10 ml. Specific examples of the ampoule include 10P ampoule, 5P ampoule, 3P ampoule and the like.

In addition, a pre-filled syringe by filling an injection syringe in advance can also be used.

The preparation container can be coated with a packaging film. While the packaging film is not particularly limited, one embodiment includes cellophane, vinylidene chloride-coated cellophane, polyethylene, vinylidene chloride-coated stretched polypropylene, nylon, stretched nylon, vinylidene chloride-coated stretched nylon, stretched polypropylene, unstretched polypropylene, polyester, vinylidene chloride-coated polyester, aluminum, ethylenevinylalcohol polymer and the like, which may be transparent or colored. The packaging film may have light-shading property, or may shade a particular wavelength range that promotes photolysis. Preferably, a film capable of shading UV light and visible light can be mentioned. While the material of the shading film is not particularly limited, a material capable of shading the object wavelength range can be used, which may contain a UV absorber. In addition, the shading property may be achieved by paper. The film may block oxygen, or contain an oxygen absorber. It may have heat resistance to enable disinfection and sterilization. To enhance gas permeability, the film may have fine pores, or may be able to control gas permeability by way of the thickness and number of pores. Moreover, the film may be attached, closely adhered or bonded to a container by heating or adhesion.

When the preparation of the present invention is a freeze-dry injection requiring time for becoming clear after reconstitution when in use, due to vigorous foaming of the content and the like, a silicone-coated vial or ampoule is used to shorten the time required for the reconstitution. The silicone used for coating includes, for example, silicone oil such as dimethylpolysiloxane, methylhydrogenpolysiloxane and the like; and varnish silicone such as methyl varnish silicone, methylphenyl varnish silicone and the like, with preference given to KM-740 [Shin-Etsu Chemical Co., Ltd.].

When the liquid preparation of the present invention is a solution for injection and used in the form of a vial or ampoule, a desired, given amount is extracted with an injection syringe and the like and directed administered or, where necessary, combined and mixed with the below-mentioned infusion and the like when in use and administered by drip infusion. Therefore, the present invention also provides an injection kit containing the aforementioned solution for injection and an infusion in combination.

On the other hand, when the preparation is a freeze-dry preparation, it is used by redissolving in a solvent when in use.

Examples of the infusion include electrolyte liquids (saline, Ringer's solution and the like), nutrition infusions (carbohydrate solution (e.g., glucose solution such as 5%(w/v) glucose solution and the like)), and the like.

Examples of the "solvent used for reconstitution" include one kind of water for injection (distilled water for injection) and infusion [electrolyte liquid (saline, Ringer's solution and the like), nutrition infusion (carbohydrate solution (e.g., glucose solution such as 5%(w/v) glucose solution and the like, etc.), protein amino acid injection, vitamin injection and the like), blood substitute containing electrolyte liquid and nutrition infusion (carbohydrate solution and the like) in combination, lipid emulsion wherein lipid is emulsified and the like], or a mixed solvent two or more kinds thereof. The solvent may contain a pH adjusting agent (e.g., acidic substance, weak alkaline substance etc.) and the like as necessary.

The aforementioned "electrolyte liquid" is a solution of electrolyte dissolved in water for injection and includes, for example, a solution containing one or more kinds of sodium chloride, potassium chloride, calcium chloride, sodium lactate, sodium dihydrogen phosphate, magnesium carbonate and the like, lactic acid Ringer's solution, acetic acid Ringer's solution and the like. Preferable electrolyte liquid is a solution containing sodium chloride, particularly preferably physiological saline [0.9% (w/v) sodium chloride solution].

The aforementioned "carbohydrate solution" is a solution of saccharide dissolved in water for injection and includes, for example, a solution containing one or more kinds of glucose, fructose, sorbitol, mannitol, dextran and the like, etc. Preferable carbohydrate solution is 5 - 70%(w/v) glucose solution, particularly preferably 5% (w/v) glucose solution and 10% (w/v) glucose solution and the like.

The aforementioned "protein amino acid injection" is a solution of amino acid dissolved in water for injection and includes, for example, a solution containing one or more kinds of glycine, aspartic acid, lysine and the like, etc.

The aforementioned "vitamin injection" is a solution of vitamin dissolved in water for injection and includes, for example, a solution containing one or more kinds of vitamin B₁, vitamin C and the like, etc.

Preferable "solvent used for reconstitution" includes water for injection, physiological saline, and glucose solution (e.g., 5%(w/v) glucose solution and the like).

The amount of the aforementioned "infusion" or "solvent used for reconstitution" to be used for a single dose is 5 - 1000 ml, preferably 5 - 500 ml.

The liquid preparation of the present invention (particularly solution for injection) can be produced by, for example, dissolving the above-mentioned first to third components together with additives such as a buffering agent (e.g., citric acid, sodium citrate and the like), and the like in distilled water for injection by a method known per se, adjusting, where necessary, to a desired pH with a pH regulating agent such as aqueous sodium hydroxide solution and the like, and filling the solution in a vial or ampoule.

The liquid preparation of the present invention (particularly solution for injection) can also be produced by dissolving or suspending an organic acid salt of a pharmaceutically active ingredient having a primary or secondary amino group as defined above and a salt as defined above in a solvent. In this case, the organic acid is liberated in the liquid. The "pharmaceutically active ingredient having a primary or secondary amino group" as defined above in the liquid preparation may be present as a compound without forming a salt with the organic acid (free form) or a compound in the form of a salt with the organic acid, the two compounds being in an equilibrium.

The "organic acid salt" is a salt with a compound represented by the above-mentioned formula (IV) or ascorbic acid.

The liquid preparation of the present invention is superior in the preservation stability. The pharmaceutical composition described herein is useful for the treatment or prophylaxis of peptic ulcer (e.g., gastric ulcer, gastric ulcer due to postoperative stress, duodenal ulcer, anastomotic ulcer, ulcer caused by non-steroidal anti-inflammatory agents etc.); Zollinger-Ellison syndrome; gastritis; erosive esophagitis; reflux esophagitis such as erosive reflux esophagitis and the like; symptomatic gastroesophageal reflux disease (symptomatic GERD) such as non-erosive reflux disease or gastroesophageal reflux disease free of esophagitis and the like; functional dyspepsia; Barrett's esophagus; gastric cancer (including gastric cancer associated with promoted production of interleukin-1β due to gene polymorphism of interleukin-1); stomach MALT lymphoma; gastric hyperacidity; upper gastrointestinal hemorrhage due to peptic ulcer, acute stress ulcer, hemorrhagic gastritis or invasive stress (e.g. stress caused by major surgery requiring postoperative intensive management, and cerebrovascular disorder, head trauma, multiple organ failure and extensive burn, each requiring intensive treatment) and the like; airway disorders; asthma and the like, pre-anesthetic administration, eradication of Helicobacter pylori or eradication assistance and the like, in mammals (e.g., human, monkey, sheep, cattle, horse, dog, cat, rabbit, rat, mouse etc.).

Particularly, since the liquid preparation of the present invention (solution for injection and the like) provides a rapid hemostatic effect on upper gastrointestinal hemorrhage, it can be used as a therapeutic agent having an immediate effect for patients with upper gastrointestinal hemorrhage such as gastric ulcer, duodenal ulcer, acute stress ulcer, acute stomach mucosa lesion and the like accompanied by bleeding, who have difficulty in oral administration.

While the dose of the liquid preparation of the present invention also varies depending on the subject of administration, administration route, disease and the like, for example, when the preparation is administered as a solution for injection to an adult (60 kg) with gastric ulcer accompanied by bleeding, it is preferably administered in an amount corresponding to about 0.5 - about 1500 mg/day, preferably about 5 - about 150 mg/day, of the pharmaceutically active ingredient. The liquid preparation of the present invention may be administered once per day or in 2 or 3 portions per day. For example, when combined with infusion, it may be dripped over 1 min - 120 min, preferably 5 min - 90 min. The dosing period is about 1 day - 2 weeks, preferably about 1 day - 1 week, to ensure an efficient treatment effect.

When hemostasis is confirmed by endoscopy and the like, the liquid preparation may be changed to a solid preparation to reduce the burden on patients and the like.

The liquid preparation of the present invention may be used in combination with other active ingredients, as long as the activity of the pharmaceutically active ingredient having a primary or secondary amino group as described above is not impaired.

Examples of the "other active ingredients" include anti-Helicobacter pylori active substances, imidazole compounds, bismuth salts, quinolone compounds, and the like.

Examples of the "anti-Helicobacter pylori active substances" include penicillin antibiotic (e.g., amoxicillin, benzylpenicillin, piperacillin, mecillinam, ampicillin, temocillin, bacampicillin, aspoxicillin, sultamicillin, lenampicillin etc.), cephem antibiotic (e.g., cefixime, cefaclor etc.), macrolide antibiotic (e.g., erythromycin, clarithromycin, roxithromycin, rokitamycin, flurithromycin, telithromycin etc.), tetracycline antibiotic (e.g., tetracycline, minocycline, streptomycin etc.), aminoglycoside antibiotic (e.g., gentamicin, amikacin etc.), imipenem and the like. Of these substances, preferred are penicillin antibiotic, macrolide antibiotic and the like.

Examples of the "imidazole compounds" include metronidazole, miconazole and the like.

Examples of the "bismuth salts" include bismuth acetate, bismuth citrate, bithmuth subsalicylate and the like.

Examples of the "quinolone compounds" include ofloxacin, ciploxacin and the like.

Particularly, for bacteria elimination of Helicobacter pylori, a medicament containing the liquid preparation of the present invention, penicillin antibiotic (e.g., amoxicillin and the like) and erythromycin antibiotic (e.g., clarithromycin and the like) in combination is preferably used. For the purpose of eradication of Helicobacter pylori, while the liquid preparation of the present invention has an anti-H. pylori action (bacteriostatic action or eradication action) by itself, it can enhance the antibacterial action of other antibiotics based on the pH controlling action in the stomach and the like, and also provides an assistant effect such as an eradication effect based on the action of the antibiotics to be used in combination.

In addition, the pharmaceutical composition of the present invention may be used in combination with a gastric motility enhancer, a drug acting on lower esophageal sphincter (e.g., temporary lower esophageal sphincter relaxation suppressant etc.), ClC-2 channel opener (intestinal juice secretion enhancer), a histamine H2 receptor antagonist, an antacid, a sedative, a stomachic digestant or a non-steroidal anti-inflammatory drug (NSAID).

Examples of the "gastric motility enhancer" include domperidone, metoclopramide, mosapride, itopride, tegaserod and the like.

Examples of the "drug acting on lower esophageal sphincter" include GABA-B receptor agonists such as baclofen, an optically active form thereof and the like, etc.

Examples of the "ClC-2 channel opener (intestinal juice secretion enhancer)" include lubiprostone and the like.

Examples of the "histamine H2 receptor antagonist" include cimetidine, ranitidine, famotidine, roxatidine, nizatidine, lafutidine and the like.

Examples of the "antacid" include sodium hydrogencarbonate, aluminum hydroxide and the like.

Examples of the "sedatives" include diazepam, chlordiazepoxide and the like.

Examples of the "stomachic digestant" include gentiana, Swertia japonica, diastase and the like.

Examples of the "non-steroidal anti-inflammatory drug" include Aspirin, indomethacin, ibuprofen, mefenamic acid, diclofenac, etodorac, piroxicam, celecoxib and the like.

The pharmaceutical composition of the present invention may be used in combination with the following drugs.
(i) proton pump inhibitors, e.g., omeprazole, esomeprazole, pantoprazole, rabeprazole, tenatoprazole, ilaprazole and lansoprazole;
(ii) oral antacid mixtures, e.g., Maalox (registered trade mark), Aludrox (registered trade mark) and Gaviscon (registered trade mark);
(iii) mucosal protective agents, e.g., polaprezinc, ecabet sodium, rebamipide, teprenone, cetraxate, sucralfate, chloropylline-copper and plaunotol;
(iv) anti-gastric agents, e.g., anti-gastrin vaccine, itriglumide and Z-360;
(v) 5-HT₃ antagonists, e.g., dolasetron, palonosetron, alosetron, azasetron, ramosetron, mitrazapine, granisetron, tropisetron, E-3620, ondansetron and indisetron;
(vi) 5-HT₄ agonists, e.g., tegaserod, mosapride, cinitapride and oxtriptane;
(vii) laxatives, e.g., Trifyba (registered trade mark), Fybogel (registered trade mark), Konsyl (registered trade mark), Isogel (registered trade mark), Regulan (registered trade mark), Celevac (registered trade mark) and Normacol (registered trade mark);
(viii) GABA_{B} agonists, e.g., baclofen and AZD-3355;
(ix) GABA_{B} antagonists, e.g., GAS-360 and SGS-742;
(x) calcium channel blockers, e.g., aranidipine, lacidipine, falodipine, azelnidipine, clinidipine, lomerizine, diltiazem, gallopamil, efonidipine, nisoldipine, amlodipine, lercanidipine, bevantolol, nicardipine, isradipine, benidipine, verapamil, nitrendipine, barnidipine, propafenone, manidipine, bepridil, nifedipine, nilvadipine, nimodipine and fasudil;
(xi) dopamine antagonists, e.g., metoclopramide, domperidone and levosulpiride;
(xii) Tachykinin (NK) antagonists, particularly NK-3, NK-2 and NK-1 antagonists, e.g., nepadutant, saredutant, talnetant, (αR,9R)-7-[3,5-bis(trifluoromethyl)benzyl]-8,9,10,11-tetrahydro-9-methyl-5-(4-methylphenyl)-7H-[1,4]diazocino[2,1-g][1,7]naphthridine-6-13-dione (TAK-637), 5-[[(2R,3S)-2-[(1R)-1-[3,5-bis(trifluoromethyl)phenyl]ethoxy-3-(4-fluorophenyl)-4-morpholinyl]methyl]-1,2-dihydro-3H-1,2,4-triazol-3-one (MK-869), lanepitant, dapitant and 3-[[2-methoxy-5-(trifluoromethoxy)phenyl]methylamino]-2-phenyl-piperidine (2S,3S) ;
(xiii) nitric oxide synthase inhibitors, e.g., GW-274150, tilarginine, P54, guanidioethyldisulfide and nitroflurbiprofen;
(xiv) vanilloid receptor 1 antagonists, e.g., AMG-517 and GW-705498;
(xv) ghrelin agonists, e.g., capromorelin and TZP-101;
(xvi) AchE release stimulants, e.g., Z-338 and KW-5092;
(xvii) insomnia therapeutic agent(etizolam, zopiclone, triazolam, zolpidem, ramelteon, indiplon etc.);
(xviii) potassium-competitive acid blocker (P-CAB);
(xix) melatonin agonist;
(xx) melatonin, and the like.

The above-mentioned medicaments (i) - (xx) may be combinedly used by adding to the liquid preparation of the present invention, or the above-mentioned medicaments (i) - (xx) and the liquid preparation of the present invention may also be prepared as separate preparations and administered to the same subject simultaneously or in a staggered manner. Examples

The present invention is explained in more detail in the following by referring to Comparative Examples, Examples and Experimental Examples.

### Comparative Example 1

1-[5-(2-Fluorophenyl)-1-(pyridin-3-ylsulfonyl)-1H-pyrrol-3-yl]-N-methylmethanamine fumarate (hereinafter to be indicated as fumarate of compound A) (80 mg) was measured in a glass beaker, ultrapure water (produced by "ultrapure water production system WRX10 manufactured by YAMATO SCIENTIFIC CO., LTD.", resistance value not less than 15.0 MQcm) (50 mL) was added and the fumarate was dissolved by stirring the mixture with a stirrer. A 5 mol/L aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 60 mL, whereby a drug solution having the following composition was obtained.

| | |
|---|---|
| fumarate of compound A | 80 mg |
| 5 mol/L aqueous sodium hydroxide solution | q.s. (pH was adjusted to 4.0) |
| water | measured up to 60 mL |

### Example 1

Fumarate of compound A (80 mg) and sodium chloride (reagent special grade, Wako Pure Chemical Industries, Ltd.) (540 mg) were measured in a glass beaker, ultrapure water (produced by "ultrapure water production system WRX10 manufactured by YAMATO SCIENTIFIC CO., LTD.", resistance value not less than 15.0 MΩcm) (50 mL) was added and they were dissolved by stirring the mixture with a stirrer. A 5 mol/L aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 60 mL, whereby a drug solution having the following composition was obtained.

| | |
|---|---|
| fumarate of compound A | 80 mg |
| sodium chloride | 540 mg |
| 5 mol/L aqueous sodium hydroxide solution | q.s. (pH was adjusted to 4.0) |
| water | measured up to 60 mL |

### Example 2

Fumarate of compound A (80 mg) and calcium chloride dihydrate (reagent special grade, Wako Pure Chemical Industries, Ltd.) (1360 mg) were measured in a glass beaker, ultrapure water (produced by "ultrapure water production system WRX10 manufactured by YAMATO SCIENTIFIC CO., LTD.", resistance value not less than 15.0 MΩcm) (50 mL) was added and they were dissolved by stirring with a stirrer. A 5 mol/L aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 60 mL, whereby a drug solution having the following composition was obtained.

| | |
|---|---|
| fumarate of compound A | 80 mg |
| calcium chloride dihydrate | 1360 mg |
| 5 mol/L aqueous sodium hydroxide solution | q.s. (pH was adjusted to 4.0) |
| water | measured up to 60 mL |

### Example 3

Fumarate of compound A (80 mg) and magnesium chloride hexahydrate (reagent special grade, Wako Pure Chemical Industries, Ltd.) (1880 mg) were measured in a glass beaker, ultrapure water (produced by "ultrapure water production system WRX10 manufactured by YAMATO SCIENTIFIC CO., LTD.", resistance value not less than 15.0 MΩcm) (50 mL) was added and they were dissolved by stirring with a stirrer. A 5 mol/L aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 60 mL, whereby a drug solution having the following composition was obtained.

| | |
|---|---|
| fumarate of compound A | 80 mg |
| magnesium chloride hexahydrate | 1880 mg |
| 5 mol/L aqueous sodium hydroxide solution | q.s. (pH was adjusted to 4.0) |
| water | measured up to 60 mL |

### Experimental Example 1 (measurement method of reaction product)

The drug solutions of Comparative Example 1 and Examples 1 - 3 were each placed by about 10 mL in a glass vial (VIAL 17PC TOKAN, DAIWA SPECIAL GLASS Co., Ltd.), and the vial was tightly sealed, and preserved for 1 week at 70°C (TEMP.&HUMID. CHAMBER PR-4S, ESPEC CORP.).

Using the drug solutions before and after storage, production of the reaction product of compound A (relative retention time when elution time of compound A is 1, (Rt): about 0.75) was examined. The reaction product was measured by the HPLC method and using the drug solutions diluted 2.5-fold with an ultrapure water (produced by "ultrapure water production system WRX10 manufactured by YAMATO SCIENTIFIC CO., LTD.", resistance value not less than 15.0 MΩcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (waters 2487 Dual λ Absorbance Detector)
column: CAPCELL PAK C18 MGII, 3 µm, 4.6 mm i.d.×150 mm (manufactured by Shiseido Co., Ltd.)
column temperature: constant temperature near 25°C
mobile phase A: 0.05 mol/L sodium phosphate buffer (pH 6.0)/acetonitrile mixed solution (19:1)
mobile phase B: acetonitrile/0.05 mol/L sodium phosphate buffer (pH 6.0) mixed solution (3:2)

### [Preparation method of 0.05 mol/L sodium phosphate buffer (pH 6.0) ]

Disodium hydrogen phosphate (anhydrous) (reagent special grade, Wako Pure Chemical Industries, Ltd.) (7.1 g) was dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was adjusted to pH 6.0 with phosphoric acid (reagent special grade, Wako Pure Chemical Industries, Ltd.).

The concentration gradient of the feed for the mobile phase was controlled by changing the mixing ratio of the mobile phase as follows.

**Table 4**

| time (min) | mobile phase A (%) | mobile phase B (%) |
|---|---|---|
| 0 (injection) | 100 | 0 |
| 20 | 80 | 20 |
| 60 | 70 | 30 |
| 110 | 0 | 100 |
| 110.1 | 100 | 0 |
| 120 (injection) | 100 | 0 |

In Comparative Example 1 and Examples 1 - 3, the reaction product was measured before and after storage at 70°C for 1 week. The results are shown in Table 5. Addition of chloride suppressed an increase of the reaction product.

**Table 5**

| Reaction product after storage at 70°C for 1 week | | | | |
|---|---|---|---|---|
| sample | additive | concentration of additive | before storage | after storage |

| reaction product (Rt: about 0.79) (%) | reaction product (Rt: about 0.79) (%) | | | |
|---|---|---|---|---|
| Comp. Ex. 1 | none | - | 0.13 | 0.25 |
| Ex. 1 | sodium chloride | 154 mmol/L | 0.13 | 0.18 |
| Ex. 2 | calcium chloride dihydrate | 154 mmol/L | 0.13 | 0.15 |
| Ex. 3 | magnesium chloride hexahydrate | 154 mmol/L | 0.13 | 0.16 |

### Comparative Example 2

Fumarate of compound A (67 mg) was measured in a glass beaker, ultrapure water (produced by "ultrapure water production system WRX10 manufactured by YAMATO SCIENTIFIC CO., LTD.", resistance value not less than 15.0 MΩcm) (50 mL) was added and the fumarate was dissolved by stirring the mixture with a stirrer, whereby a drug solution having the following composition was obtained (pH=3.6, measured using HORIBA pH METER F-52).

| | |
|---|---|
| fumarate of compound A | 67 mg |
| water | 50 mL |

### Example 4

Fumarate of compound A (67 mg) was measured in a glass beaker, OTSUKA NORMAL SALINE (Otsuka Pharmaceutical Factory, Inc.) (50 mL) was added and the fumarate was dissolved by stirring the mixture with a stirrer, whereby a drug solution having the following composition was obtained (pH=3.6, measured using HORIBA pH METER F-52).

| | |
|---|---|
| fumarate of compound A | 67 mg |
| sodium chloride | 450 mg |
| water | 50 mL |

### Example 5

Fumarate of compound A (67 mg) and sodium bromide (reagent special grade, Wako Pure Chemical Industries, Ltd.) (1154 mg) were measured in a glass beaker, ultrapure water (produced by "ultrapure water production system WRX10 manufactured by YAMATO SCIENTIFIC CO., LTD.", resistance value not less than 15.0 MΩcm) (50 mL) was added and they were dissolved by stirring the mixture with a stirrer, whereby a drug solution having the following composition was obtained (pH=3.6, measured using HORIBA pH METER F-52).

| | |
|---|---|
| fumarate of compound A | 67 mg |
| sodium bromide | 1154 mg |
| water | 50 mL |

### Experimental Example 2 (measurement method of reaction product)

The drug solutions of Comparative Example 2 and Examples 4, 5 were each placed by about 5 mL in a glass vial (VIAL 17PC TOKAN, DAIWA SPECIAL GLASS Co., Ltd.), and the vial was tightly sealed, and preserved at 60°C (TABAI PERFECT OVEN-ORIGINAL PV-220, ESPEC CORP.) for 1 week.

Using the drug solutions before and after storage, production of the reaction product of compound A (relative retention time when elution time of compound A is 1, (Rt): about 0.79) was examined. The reaction product was measured by the HPLC method and using the drug solutions diluted 2.5-fold with an ultrapure water (produced by "ultrapure water production system WRX10 manufactured by YAMATO SCIENTIFIC CO., LTD.", resistance value not less than 15.0 MΩcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (waters 2487 Dualλ Absorbance Detector)
column: CAPCELL PAK C18 MGII, 3 µm, 4.6 mm i.d.×100 mm (manufactured by Shiseido Co., Ltd.)
column temperature: constant temperature near 25°C
mobile phase A: 0.025 mol/L sodium phosphate buffer (pH 6.8)/methanol/acetonitrile mixed solution (14:5:1)
mobile phase B: acetonitrile/0.025 mol/L sodium phosphate buffer (pH 6.8) mixed solution (7:3)

### [Preparation method of 0.025 mol/L sodium phosphate buffer (pH 6.8)]

Potassium dihydrogen phosphate (reagent special grade, Wako Pure Chemical Industries, Ltd.) (3.40 g) and disodium hydrogen phosphate anhydrous (reagent special grade, Wako Pure Chemical Industries, Ltd.) (3.55 g) was dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was diluted 2-fold. It was confirmed that the pH after the dilution was 6.8. When it was not 6.8, phosphoric acid (reagent special grade, Wako Pure Chemical Industries, Ltd.) or 0.1 mol/L sodium hydroxide solution (for volumetric analysis, Wako Pure Chemical Industries, Ltd.) was added to adjust the pH to 6.8.

The concentration gradient of the feed for the mobile phase was controlled by changing the mixing ratio of the mobile phase as follows.

**Table 6**

| time (min) | mobile phase A (%) | mobile phase B (%) |
|---|---|---|
| 0 (injection) | 100 | 0 |
| 10 | 100 | 0 |
| 30 | 50 | 50 |
| 40 | 0 | 100 |
| 45 | 0 | 100 |
| 45.1 | 100 | 0 |
| 55 (injection) | 100 | 0 |

In Comparative Example 2, and Examples 4, 5, the reaction product was measured before and after storage at 60°C for 1 week. The results are shown in Table 7. Addition of sodium chloride or sodium bromide suppressed an increase of the reaction product.

**Table 7**

| Reaction product after storage at 60°C for 1 week | | | | |
|---|---|---|---|---|
| sample | additive | concentration of additive | before storage | after storage |
| | | | reaction product (Rt: about 0.79) (%) | reaction product (Rt: about 0.79) (%) |
| Comp. Ex. 2 | none | - | 0.10 | 0.14 |
| Ex. 4 | sodium chloride | 154 mmol/L | 0.11 | 0.10 |
| Ex. 5 | sodium bromide | 154 mmol/L | 0.10 | 0.11 |

### Example 6

OTSUKA NORMAL SALINE (Otsuka Pharmaceutical Factory, Inc.) was diluted 9-fold with ultrapure water (produced by "ultrapure water production system WRX10 manufactured by YAMATO SCIENTIFIC CO., LTD.", resistance value not less than 15.0 MΩcm) to give a 17 mmol/L aqueous sodium chloride solution. Fumarate of compound A (134 mg) was measured in a glass beaker, 17 mmol/L aqueous sodium chloride solution (100 mL) was added and the fumarate was dissolved by stirring the mixture with a stirrer, whereby a drug solution having the following composition was obtained (pH=3.6, measured using HORIBA pH METER F-52).

| | |
|---|---|
| fumarate of compound A | 134 mg |
| sodium chloride | 100 mg |
| water | 100 mL |

### Example 7

OTSUKA NORMAL SALINE (Otsuka Pharmaceutical Factory, Inc.) was diluted 3-fold with ultrapure water (produced by "ultrapure water production system WRX10 manufactured by YAMATO SCIENTIFIC CO., LTD.", resistance value not less than 15.0 MΩcm) to give a 51 mmol/L aqueous sodium chloride solution. Fumarate of compound A (134 mg) was measured in a glass beaker, 51 mmol/L aqueous sodium chloride solution (100 mL) was added and the fumarate was dissolved by stirring the mixture with a stirrer, whereby a drug solution having the following composition was obtained (pH=3.6, measured using HORIBA pH METER F-52).

| | |
|---|---|
| fumarate of compound A | 134 mg |
| sodium chloride | 300 mg |
| water | 100 mL |

### Example 8

OTSUKA NORMAL SALINE (Otsuka Pharmaceutical Factory, Inc.) was diluted 2-fold with ultrapure water (produced by "ultrapure water production system WRX10 manufactured by YAMATO SCIENTIFIC CO., LTD.", resistance value not less than 15.0 MΩcm) to give a 77 mmol/L aqueous sodium chloride solution. Fumarate of compound A (134 mg) was measured in a glass beaker, 77 mmol/L aqueous sodium chloride solution (100 mL) was added and the fumarate was dissolved by stirring the mixture with a stirrer, whereby a drug solution having the following composition was obtained (pH=3.6, measured using HORIBA pH METER F-52).

| | |
|---|---|
| fumarate of compound A | 134 mg |
| sodium chloride | 450 mg |
| water | 100 mL |

### Example 9

Fumarate of compound A (67 mg) was measured in a glass beaker, OTSUKA NORMAL SALINE (Otsuka Pharmaceutical Factory, Inc.) (50 mL) was added and the fumarate was dissolved by stirring the mixture with a stirrer, whereby a drug solution having the following composition was obtained (pH=3.6, measured using HORIBA pH METER F-52).

| | |
|---|---|
| fumarate of compound A | 67 mg |
| sodium chloride | 450 mg |
| water | 50 mL |

### Experimental Example 3 (measurement method of reaction product)

The drug solutions of Comparative Example 2 and Examples 6 - 9 were each placed by about 5 mL in a glass vial (VIAL 17PC TOKAN, DAIWA SPECIAL GLASS Co., Ltd.), and the vial was tightly sealed, and preserved at 60°C (TABAI PERFECT OVEN-ORIGINAL PV-220, ESPEC CORP.) for 1 week.

In the drug solutions before storage and after storage, production of the reaction product of compound A (relative retention time when elution time of compound A is 1, (Rt): about 0.79) was examined. The reaction product was measured by the HPLC method and using the drug solutions diluted 2.5-fold with an ultrapure water (produced by "ultrapure water production system WRX10 manufactured by YAMATO SCIENTIFIC CO., LTD.", resistance value not less than 15.0 MΩcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (waters 2487 Dual λ Absorbance Detector)
column: CAPCELL PAK C18 MGII, 3 µm, 4.6 mm i.d.×100 mm (manufactured by Shiseido Co., Ltd.)
column temperature: constant temperature near 25°C
mobile phase A: 0.025 mol/L sodium phosphate buffer (pH 6.8)/methanol/acetonitrile mixed solution (14:5:1)
mobile phase B: acetonitrile/0.025 mol/L sodium phosphate buffer (pH 6.8) mixed solution (7:3)

### [Preparation method of 0.025 mol/L sodium phosphate buffer (pH 6.8)]

Potassium dihydrogen phosphate (reagent special grade, Wako Pure Chemical Industries, Ltd.) (3.40 g) and disodium hydrogen phosphate anhydrous (reagent special grade, Wako Pure Chemical Industries, Ltd.) (3.55 g) were dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was diluted 2-fold. It was confirmed that the pH after the dilution was 6.8. When it was not 6.8, phosphoric acid (reagent special grade, Wako Pure Chemical Industries, Ltd.) or 0.1 mol/L sodium hydroxide solution (for volumetric analysis, Wako Pure Chemical Industries, Ltd.) was added to adjust the pH to 6.8.

The concentration gradient of the feed for the mobile phase was controlled by changing the mixing ratio of the mobile phase as follows.

**Table 8**

| time (min) | mobile phase A (%) | mobile phase B (%) |
|---|---|---|
| 0 (injection) | 100 | 0 |
| 10 | 100 | 0 |
| 30 | 50 | 50 |
| 40 | 0 | 100 |
| 45 | 0 | 100 |
| 45.1 | 100 | 0 |
| 55 (injection) | 100 | 0 |

In Comparative Example 2 and Examples 6 - 9, the reaction product was measured before and after storage at 60°C for 1 week. The results are shown in Table 9. An increase of the reaction product was suppressed also in a liquid preparation free of isotonization, irrespective of the concentration of sodium chloride.

**Table 9**

| Reaction product after storage at 60°C for 1 week | | | | |
|---|---|---|---|---|
| sample | additive | concentration of additive | before storage | after storage |
| | | | reaction product (Rt: about 0.79) (%) | reaction product (Rt: about 0.79) (%) |
| Comp. Ex. 2 | none | - | 0.10 | 0.14 |
| Ex. 6 | sodium chloride | 17 mmol/L | 0.11 | 0.12 |
| Ex. 7 | sodium chloride | 51 mmol/L | 0.11 | 0.11 |
| Ex. 8 | sodium chloride | 77 mmol/L | 0.11 | 0.11 |
| Ex. 9 | sodium chloride | 154 mmol/L | 0.11 | 0.10 |

### Comparative Example 3

N-methyl-1-[5-(2-methylphenyl)-1-(pyridin-3-ylsulfonyl)-1H-pyrrol-3-yl]methanamine fumarate (hereinafter to be indicated as fumarate of compound B) (66 mg) was measured in a glass beaker, ultrapure water (produced by "ultrapure water production system WRX10 manufactured by YAMATO SCIENTIFIC CO., LTD.", resistance value not less than 15.0 MΩcm) (50 mL) was added and the fumarate was dissolved by stirring the mixture with a stirrer, whereby a drug solution having the following composition was obtained.

| | |
|---|---|
| fumarate of compound B | 66 mg |
| water | 50 mL |

### Example 10

Fumarate of compound B (66 mg) was measured in a glass beaker, OTSUKA NORMAL SALINE (Otsuka Pharmaceutical Factory, Inc.) (50 mL) was added and the fumarate was dissolved by stirring the mixture with a stirrer, whereby a drug solution having the following composition was obtained.

| | |
|---|---|
| fumarate of compound B | 66 mg |
| sodium chloride | 450 mg |
| water | 50 mL |

### Experimental Example 4 (measurement method of reaction product)

The drug solutions of Comparative Example 3 and Example 10 were each placed by about 5 mL in a glass vial (VIAL 17PC TOKAN, DAIWA SPECIAL GLASS Co., Ltd.), and the vial was tightly sealed, and preserved at 60°C (TABAI PERFECT OVEN-ORIGINAL PV-220, ESPEC CORP.) for 1 week.

Using the drug solutions before and after storage, production of the reaction product of compound B (relative retention time when elution time of compound B is 1, (Rt): about 0.38) was examined. The reaction product was measured by the HPLC method and using the drug solutions diluted 2.5-fold with an ultrapure water (produced by "ultrapure water production system WRX10 manufactured by YAMATO SCIENTIFIC CO., LTD.", resistance value not less than 15.0 MQcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (waters 2487 Dual λ Absorbance Detector)
column: CAPCELL PAK C18 MGII, 3 µm, 4.6 mm i.d.×100 mm (manufactured by Shiseido Co., Ltd.)
column temperature: constant temperature near 25°C
mobile phase A: 0.025 mol/L sodium phosphate buffer (pH 6.8)/methanol/acetonitrile mixed solution (14:5:1)
mobile phase B: acetonitrile/0.025 mol/L sodium phosphate buffer (pH 6.8) mixed solution (7:3)

### [Preparation method of 0.025 mol/L sodium phosphate buffer (pH 6.8)]

Potassium dihydrogen phosphate (reagent special grade, Wako Pure Chemical Industries, Ltd.) (3.40 g) and disodium hydrogen phosphate anhydrous (reagent special grade, Wako Pure Chemical Industries, Ltd.) (3.55 g) were dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was diluted 2-fold. It was confirmed that the pH after the dilution was 6.8. When it was not 6.8, phosphoric acid (reagent special grade, Wako Pure Chemical Industries, Ltd.) or 0.1 mol/L sodium hydroxide solution (for volumetric analysis, Wako Pure Chemical Industries, Ltd.) was added to adjust the pH to 6.8.

The concentration gradient of the feed for the mobile phase was controlled by changing the mixing ratio of the mobile phase as follows.

**Table 10**

| time (min) | mobile phase A (%) | mobile phase B (%) |
|---|---|---|
| 0 (injection) | 100 | 0 |
| 10 | 100 | 0 |
| 30 | 50 | 50 |
| 40 | 0 | 100 |
| 45 | 0 | 100 |
| 45.1 | 100 | 0 |
| 55 (injection) | 100 | 0 |

In Comparative Example 3, and Example 10, production of the reaction product before and after storage at 60°C for 1 week was measured. The results are shown in Table 11. Addition of sodium chloride suppressed an increase of the reaction product.

**Table 11**

| Reaction product after storage at 60°C for 1 week | | | | |
|---|---|---|---|---|
| sample | additive | concentration of additive | before storage | after storage |
| | | | reaction product (Rt: about 0.38) (%) | reaction product (Rt: about 0.38) (%) |
| Comp. Ex. 3 | none | - | 0.06 | 0.12 |
| Ex. 10 | sodium chloride | 154 mmol/L | 0.07 | 0.09 |

### Comparative Example 4

1-[4-Fluoro-5-(2-fluoropyridin-3-yl)-1-(pyridin-3-ylsulfonyl)-1H-pyrrol-3-yl]-N-methylmethanamine 0.5 fumarate (hereinafter to be indicated as 0.5 fumarate of compound C) (106 mg) was measured in a glass beaker, ultrapure water (produced by "ultrapure water production system WRX10 manufactured by YAMATO SCIENTIFIC CO., LTD.", resistance value not less than 15.0 MΩcm) (50 mL) was added and the 0.5 fumarate was dissolved by stirring the mixture with a stirrer, whereby a drug solution having the following composition was obtained.

| | |
|---|---|
| 0.5 fumarate of compound C | 106 mg |
| water | 50 mL |

### Example 11

0.5 Fumarate of compound C (106 mg) was measured in a glass beaker, OTSUKA NORMAL SALINE (Otsuka Pharmaceutical Factory, Inc.) (50 mL) was added and the 0.5 fumarate was dissolved by stirring the mixture with a stirrer, whereby a drug solution having the following composition was obtained.

| | |
|---|---|
| 0.5 fumarate of compound C | 106 mg |
| sodium chloride | 450 mg |
| water | 50 mL |

### Experimental Example 5 (measurement method of reaction product)

The drug solutions of Comparative Example 4 and Example 11 were each placed by about 5 mL in a glass vial (VIAL 17PC TOKAN, DAIWA SPECIAL GLASS Co., Ltd.), and the vial was tightly sealed, and preserved at 60°C (TABAI PERFECT OVEN-ORIGINAL PV-220, ESPEC CORP.) for 2 weeks.

Using the drug solutions of Comparative Example 4 and Example 11 before and after storage, production of the reaction product of compound C (relative retention time when elution time of compound C is 1, (Rt): about 0.8) was examined. The reaction product was measured by the HPLC method and using the drug solutions diluted with a 0.02 mol/L sodium phosphate buffer (pH 7.0)/acetonitrile (for high-performance liquid chromatography, Wako Pure Chemical Industries, Ltd.) mixed solution (2:1). The test conditions of HPLC were as follows.
system: Waters 2690 Separation Module
detector: ultraviolet absorption spectrophotometer (measurement wavelength: 230 nm) (waters 2487 Dual λ Absorbance Detector)
column: Zorbax Eclipse XDB-C18, 5 µm, 4.6 mm i.d.×150 mm (manufactured by Agilent)
column temperature: constant temperature near 25°C
mobile phase A: 0.02 mol/L sodium phosphate buffer (pH 7.0)/acetonitrile mixed solution (19:1)
mobile phase B: acetonitrile/0.02 mol/L sodium phosphate buffer (pH 7.0) mixed solution (3:2)

### [Preparation method of 0.02 mol/L sodium phosphate buffer (pH 7.0)]

Sodium dihydrogen phosphate dihydrate (reagent special grade, Wako Pure Chemical Industries, Ltd.) (3.1 g) was dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was adjusted to pH 7.0 with a solution of disodium hydrogen phosphate dodecahydrate (reagent special grade, Wako Pure Chemical Industries, Ltd.) (7.2 g) dissolved in ultrapure water (produced by "ultrapure water production system WRX10 manufactured by YAMATO SCIENTIFIC CO., LTD.", resistance value not less than 15.0 MΩcm) to give a total volume of 1000 mL.

The concentration gradient of the feed for the mobile phase was controlled by changing the mixing ratio of the mobile phase as follows.

**Table 12**

| time (min) | mobile phase A (%) | mobile phase B (%) |
|---|---|---|
| 0 (injection) | 100 | 0 |
| 5 | 100 | 0 |
| 80 | 0 | 100 |
| 81 | 100 | 0 |
| 90 | 100 | 0 |

In Comparative Example 4, and Example 11, production of the reaction product before and after storage at 60°C for 2 weeks was measured. The results are shown in Table 13. Addition of sodium chloride suppressed an increase of the reaction product.

**Table 13**

| Reaction product after storage at 60°C for 2 weeks | | | | |
|---|---|---|---|---|
| sample | additive | concentration of additive | before storage | after storage |
| | | | reaction product (Rt: about 0.8) (%) | reaction product (Rt: about 0.8) (%) |
| Comp. Ex. 4 | none | - | 0.40 | 0.76 |
| Ex. 11 | sodium chloride | 154 mmol/L | 0.41 | 0.56 |

### Example 12

Fumarate of compound A (134 mg) was measured in a glass beaker, 50 mL of a solution of sodium chloride (reagent special grade, Wako Pure Chemical Industries, Ltd.) (18 g) dissolved in ultrapure water (produced by "ultrapure water production system WRX10 manufactured by YAMATO SCIENTIFIC CO., LTD.", resistance value not less than 15.0 MΩcm) to give a total volume of 1000 mL and 30 mL of ultrapure water were added and the fumarate was dissolved by stirring the mixture with a stirrer. An aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 100 mL, whereby a drug solution having the following composition was obtained.

| | |
|---|---|
| fumarate of compound A | 134 mg |
| sodium chloride | 900 mg |
| aqueous sodium hydroxide solution q.s. (pH was adjusted to 4.0) | |
| water | measured up to 100 mL |

### Experimental Example 6 (measurement method of compound A)

The drug solution of Example 12 (1 mL) diluted by adding to OTSUKA NORMAL SALINE 50 mL PLABOTTLE (Otsuka Pharmaceutical Factory, Inc.) containing 24 mL therein, and that diluted by adding to OTSUKA GLUCOSE INJECTION 5% 50 mL PLABOTTLE (Otsuka Pharmaceutical Factory, Inc.) containing 24 mL therein were each examined for the content of compound A immediately after dilution, 1 hr after dilution, and 24 hr after dilution at room temperature under shading. The content was measured by applying the drug solutions to the HPLC method. The test conditions of HPLC were as follows.
system: Waters 2690 Separation Module
detector: ultraviolet absorption spectrophotometer (measurement wavelength: 230 nm) (waters 2487 Dual λ Absorbance Detector)
column: CAPCELL PAK C18 MGII, 3 µm, 4.6 mm i.d.×100 mm (manufactured by Shiseido Co., Ltd.)
column temperature: constant temperature near 25°C
mobile phase: 0.05 mol/L sodium phosphate buffer (pH 6.8)/methanol/acetonitrile mixed solution (17:6:7)

### [Preparation method of 0.05 mol/L sodium phosphate buffer (pH 6.8)]

Potassium dihydrogen phosphate (reagent special grade, Wako Pure Chemical Industries, Ltd.) (3.40 g) and disodium hydrogen phosphate anhydrous (reagent special grade, Wako Pure Chemical Industries, Ltd.) (3.55 g) were dissolved in ultrapure water (produced by "ultrapure water production system WRX10 manufactured by YAMATO SCIENTIFIC CO., LTD.", resistance value not less than 15.0 MQcm) to give a total volume of 1000 mL. It was confirmed that the pH after the dilution was 6.8. When it was not 6.8, phosphoric acid (reagent special grade, Wako Pure Chemical Industries, Ltd.) or 0.1 mol/L sodium hydroxide solution (for volumetric analysis, Wako Pure Chemical Industries, Ltd.) was added to adjust the pH to 6.8.

The results of the measurement of the content of compound A immediately after dilution, 1 hr after dilution, and 24 hr after dilution of the drug solutions of Example 12 with saline or 5% OTSUKA GLUCOSE INJECTION in the PLABOTTLE (infusion bag) are shown in Table 14. The content did not decrease even by diluting the drug solution in the infusion bag.

The results show that the liquid preparation of the present invention is a solution for injection and, even when combined with an infusion, it is stable without a decrease in the content.

**Table 14**

| Change of drug content after dilution with infusion | | | |
|---|---|---|---|
| | content (immediately after dilution) | content (after dilution for 1 hr) | content (after dilution for 24 hr) |
| OTSUKA NORMAL SALINE | 1.03 mg/mL | 1.02 mg/mL | 1.02 mg/mL |
| OTSUKA GLUCOSE INJECTION 5% | 1.05 mg/mL | 1.05 mg/mL | 1.04 mg/mL |

### Comparative Example 5

A solution of Citric Acid (reagent special grade, Wako Pure Chemical Industries, Ltd.) (3.84 g) diluted with ultrapure water (produced by "ultrapure water production system WRX10 manufactured by YAMATO SCIENTIFIC CO., LTD.", resistance value not less than 15.0 MΩcm) to 1L and a solution of Sodium Citrate Hydrate (Japanese Pharmacopoeia Grade, Wako Pure Chemical Industries, Ltd.) (5.88 g) diluted with ultrapure water to 1L were mixed, the mixture was adjusted to pH 4.0, and fumarate of compound A (134 mg) was dissolved in the resulting buffer (50 mL). An aqueous sodium hydroxide solution (for volumetric analysis, Wako Pure Chemical Industries, Ltd.) was added thereto, and the mixture was adjusted to pH 4.0 using HORIBA pH METER F-52 and measured up to a total amount of 100 mL by adding ultrapure water, whereby a drug solution having the following composition was obtained.

| | |
|---|---|
| fumarate of compound A | 134 mg |
| citrate buffer | 10 mM |
| aqueous sodium hydroxide solution q.s. (pH was adjusted to 4.0) | |
| water | measured up to 100 mL |

### Comparative Example 6

A solution of Citric Acid (reagent special grade, Wako Pure Chemical Industries, Ltd.) (3.84 g) diluted with ultrapure water (produced by "ultrapure water production system WRX10 manufactured by YAMATO SCIENTIFIC CO., LTD.", resistance value not less than 15.0 MΩcm) to 1L and a solution of Sodium Citrate Hydrate (Japanese Pharmacopoeia Grade, Wako Pure Chemical Industries, Ltd.) (5.88 g) diluted with ultrapure water to 1L were mixed, the mixture was adjusted to pH 4.0, and fumaric acid (reagent special grade, Wako Pure Chemical Industries, Ltd.) (100 mg) was dissolved in the resulting buffer (25 mL). Then, fumarate (66.8 mg) of compound A was dissolved therein. An aqueous sodium hydroxide solution (for volumetric analysis, Wako Pure Chemical Industries, Ltd.) was added thereto, and the mixture was adjusted to pH 4.0 using HORIBA pH METER F-52 and measured up to a total amount of 50 mL by adding ultrapure water, whereby a drug solution having the following composition was obtained.

| | |
|---|---|
| fumarate of compound A | 66.8 mg |
| fumaric acid | 100 mg |
| citrate buffer | 10 mM |
| aqueous sodium hydroxide solution q.s. (pH was adjusted to 4.0) | |
| water | measured up to 50 mL |

### Experimental Example 7 (measurement method of reaction product)

The drug solutions of Comparative Examples 5, 6 were each placed by about 8 mL in a glass vial (VIAL 17PC TOKAN, DAIWA SPECIAL GLASS Co., Ltd.), and the vial was tightly sealed, and preserved for 1 week at 60°C (TABAI PERFECT OVEN-ORIGINAL PV-220, ESPEC CORP.).

Using the drug solutions before and after storage, production of the reaction product of compound A (relative retention time when elution time of compound A is 1, (Rt): about 0.79) was examined. The reaction product was measured by the HPLC method and using the drug solutions diluted 2.5-fold with an ultrapure water (produced by "ultrapure water production system WRX10 manufactured by YAMATO SCIENTIFIC CO., LTD.", resistance value not less than 15.0 MΩcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (Waters 2487 Dual λ Absorbance Detector)
column: CAPCELL PAK C18 MGII, 3 µm, 4.6 mm i.d.×150 mm (manufactured by Shiseido Co., Ltd.)
column temperature: constant temperature near 25°C
mobile phase A: 0.05 mol/L sodium phosphate buffer (pH 6.0)/acetonitrile mixed solution (19:1)
mobile phase B: acetonitrile/0.05 mol/L sodium phosphate buffer (pH 6.0) mixed solution (3:2)

### [Preparation method of 0.05 mol/L sodium phosphate buffer (pH 6.0) ]

Disodium hydrogen phosphate (anhydrous) (reagent special grade, Wako Pure Chemical Industries, Ltd.) (7.1 g) was dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was adjusted to pH 6.0 with phosphoric acid (reagent special grade, Wako Pure Chemical Industries, Ltd.).

The concentration gradient of the feed for the mobile phase was controlled by changing the mixing ratio of the mobile phase as follows.

**Table 15**

| time (min) | mobile phase A (%) | mobile phase B (%) |
|---|---|---|
| 0 (injection) | 100 | 0 |
| 20 | 80 | 20 |
| 60 | 70 | 30 |
| 110 | 0 | 100 |
| 110.1 | 100 | 0 |
| 120 (injection) | 100 | 0 |

In Comparative Examples 5, 6, the reaction product was measured before and after storage at 60°C for 1 week. The results are shown in Table 16. Addition of fumaric acid increased the reaction product and could not stabilize the solution.

**Table 16**

| Reaction product after storage at 60°C for 1 week | | | | |
|---|---|---|---|---|
| sample | additive | concentration of additive | before storage | after storage |
| | | | reaction product (Rt: about 0.79) (%) | reaction product (Rt: about 0.79) (%) |
| Comp. Ex. 5 | - | - | 0.13 | 0.20 |
| Comp. Ex. 6 | fumaric acid | 17.2 mmol/L | 0.13 | 0.58 |

### Comparative Example 7

Fumaric acid (reagent special grade, Wako Pure Chemical Industries, Ltd.) (200 mg) was dissolved in ultrapure water (produced by "ultrapure water production system WRX10 manufactured by YAMATO SCIENTIFIC CO., LTD.", resistance value not less than 15.0 MΩcm) to give a total volume of 90 mL and fumarate of compound A (66.8 mg) was dissolved therein. An aqueous sodium hydroxide solution (for volumetric analysis, Wako Pure Chemical Industries, Ltd.) was added thereto and the mixture was adjusted to pH 5.0 using HORIBA pH METER F-52 and ultrapure water was added to measure up to the total amount of 100 mL, whereby a drug solution having the following composition was obtained.

| | |
|---|---|
| fumarate of compound A | 66.8 mg |
| fumaric acid | 200 mg |
| aqueous sodium hydroxide solution q.s. (pH was adjusted to 5.0) | |
| water | measured up to 100 mL |

### Example 13

Fumaric acid (reagent special grade, Wako Pure Chemical Industries, Ltd.) (200 mg) and sodium chloride (reagent special grade, Wako Pure Chemical Industries, Ltd.) (900 mg) were dissolved in ultrapure water (produced by "ultrapure water production system WRX10 manufactured by YAMATO SCIENTIFIC CO., LTD.", resistance value not less than 15.0 MΩcm) to give a total volume of 90 mL and fumarate of compound A (66.8 mg) was dissolved therein. An aqueous sodium hydroxide solution (for volumetric analysis, Wako Pure Chemical Industries, Ltd.) was added thereto and the mixture was adjusted to pH 5.0 using HORIBA pH METER F-52 and ultrapure water was added to measure up to the total amount of 100 mL, whereby a drug solution having the following composition was obtained.

| | |
|---|---|
| fumarate of compound A | 66.8 mg |
| fumaric acid | 200 mg |
| sodium chloride | 900 mg |
| aqueous sodium hydroxide solution q.s. (pH was adjusted to 5.0) | |
| water | measured up to 100 mL |

### Experimental Example 8 (measurement method of reaction product)

The drug solutions of Comparative Example 7 and Example 13 were each placed by about 20 mL in a glass vial (VIAL 35PV TOKAN, DAIWA SPECIAL GLASS Co., Ltd.), and the vial was tightly sealed, and subjected to an autoclave treatment at 123°C (LABO AUTOCLAVE MLS-3780F, SANYO Electric Biomedical Co., Ltd.) for 3 hr and 6 hr.

Using the drug solutions before and after autoclave treatment, production of the reaction product of compound A (relative retention time when elution time of compound A is 1, (Rt): about 0.79) was examined. The reaction product was measured by the HPLC method and using the drug solutions diluted 1.7-fold with an ultrapure water (produced by "ultrapure water production system WRX10 manufactured by YAMATO SCIENTIFIC CO., LTD.", resistance value not less than 15.0 MΩcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (waters 2487 Dual λ Absorbance Detector)
column: CAPCELL PAK C18 MGII, 3 µm, 4.6 mm i.d.×100 mm (manufactured by Shiseido Co., Ltd.)
column temperature: constant temperature near 25°C
mobile phase A: 0.025 mol/L sodium phosphate buffer (pH 6.8)/methanol/acetonitrile mixed solution (14:5:1)
mobile phase B: acetonitrile/0.025 mol/L sodium phosphate buffer (pH 6.8) mixed solution (7:3)

### [Preparation method of 0.025 mol/L sodium phosphate buffer (pH 6.8)]

Potassium dihydrogen phosphate (reagent special grade, Wako Pure Chemical Industries, Ltd.) (3.40 g) and disodium hydrogen phosphate anhydrous (reagent special grade, Wako Pure Chemical Industries, Ltd.) (3.55 g) were dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was diluted 2-fold. It was confirmed that the pH after the dilution was 6.8. When it was not 6.8, phosphoric acid (reagent special grade, Wako Pure Chemical Industries, Ltd.) or 0.1 mol/L sodium hydroxide solution (for volumetric analysis, Wako Pure Chemical Industries, Ltd.) was added to adjust the pH to 6.8.

The concentration gradient of the feed for the mobile phase was controlled by changing the mixing ratio of the mobile phase as follows.

**Table 17**

| time (min) | mobile phase A (%) | mobile phase B (%) |
|---|---|---|
| 0 (injection) | 100 | 0 |
| 10 | 100 | 0 |
| 30 | 50 | 50 |
| 40 | 0 | 100 |
| 45 | 0 | 100 |
| 45.1 | 100 | 0 |
| 55 (injection) | 100 | 0 |

In Comparative Example 7 and Example 13, the reaction product was measured before and after the autoclave treatment for 3 hr and 6 hr. The results are shown in Table 18. An increase of the reaction product that increases by the addition of fumaric acid was suppressed by the addition of sodium chloride.

**Table 18**

| Reaction product after autoclave treatment at 123°C | | | | | |
|---|---|---|---|---|---|
| sample | additive | concentration of additive | before treatment | after treatment for 3 hr | after treatment for 6 hr |
| | | | reaction product (Rt: about 0.79) (%) | reaction product (Rt: about 0.79) (%) | reaction product (Rt: about 0.79) (%) |
| Comp. Ex. 7 | fumaric acid | 17.2 mmol/L | 0.18 | 0.71 | 1.13 |
| Ex. 13 | fumaric acid+sodium chloride | 17.2 mmol/L+ 154 mmol/L | 0.16 | 0.43 | 0.64 |

### Example 14

Citric Acid Hydrate (Japanese Pharmacopoeia Grade, San-Ei Gen F.F.I., Inc.) (263 mg), Sodium Citrate Hydrate (Japanese Pharmacopoeia Grade, San-Ei Gen F.F.I., Inc.) (221 mg), sodium chloride (reagent special grade, Wako Pure Chemical Industries, Ltd.) (1800 mg) and fumarate of compound A (267.2 mg) were dissolved in about 180 mL of ultrapure water (produced by "ultrapure water production system WRX10 manufactured by YAMATO SCIENTIFIC CO., LTD.", resistance value not less than 15.0 MΩcm). An aqueous sodium hydroxide solution (for volumetric analysis, Wako Pure Chemical Industries, Ltd.) was added thereto, and the mixture was adjusted to pH 3.8 using HORIBA pH METER F-52 and measured up to a total amount of 200 mL by adding ultrapure water, whereby a drug solution having the following composition was obtained.

| | |
|---|---|
| fumarate of compound A | 267.2 mg |
| citrate buffer | 10 mM |
| sodium chloride | 1800 mg |
| aqueous sodium hydroxide solution | q.s. (pH was adjusted to 3.8) |
| water | measured up to 200 mL |

### Experimental Example 9 (measurement method of compound A)

The drug solution of Example 14 (5 mL) diluted by adding to OTSUKA NORMAL SALINE 50 mL PLABOTTLE (Otsuka Pharmaceutical Factory, Inc.) containing 45 mL therein, and that diluted by adding to OTSUKA GLUCOSE INJECTION 5% 50 mL PLABOTTLE (Otsuka Pharmaceutical Factory, Inc.) containing 45 mL therein were each examined for changes of the concentration of compound A immediately after dilution and 6 hr after dilution at room temperature under about 1500 lux. The content was measured by applying the drug solutions to the HPLC method. The test conditions of HPLC were as follows.
system: Waters 2690 Separation Module
detector: ultraviolet absorption spectrophotometer (measurement wavelength: 230 nm) (Waters 2487 Dual λ Absorbance Detector)
column: CAPCELL PAK C18 MGII, 3 µm, 4.6 mm i.d.×100 mm (manufactured by Shiseido Co., Ltd.)
column temperature: constant temperature near 25°C
mobile phase A: 0.025 mol/L sodium phosphate buffer (pH 6.8)/methanol/acetonitrile mixed solution (14:5:1)
mobile phase B: acetonitrile/0.025 mol/L sodium phosphate buffer (pH 6.8) mixed solution (7:3)

### [Preparation method of 0.025 mol/L sodium phosphate buffer (pH 6.8)]

Potassium dihydrogen phosphate (reagent special grade, Wako Pure Chemical Industries, Ltd.) (3.40 g) and disodium hydrogen phosphate anhydrous (reagent special grade, Wako Pure Chemical Industries, Ltd.) (3.55 g) were dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was diluted 2-fold. It was confirmed that the pH after the dilution was 6.8. When it was not 6.8, phosphoric acid (reagent special grade, Wako Pure Chemical Industries, Ltd.) or 0.1 mol/L sodium hydroxide solution (for volumetric analysis, Wako Pure Chemical Industries, Ltd.) was added to adjust the pH to 6.8.

The concentration gradient of the feed for the mobile phase was controlled by changing the mixing ratio of the mobile phase as follows.

**Table 19**

| time (min) | mobile phase A (%) | mobile phase B (%) |
|---|---|---|
| 0 (injection) | 100 | 0 |
| 10 | 100 | 0 |
| 30 | 50 | 50 |
| 40 | 0 | 100 |
| 45 | 0 | 100 |
| 45.1 | 100 | 0 |
| 55 (injection) | 100 | 0 |

The results of the measurement of the changes of the concentration of compound A immediately after dilution and 6 hr after dilution of the drug solutions of Example 14 with saline or 5% OTSUKA GLUCOSE INJECTION in the PLABOTTLE (infusion bag) are shown in Table 20. The concentration did not decrease even by diluting the drug solution in the infusion bag.

**Table 20**

| Changes of drug content after dilution with infusion | | |
|---|---|---|
| | content (immediately after dilution) | content (after dilution for 6 hr) |
| OTSUKA NORMAL SALINE | 100% | 99.0% |
| OTSUKA GLUCOSE INJECTION 5% | 100% | 98.8% |

### Example 15

Fumarate of compound A (80 mg) and sodium chloride (reagent special grade, Wako Pure Chemical Industries, Ltd.) (840 mg) were measured in a glass beaker, ultrapure water (produced by "ultrapure water production system WRX10 manufactured by YAMATO SCIENTIFIC CO., LTD.", resistance value not less than 15.0 MΩcm) (50 mL) was added and they were dissolved by stirring the mixture with a stirrer. A 5 mol/L aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 60 mL, whereby a drug solution having the following composition was obtained.

| | |
|---|---|
| fumarate of compound A | 80 mg |
| sodium chloride | 840 mg |
| 5 mol/L aqueous sodiumhydroxide solution | q.s. (pH was adjusted to 4.0) |
| water | measured up to 60 mL |

### Example 16

Fumarate of compound A (80 mg) and sodium chloride (reagent special grade, Wako Pure Chemical Industries, Ltd.) (1080 mg) were measured in a glass beaker, ultrapure water (produced by "ultrapure water production system WRX10 manufactured by YAMATO SCIENTIFIC CO., LTD.", resistance value not less than 15.0 MΩcm) (50 mL) was added and they were dissolved by stirring the mixture with a stirrer. A 5 mol/L aqueous sodium hydroxide solution (for volumetric analysis, Wako Pure Chemical Industries, Ltd.) was added, and the mixture was adjusted to pH 4.0 using HORIBA pH METER F-52 and ultrapure water was added to measure up to the total amount of 60 mL, whereby a drug solution having the following composition was obtained.

| | |
|---|---|
| fumarate of compound A | 80 mg |
| sodium chloride | 1080 mg |
| 5 mol/L aqueous sodium hydroxide solution | q.s. (pH was adjusted to 4.0) |
| water | measured up to 60 mL |

### Experimental Example 10 (measurement method of reaction product)

The drug solutions of Comparative Example 1 and Examples 1, 15 and 16 were each placed by about 10 mL in a glass vial (VIAL 17PC TOKAN, DAIWA SPECIAL GLASS Co., Ltd.), and the vial was tightly sealed, and preserved for 1 week at 70°C (TEMP.&HUMID. CHAMBER PR-4S, ESPEC CORP.).

Using the drug solutions before and after storage, production of the reaction product of compound A (relative retention time when elution time of compound A is 1, (Rt): about 0.79) was examined. The reaction product was measured by the HPLC method and using the drug solutions diluted 2.5-fold with an ultrapure water (produced by "ultrapure water production system WRX10 manufactured by YAMATO SCIENTIFIC CO., LTD.", resistance value not less than 15.0 MΩcm)/acetonitrile (for high-performance liquid chromatography, Wako Pure Chemical Industries, Ltd.) mixed solution (19:1). The test conditions of HPLC were as follows.
system: Waters 2690 Separation Module
detector: ultraviolet absorption spectrophotometer (measurement wavelength: 230 nm) (waters 2487 Dual λ Absorbance Detector)
column: CAPCELL PAK C18 MGII, 3 µm, 4.6 mm i.d.×150 mm (manufactured by Shiseido Co., Ltd.)
column temperature: constant temperature near 25°C
mobile phase A: 0.05 mol/L sodium phosphate buffer (pH 6.0)/acetonitrile mixed solution (19:1)
mobile phase B: acetonitrile/0.05 mol/L sodium phosphate buffer (pH 6.0) mixed solution (3:2)

### [Preparation method of 0.05 mol/L sodium phosphate buffer (pH 6.0) ]

Disodium hydrogen phosphate (anhydrous) (reagent special grade, Wako Pure Chemical Industries, Ltd.) (7.1 g) was dissolved in ultrapure water (produced by "ultrapure water production system WRX10 manufactured by YAMATO SCIENTIFIC CO., LTD.", resistance value not less than 15.0 MΩcm) to give a total volume of 1000 mL, and the solution was adjusted to pH 6.0 with phosphoric acid (reagent special grade, Wako Pure Chemical Industries, Ltd.).

The concentration gradient of the feed for the mobile phase was controlled by changing the mixing ratio of the mobile phase as follows.

**Table 21**

| time (min) | mobile phase A (%) | mobile phase B (%) |
|---|---|---|
| 0 (injection) | 100 | 0 |
| 20 | 80 | 20 |
| 60 | 70 | 30 |
| 110 | 0 | 100 |
| 110.1 | 100 | 0 |
| 120 (injection) | 100 | 0 |

In Comparative Example 1 and Examples 1, 15, 16, the reaction product was measured before and after storage at 70°C for 1 week. The results are shown in Table 22. An increase of the reaction product was suppressed also in a liquid preparation free of isotonization, irrespective of the concentration of sodium chloride.

**Table 22**

| Reaction product after storage at 70°C for 1 week | | | | |
|---|---|---|---|---|
| sample | additive | concentration of additive | before storage | after storage |
| | | | reaction product (Rt: about 0.79) (%) | reaction product (Rt: about 0.79) (%) |
| Comp. Ex. 1 | none | - | 0.13 | 0.25 |
| Ex. 1 | sodium chloride | 154 mmol/L | 0.13 | 0.18 |
| Ex. 15 | sodium chloride | 240 mmol/L | 0.13 | 0.17 |
| Ex. 16 | sodium chloride | 308 mmol/L | 0.13 | 0.16 |

The present invention is based on the finding that, in the production of a liquid preparation using an organic acid salt compound of a pharmaceutically active ingredient having a primary or secondary amino group as defined above wherein the amino group does not constitute a part of the amide structure as a starting material, addition of a salt as defined above suppresses production of a reaction product of the pharmaceutically active ingredient having a primary or secondary amino group as defined above and the organic acid liberated in the liquid. Since the present invention has found, for the first time, that a salt as defined above has a "suppressive action on the production of a reaction product of a pharmaceutically active ingredient having a primary or secondary amino group as defined above and an organic acid as defined above in a liquid preparation", namely, a "stabilizing action on a liquid preparation containing a pharmaceutically active ingredient having a primary or secondary amino group and an organic acid" as defined above, it can provide a liquid preparation wherein the amount of a reaction product of the pharmaceutically active ingredient having a primary or secondary amino group and the liberated organic acid is controlled by a salt, which is produced from an organic acid salt compound of the pharmaceutically active ingredient and a salt as starting materials as defined above.

## Claims

1. A liquid preparation comprising a pharmaceutically active ingredient, an organic acid and a salt,
wherein a content of a reaction product of the pharmaceutically active ingredient and the organic acid in the preparation is not more than 5%,
wherein the pharmaceutically active ingredient is 1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or
1-[4-fluoro-5-(2-fluoropyridin-3-yl)-1-(pyridin-3-ylsulfonyl)-1H-pyrrol-3-yl]-N-methylmethanamine,
wherein the organic acid is a compound represented by the formula (IV): 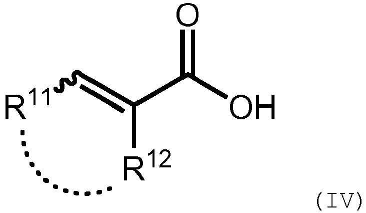 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
wherein the salt is one or more kinds selected from the group consisting of sodium chloride, calcium chloride, magnesium chloride, sodium bromide and calcium bromide,
wherein the reaction product is a compound represented by the formula (V) or (V'): 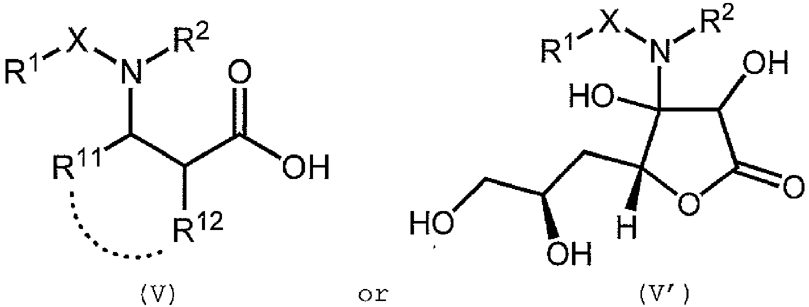 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.

2. The liquid preparation according to claim 1, which is a solution for injection.

3. The liquid preparation according to claim 1, comprising the reaction product of the pharmaceutically active ingredient and the organic acid at not more than 1.8-fold % after storage at 70°C for 1 week than before the storage.

4. The liquid preparation according to claim 1, comprising the reaction product of the pharmaceutically active ingredient and the organic acid at not more than 1.3-fold % after storage at 60°C for 1 week than before the storage.

5. A method of producing the liquid preparation according to claim 1, comprising a step of dissolving or suspending an organic acid salt of the pharmaceutically active ingredient, and the salt in a solvent.

6. The liquid preparation according to claim 1, wherein the organic acid is one or more kinds selected from the group consisting of ascorbic acid, benzoic acid, sorbic acid, fumaric acid and maleic acid.

7. The liquid preparation according to claim 1, wherein the pH is a physiologically acceptable pH.

8. The liquid preparation according to claim 1, wherein the pH is about 3.0 to about 5.0.

9. The liquid preparation according to claim 1, wherein the pharmaceutically active ingredient and the organic acid are contained at a molar ratio of 1:0.001 - 1:1000.

10. The liquid preparation according to claim 1, wherein the pharmaceutically active ingredient and the salt are contained at a molar ratio of 1:0.001 - 1:10000.

11. The liquid preparation according to claim 1, wherein the pharmaceutically active ingredient has a concentration of 0.1 - 100 mg/mL.

12. The liquid preparation according to claim 1, for use in the prophylaxis or treatment of gastric ulcer accompanied by bleeding, duodenal ulcer, acute stress ulcer or acute stomach mucosal lesion.

13. A freeze-dried preparation obtained by freeze-drying the liquid preparation according to claim 1.

14. An injection kit comprising the solution for injection according to claim 2 and an infusion in combination.

15. An injection kit comprising the freeze-dry preparation according to claim 13 and an infusion in combination.

16. A method of stabilizing a liquid preparation, comprising adding a salt to a composition containing a pharmaceutically active ingredient and an organic acid,
wherein the pharmaceutically active ingredient is 1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or
1-[4-fluoro-5-(2-fluoropyridin-3-yl)-1-(pyridin-3-ylsulfonyl)-1H-pyrrol-3-yl]-N-methylmethanamine,
wherein the organic acid is a compound represented by the formula (IV): 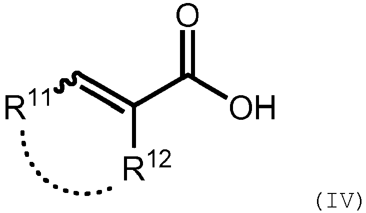 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
wherein the salt is one or more kinds selected from the group consisting of sodium chloride, calcium chloride, magnesium chloride, sodium bromide and calcium bromide.

17. A method of suppressing the production of a reaction product of a pharmaceutically active ingredient and an organic acid, comprising adding a salt to a composition containing the pharmaceutically active ingredient and the organic acid, wherein the pharmaceutically active ingredient is
1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or
1-[4-fluoro-5-(2-fluoropyridin-3-yl)-1-(pyridin-3-ylsulfonyl)-1H-pyrrol-3-yl]-N-methylmethanamine,
wherein the organic acid is a compound represented by the formula (IV): 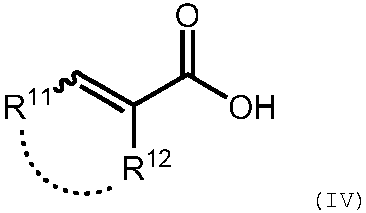 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
wherein the salt is one or more kinds selected from the group consisting of sodium chloride, calcium chloride, magnesium chloride, sodium bromide and calcium bromide,
wherein the reaction product is a compound represented by the formula (V) or (V'): 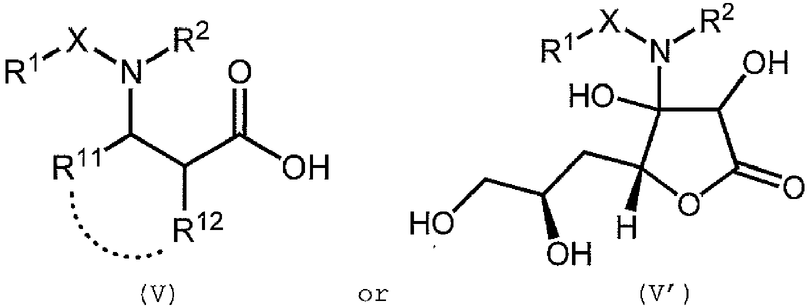 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.

18. Use of a salt as a stabilizer in a liquid preparation comprising a pharmaceutically active ingredient and an organic acid,
wherein a content of a reaction product of the pharmaceutically active ingredient and the organic acid in the preparation is not more than 5%,
wherein the pharmaceutically active ingredient is
1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine, N-methyl-1-[5-(4-methyl-3-thienyl)-1-(pyridin-3-ylsulfonyl)-1H-pyrrol-3-yl]methanamine,
1-[5- (2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or
1-[4-fluoro-5-(2-fluoropyridin-3-yl)-1-(pyridin-3-ylsulfonyl)-1H-pyrrol-3-yl]-N-methylmethanamine,
wherein the organic acid is a compound represented by the formula (IV): 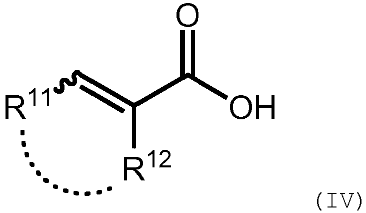 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
wherein the salt is one or more kinds selected from the group consisting of sodium chloride, calcium chloride, magnesium chloride, sodium bromide and calcium bromide,
wherein the reaction product is a compound represented by the formula (V) or (V'): 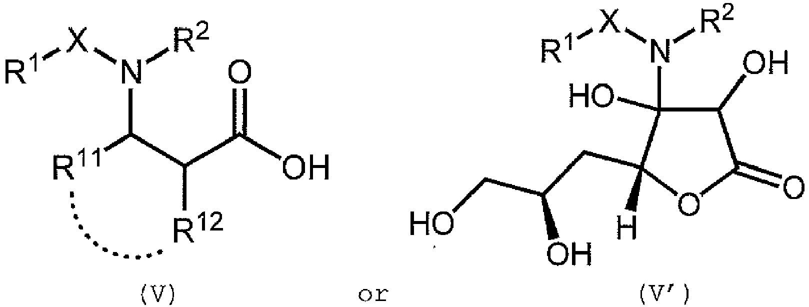 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.

19. Use of a salt for the production of a stabilized liquid preparation comprising a pharmaceutically active ingredient and an organic acid,
wherein a content of a reaction product of the pharmaceutically active ingredient and the organic acid in the preparation is not more than 5%,
wherein the pharmaceutically active ingredient is 1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-lH-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or
1-[4-fluoro-5-(2-fluoropyridin-3-yl)-1-(pyridin-3-ylsulfonyl)-1H-pyrrol-3-yl]-N-methylmethanamine,
wherein the organic acid is a compound represented by the formula (IV): 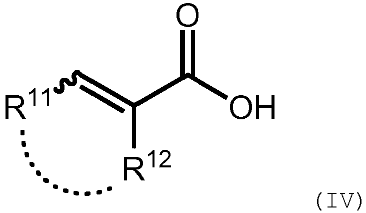 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
wherein the salt is one or more kinds selected from the group consisting of sodium chloride, calcium chloride, magnesium chloride, sodium bromide and calcium bromide,
wherein the reaction product is a compound represented by the formula (V) or (V'): 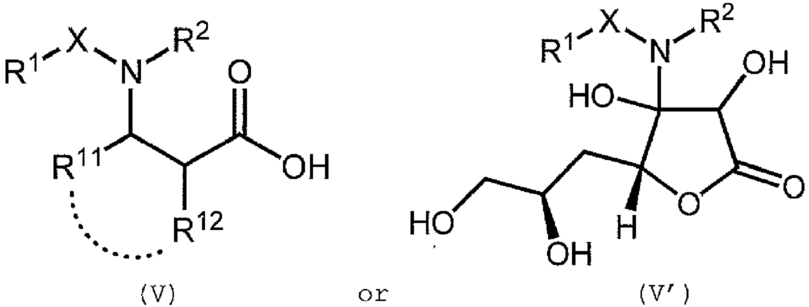 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.

20. A liquid preparation produced from an organic acid salt compound of a pharmaceutically active ingredient, and a salt as starting materials, wherein the amount of a reaction product of the pharmaceutically active ingredient and the liberated organic acid is suppressed by the salt,
wherein the pharmaceutically active ingredient is 1-{5-(2-fluorophenyl)-1-[(6-methylpyridin-3-yl)sulfonyl]-1H-pyrrol-3-yl}-N-methylmethanamine,
1-[4-fluoro-5-phenyl-1-(pyridin-3-ylsulfonyl)-1H-pyrrol-3-yl]-N-methylmethanamine,
N-methyl-1-[5-(4-methyl-3-thienyl)-1-(pyridin-3-ylsulfonyl)-1H-pyrrol-3-yl]methanamine,
1-[5-(2-fluorophenyl)-1-(pyridin-3-ylsulfonyl)-1H-pyrrol-3-yl]-N-methylmethanamine,
N-methyl-1-[5-(2-methylphenyl)-1-(pyridin-3-ylsulfonyl)-1H-pyrrol-3-yl]methanamine,
1-{4-fluoro-5-(2-fluoropyridin-3-yl)-1-[(4-methylpyridin-2-yl)sulfonyl]-1H-pyrrol-3-yl}-N-methylmethanamine, or 1-[4-fluoro-5-(2-fluoropyridin-3-yl)-1-(pyridin-3-ylsulfonyl)-1H-pyrrol-3-yl]-N-methylmethanamine,
wherein the organic acid is a compound represented by the formula (IV): 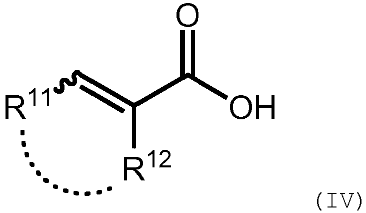 wherein R¹¹ and R¹² are the same or different and each is a hydrogen atom, an optionally substituted hydrocarbon group, a carboxyl group, a halogen atom, a C₁₋₆ alkoxy-carbonyl group or a C₁₋₆ alkoxy group, or R¹¹ and R¹² jointly form an optionally substituted ring, or ascorbic acid, and
wherein the salt is one or more kinds selected from the group consisting of sodium chloride, calcium chloride, magnesium chloride, sodium bromide and calcium bromide,
wherein the reaction product is a compound represented by the formula (V) or (V'): 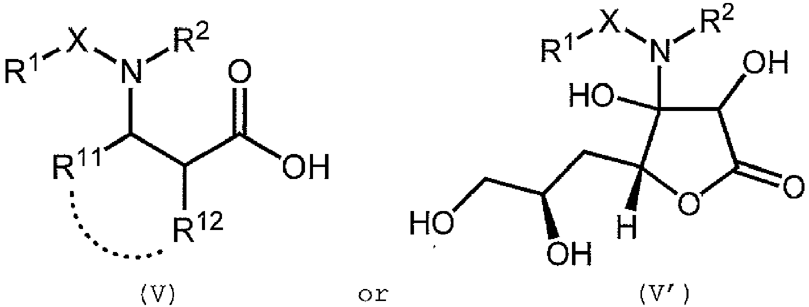 wherein R¹¹ and R¹² are as defined above; R¹ is 5-(2-fluorophenyl)-1-[(6-methylpyridin-3-yl)sulfonyl]-1H-pyrrol-3-yl, 4-fluoro-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-fluorophenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-fluoro-5-(2-fluoropyridin-3-yl)-1-[(4-methylpyridin-2-yl)sulfonyl]-1H-pyrrol-3-yl, or 4-fluoro-5-(2-fluoropyridin-3-yl)-1-(pyridin-3-ylsulfonyl)-1H-pyrrol-3-yl; R² is methyl; and X is -CH₂-.

## Patentansprüche

1. Eine flüssige Zubereitung, umfassend einen pharmazeutisch wirksamen Bestandteil, eine organische Säure und ein Salz,
wobei der Gehalt eines Reaktionsprodukts des pharmazeutisch wirksamen Bestandteils und der organischen Säure in der Zubereitung nicht mehr als 5% beträgt,
wobei der pharmazeutisch wirksame Bestandteil
1-{5-(2-Fluorphenyl)-1-[(6-methylpyridin-3-yl)sulfonyl]-1H-pyrrol-3-yl}-N-methylmethanamin,
1-[4-Fluor-5-phenyl-1-(pyridin-3-ylsulfonyl)-1H-pyrrol-3-yl]-N-methylmethanamin,
N-Methyl-1-[5-(4-methyl-3-thienyl)-1-(pyridin-3-ylsulfonyl)-1H-pyrrol-3-yl]methanamin,
1-[5-(2-Fluorphenyl)-1-(pyridin-3-ylsulfonyl)-1H-pyrrol-3-yl]-N-methylmethanamin,
N-Methyl-1-[5-(2-methylphenyl)-1-(pyridin-3-ylsulfonyl)-1H-pyrrol-3-yl]methanamin,
1-{4-Fluor-5-(2-fluorpyridin-3-yl)-1-[(4-methylpyridin-2-yl)sulfonyl]-1H-pyrrol-3-yl}-N-methylmethanamin, oder
1-[4-Fluor-5-(2-fluorpyridin-3-yl)-1-(pyridin-3-ylsulfonyl)-1H-pyrrol-3-yl]-N-methylmethanamin, ist,
wobei die organische Säure eine Verbindung ist, die durch die Formel (IV) dargestellt wird: 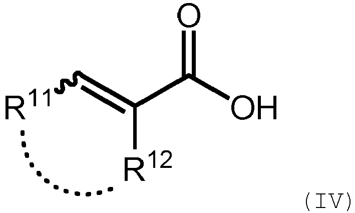 wobei R¹¹ und R¹² gleich oder verschieden sind und jeweils ein Wasserstoffatom, eine gegebenenfalls substituierte Kohlenwasserstoffgruppe, eine Carboxylgruppe, ein Halogenatom, eine C₁₋₆-Alkoxycarbonylgruppe oder eine C₁₋₆-Alkoxygruppe sind, oder R¹¹ und R¹² zusammen einen gegebenenfalls substituierten Ring bilden, oder Ascorbinsäure, und
wobei das Salz eine oder mehrere Arten ist, ausgewählt aus der Gruppe bestehend aus Natriumchlorid, Calciumchlorid, Magnesiumchlorid, Natriumbromid und Calciumbromid,
wobei das Reaktionsprodukt eine Verbindung ist, die durch die Formel (V) oder (V') dargestellt wird: 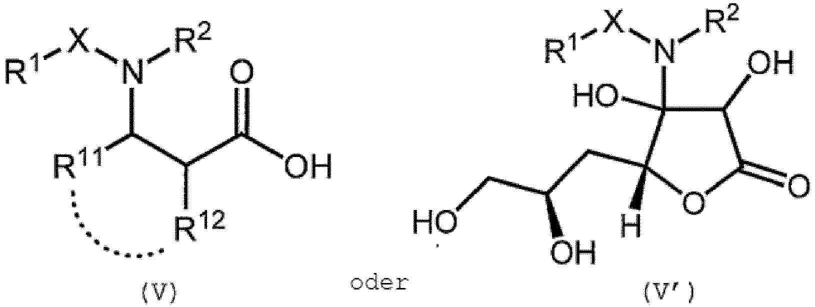 wobei R¹¹ und R¹² wie oben definiert sind; R¹ 5-(2-Fluorphenyl)-1-[(6-methylpyridin-3-yl)sulfonyl]-1H-pyrrol-3-yl, 4-Fluor-5-phenyl-1-(pyridin-3-ylsulfonyl)-1H-pyrrol-3-yl, 5-(4-methyl-3-thienyl)-1-(pyridin-3-ylsulfonyl)-1H-pyrrol-3-yl, 5-(2-Fluorphenyl)-1-(pyridin-3-ylsulfonyl)-1H-pyrrol-3-yl, 5-(2-methylphenyl)-1-(pyridin-3-ylsulfonyl)-1H-pyrrol-3-yl, 4-Fluor-5-(2-fluorpyridin-3-yl)-1-[(4-methylpyridin-2-yl)sulfonyl]-1H-pyrrol-3-yl, oder 4-Fluor-5-(2-fluorpyridin-3-yl)-1-(pyridin-3-ylsulfonyl)-1H-pyrrol-3-yl ist; R² ist Methyl; und X ist -CH₂-.

2. Die flüssige Zubereitung nach Anspruch 1, die eine Lösung zur Injektion ist.

3. Die flüssige Zubereitung nach Anspruch 1, umfassend das Reaktionsprodukt des pharmazeutisch wirksamen Bestandteils und der organischen Säure zu nicht mehr als dem 1,8-fachen % nach Lagerung bei 70° C für 1 Woche als vor der Lagerung.

4. Die flüssige Zubereitung nach Anspruch 1, umfassend das Reaktionsprodukt des pharmazeutisch wirksamen Bestandteils und der organischen Säure zu nicht mehr als dem 1,3-fachen % nach Lagerung bei 60° C für 1 Woche als vor der Lagerung.

5. Ein Verfahren zur Herstellung der flüssigen Zubereitung nach Anspruch 1, umfassend einen Schritt des Lösens oder Suspendierens eines Salzes aus einer organischen Säure und des pharmazeutisch wirksamen Bestandteils, und des Salzes in einem Lösungsmittel.

6. Die flüssige Zubereitung nach Anspruch 1, wobei die organische Säure eine oder mehrere Arten ist, ausgewählt aus der Gruppe bestehend aus Ascorbinsäure, Benzoesäure, Sorbinsäure, Fumarsäure und Maleinsäure.

7. Die flüssige Zubereitung nach Anspruch 1, wobei der pH-Wert ein physiologisch geeigneter pH-Wert ist.

8. Die flüssige Zubereitung nach Anspruch 1, wobei der pH-Wert etwa 3,0 bis etwa 5,0 beträgt.

9. Die flüssige Zubereitung nach Anspruch 1, wobei der pharmazeutisch wirksame Bestandteil und die organische Säure in einem molaren Verhältnis von 1:0,001 - 1:1000 enthalten sind.

10. Flüssige Zubereitung nach Anspruch 1, wobei der pharmazeutisch wirksame Bestandteil und das Salz in einem molaren Verhältnis von 1:0,001 - 1:10000 enthalten sind.

11. Die flüssige Zubereitung nach Anspruch 1, wobei der pharmazeutisch wirksame Bestandteil eine Konzentration von 0,1 - 100 mg/mL aufweist.

12. Die flüssige Zubereitung nach Anspruch 1 zur Verwendung in der Prophylaxe oder Behandlung von Magengeschwüren, die von Blutungen, Zwölffingerdarmgeschwüren, akuten Belastungsgeschwüren oder akuten Magenschleimhautverletzungen begleitet sind.

13. Eine gefriergetrocknete Zubereitung, erhalten durch Gefriertrocknung der flüssigen Zubereitung nach Anspruch 1.

14. Ein Injektions-Set, umfassend die Lösung zur Injektion nach Anspruch 2 und eine Infusion in Kombination.

15. Ein Injektions-Set, umfassend die gefriergetrocknete Zubereitung nach Anspruch 13 und eine Infusion in Kombination.

16. Ein Verfahren zur Stabilisierung eines flüssigen Präparats, umfassend die Zugabe eines Salzes zu einer Zusammensetzung, die einen pharmazeutisch wirksamen Bestandteil und eine organische Säure enthält,
wobei der pharmazeutisch wirksame Bestandteil
1-{5-(2-Fluorphenyl)-1-[(6-methylpyridin-3-yl)sulfonyl]-1H-pyrrol-3-yl}-N-methylmethanamin,
1-[4-Fluor-5-phenyl-1-(pyridin-3-ylsulfonyl)-1H-pyrrol-3-yl]-N-methylmethanamin,
N-Methyl-1-[5-(4-methyl-3-thienyl)-1-(pyridin-3-ylsulfonyl)-1H-pyrrol-3-yl]methanamin,
1-[5-(2-Fluorphenyl)-1-(pyridin-3-ylsulfonyl)-1H-pyrrol-3-yl]-N-methylmethanamin,
N-Methyl-1-[5-(2-methylphenyl)-1-(pyridin-3-ylsulfonyl)-1H-pyrrol-3-yl]methanamin,
1-{4-Fluor-5-(2-fluorpyridin-3-yl)-1-[(4-methylpyridin-2-yl)sulfonyl]-1H-pyrrol-3-yl}-N-methylmethanamin, oder
1-[4-Fluoro-5-(2-fluorpyridin-3-yl)-1-(pyridin-3-ylsulfonyl)-1H-pyrrol-3-yl]-N-methylmethanamin, ist,
wobei die organische Säure eine Verbindung ist, die durch die Formel (IV) dargestellt wird: 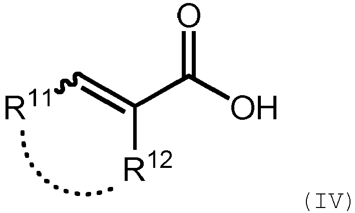 wobei R¹¹ und R¹² gleich oder verschieden sind und jeweils ein Wasserstoffatom, eine gegebenenfalls substituierte Kohlenwasserstoffgruppe, eine Carboxylgruppe, ein Halogenatom, eine C₁₋₆-Alkoxycarbonylgruppe oder eine C₁₋₆-Alkoxygruppe sind, oder R¹¹ und R¹² zusammen einen gegebenenfalls substituierten Ring bilden, oder Ascorbinsäure, und
wobei das Salz eine oder mehrere Arten ist, ausgewählt aus der Gruppe bestehend aus Natriumchlorid, Calciumchlorid, Magnesiumchlorid, Natriumbromid und Calciumbromid.

17. Ein Verfahren zur Unterdrückung der Herstellung eines Reaktionsprodukts aus einem pharmazeutisch wirksamen Bestandteil und einer organischen Säure, umfassend die Zugabe eines Salzes zu einer Zusammensetzung, die den pharmazeutisch wirksamen Bestandteil und die organische Säure enthält,
wobei der pharmazeutisch wirksame Bestandteil 1-{5-(2-Fluorphenyl)-1-[(6-methylpyridin-3-yl)sulfonyl]-1H-pyrrol-3-yl}-N-methylmethanamin,
1-[4-Fluor-5-phenyl-1-(pyridin-3-ylsulfonyl)-1H-pyrrol-3-yl]-N-methylmethanamin,
N-Methyl-1-[5-(4-methyl-3-thienyl)-1-(pyridin-3-ylsulfonyl)-1H-pyrrol-3-yl]methanamin,
1-[5-(2-Fluorphenyl)-1-(pyridin-3-ylsulfonyl)-1H-pyrrol-3-yl]-N-methylmethanamin,
N-Methyl-1-[5-(2-methylphenyl)-1-(pyridin-3-ylsulfonyl)-1H-pyrrol-3-yl]methanamin,
1-{4-Fluor-5-(2-fluorpyridin-3-yl)-1-[(4-methylpyridin-2-yl)sulfonyl]-1H-pyrrol-3-yl}-N-methylmethanamin, oder
1-[4-Fluor-5-(2-fluorpyridin-3-yl)-1-(pyridin-3-ylsulfonyl)-1H-pyrrol-3-yl]-N-methylmethanamin, ist,
wobei die organische Säure eine Verbindung ist, die durch die Formel (IV) dargestellt wird: 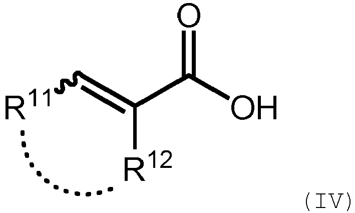 wobei R¹¹ und R¹² gleich oder verschieden sind und jeweils ein Wasserstoffatom, eine gegebenenfalls substituierte Kohlenwasserstoffgruppe, eine Carboxylgruppe, ein Halogenatom, eine C₁₋₆-Alkoxycarbonylgruppe oder eine C₁₋₆-Alkoxygruppe sind, oder R¹¹ und R¹² zusammen einen gegebenenfalls substituierten Ring bilden, oder Ascorbinsäure, und
wobei das Salz eine oder mehrere Arten ist, ausgewählt aus der Gruppe bestehend aus Natriumchlorid, Calciumchlorid, Magnesiumchlorid, Natriumbromid und Calciumbromid,
wobei das Reaktionsprodukt eine Verbindung ist, die durch die Formel (V) oder (V') dargestellt wird: 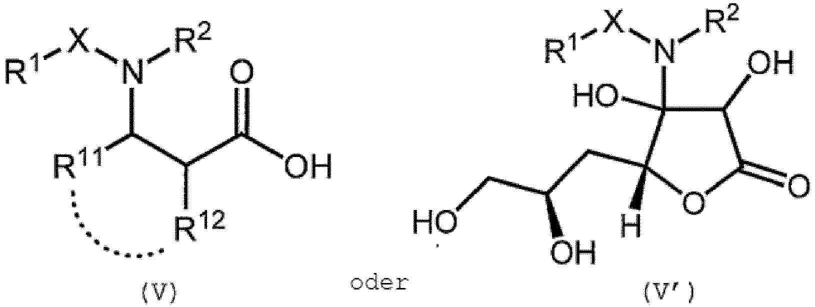 wobei R¹¹ und R¹² wie oben definiert sind; R¹ 5-(2-Fluorphenyl)-1-[(6-methylpyridin-3-yl)sulfonyl]-1H-pyrrol-3-yl, 4-Fluor-5-phenyl-1-(pyridin-3-ylsulfonyl)-1H-pyrrol-3-yl, 5-(4-Methyl-3-thienyl)-1-(pyridin-3-ylsulfonyl)-1H-pyrrol-3-yl, 5-(2-Fluorphenyl)-1-(pyridin-3-ylsulfonyl)-1H-pyrrol-3-yl, 5-(2-Methylphenyl)-1-(pyridin-3-ylsulfonyl)-1H-pyrrol-3-yl, 4-Fluor-5-(2-fluorpyridin-3-yl)-1-[(4-methylpyridin-2-yl)sulfonyl]-1H-pyrrol-3-yl, oder 4-Fluor-5-(2-fluorpyridin-3-yl)-1-(pyridin-3-ylsulfonyl)-1H-pyrrol-3-yl ist; R² ist Methyl; und X ist -CH₂-.

18. Verwendung eines Salzes als Stabilisator in einer flüssigen Zubereitung, die einen pharmazeutisch wirksamen Bestandteil und eine organische Säure umfasst,
wobei der Gehalt eines Reaktionsprodukts des pharmazeutisch wirksamen Bestandteils und der organischen Säure in der Zubereitung nicht mehr als 5% beträgt,
wobei der pharmazeutisch wirksame Bestandteil
1-{5-(2-Fluorphenyl)-1-[(6-methylpyridin-3-yl)sulfonyl]-1H-pyrrol-3-yl}-N-methylmethanamin,
1-[4-Fluor-5-phenyl-1-(pyridin-3-ylsulfonyl)-1H-pyrrol-3-yl]-N-methylmethanamin,
N-Methyl-1-[5-(4-methyl-3-thienyl)-1-(pyridin-3-ylsulfonyl)-1H-pyrrol-3-yl]methanamin,
1-[5-(2-Fluorphenyl)-1-(pyridin-3-ylsulfonyl)-1H-pyrrol-3-yl]-N-methylmethanamin,
N-Methyl-1-[5-(2-methylphenyl)-1-(pyridin-3-ylsulfonyl)-1H-pyrrol-3-yl]methanamin,
1-{4-Fluor-5-(2-fluorpyridin-3-yl)-1-[(4-methylpyridin-2-yl)sulfonyl]-1H-pyrrol-3-yl}-N-methylmethanamin, oder
1-[4-fluoro-5-(2-Fluoropyridin-3-yl)-1-(pyridin-3-ylsulfonyl)-1H-pyrrol-3-yl]-N-methylmethanamin, ist,
wobei die organische Säure eine Verbindung ist, die durch die Formel (IV) dargestellt wird: 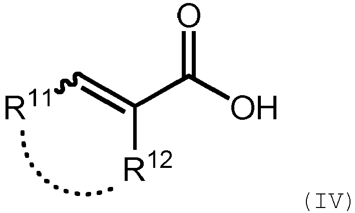 wobei R¹¹ und R¹² gleich oder verschieden sind und jeweils ein Wasserstoffatom, eine gegebenenfalls substituierte Kohlenwasserstoffgruppe, eine Carboxylgruppe, ein Halogenatom, eine C₁₋₆-Alkoxycarbonylgruppe oder eine C₁₋₆-Alkoxygruppe sind, oder R¹¹ und R¹² zusammen einen gegebenenfalls substituierten Ring bilden, oder Ascorbinsäure, und
wobei das Salz eine oder mehrere Arten ist, ausgewählt aus der Gruppe bestehend aus Natriumchlorid, Calciumchlorid, Magnesiumchlorid, Natriumbromid und Calciumbromid,
wobei das Reaktionsprodukt eine Verbindung ist, die durch die Formel (V) oder (V') dargestellt wird: 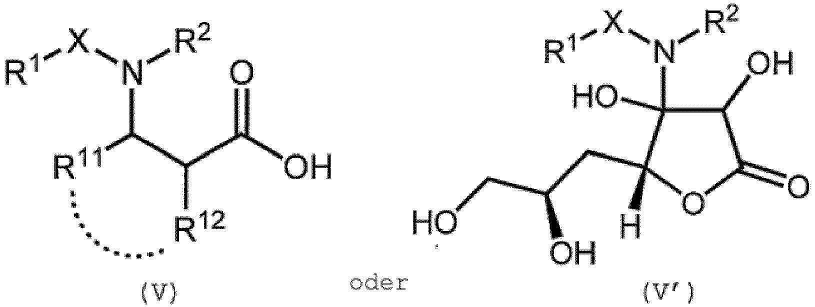 wobei R¹¹ und R¹² wie oben definiert sind; R¹ 5-(2-Fluorphenyl)-1-[(6-methylpyridin-3-yl)sulfonyl]-1H-pyrrol-3-yl, 4-Fluor-5-phenyl-1-(pyridin-3-ylsulfonyl)-1H-pyrrol-3-yl, 5-(4-Methyl-3-thienyl)-1-(pyridin-3-ylsulfonyl)-1H-pyrrol-3-yl, 5-(2-Fluorphenyl)-1-(pyridin-3-ylsulfonyl)-1H-pyrrol-3-yl, 5-(2-Methylphenyl)-1-(pyridin-3-ylsulfonyl)-1H-pyrrol-3-yl, 4-Fluor-5-(2-fluorpyridin-3-yl)-1-[(4-methylpyridin-2-yl)sulfonyl]-1H-pyrrol-3-yl, oder 4-Fluor-5-(2-fluorpyridin-3-yl)-1-(pyridin-3-ylsulfonyl)-1H-pyrrol-3-yl ist; R² ist Methyl; und X ist -CH₂-.

19. Verwendung eines Salzes zur Herstellung einer stabilisierten flüssigen Zubereitung, die einen pharmazeutisch wirksamen Bestandteil und eine organische Säure umfasst,
wobei der Gehalt eines Reaktionsprodukts des pharmazeutisch wirksamen Bestandteils und der organischen Säure in der Zubereitung nicht mehr als 5% beträgt,
wobei der pharmazeutisch wirksame Bestandteil
1-{5-(2-Fluorphenyl)-1-[(6-methylpyridin-3-yl)sulfonyl]-1H-pyrrol-3-yl}-N-methylmethanamin,
1-[4-Fluor-5-phenyl-1-(pyridin-3-ylsulfonyl)-1H-pyrrol-3-yl]-N-methylmethanamin,
N-Methyl-1-[5-(4-methyl-3-thienyl)-1-(pyridin-3-ylsulfonyl)-1H-pyrrol-3-yl]methanamin,
1-[5-(2-Fluorphenyl)-1-(pyridin-3-ylsulfonyl)-1H-pyrrol-3-yl]-N-methylmethanamin,
N-Methyl-1-[5-(2-methylphenyl)-1-(pyridin-3-ylsulfonyl)-1H-pyrrol-3-yl]methanamin,
1-{4-Fluor-5-(2-fluorpyridin-3-yl)-1-[(4-methylpyridin-2-yl)sulfonyl]-1H-pyrrol-3-yl}-N-methylmethanamin, oder
1-[4-Fluor-5-(2-fluorpyridin-3-yl)-1-(pyridin-3-ylsulfonyl)-1H-pyrrol-3-yl]-N-methylmethanamin, ist,
wobei die organische Säure eine Verbindung ist, die durch die Formel (IV) dargestellt wird: 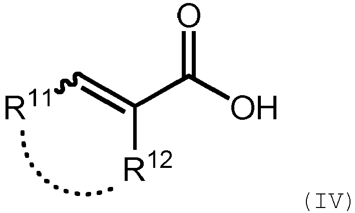 wobei R¹¹ und R¹² gleich oder verschieden sind und jeweils ein Wasserstoffatom, eine gegebenenfalls substituierte Kohlenwasserstoffgruppe, eine Carboxylgruppe, ein Halogenatom, eine C₁₋₆-Alkoxycarbonylgruppe oder eine C₁₋₆-Alkoxygruppe sind, oder R¹¹ und R¹² zusammen einen gegebenenfalls substituierten Ring bilden, oder Ascorbinsäure, und
wobei das Salz eine oder mehrere Arten ist, ausgewählt aus der Gruppe bestehend aus Natriumchlorid, Calciumchlorid, Magnesiumchlorid, Natriumbromid und Calciumbromid,
wobei das Reaktionsprodukt eine Verbindung ist, die durch die Formel (V) oder (V') dargestellt wird: 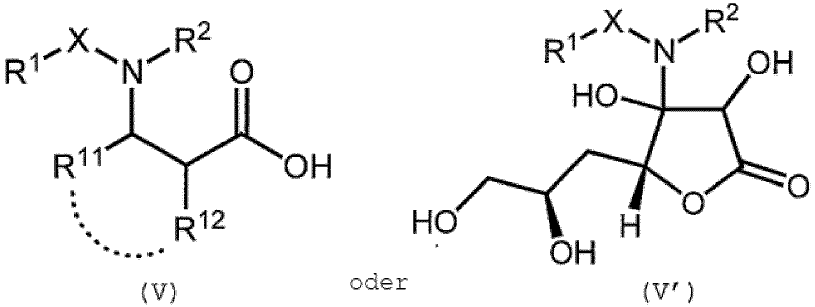 wobei R¹¹ und R¹² wie oben definiert sind; R¹ 5-(2-Fluorphenyl)-1-[(6-methylpyridin-3-yl)sulfonyl]-1H-pyrrol-3-yl, 4-Fluor-5-phenyl-1-(pyridin-3-ylsulfonyl)-1H-pyrrol-3-yl, 5-(4-Methyl-3-thienyl)-1-(pyridin-3-ylsulfonyl)-1H-pyrrol-3-yl, 5-(2-Fluorphenyl)-1-(pyridin-3-ylsulfonyl)-1H-pyrrol-3-yl, 5-(2-Methylphenyl)-1-(pyridin-3-ylsulfonyl)-1H-pyrrol-3-yl, 4-Fluor-5-(2-fluorpyridin-3-yl)-1-[(4-methylpyridin-2-yl)sulfonyl]-1H-pyrrol-3-yl, oder 4-Fluor-5-(2-fluorpyridin-3-yl)-1-(pyridin-3-ylsulfonyl)-1H-pyrrol-3-yl ist; R² ist Methyl; und X ist -CH₂-.

20. Eine flüssige Zubereitung, hergestellt aus einer Salzverbindung aus einer organischen Säure und einem pharmazeutisch wirksamen Bestandteil, und einem Salz als Ausgangsmaterialien, wobei die Menge eines Reaktionsprodukts des pharmazeutisch wirksamen Bestandteils und der freigesetzten organischen Säure durch das Salz unterdrückt wird,
wobei der pharmazeutisch wirksame Bestandteil 1-{5-(2-Fluorphenyl)-1-[(6-methylpyridin-3-yl)sulfonyl]-1H-pyrrol-3-yl}-N-methylmethanamin,
1-[4-Fluor-5-phenyl-1-(pyridin-3-ylsulfonyl)-1H-pyrrol-3-yl]-N-methylmethanamin,
N-Methyl-1-[5-(4-methyl-3-thienyl)-1-(pyridin-3-ylsulfonyl)-1H-pyrrol-3-yl]methanamin,
1-[5-(2-Fluorphenyl)-1-(pyridin-3-ylsulfonyl)-1H-pyrrol-3-yl]-N-methylmethanamin,
N-Methyl-1-[5-(2-methylphenyl)-1-(pyridin-3-ylsulfonyl)-1H-pyrrol-3-yl]methanamin,
1-{4-Fluor-5-(2-fluorpyridin-3-yl)-1-[(4-methylpyridin-2-yl)sulfonyl]-1H-pyrrol-3-yl}-N-methylmethanamin, oder
1-[4-Fluor-5-(2-fluorpyridin-3-yl)-1-(pyridin-3-ylsulfonyl)-1H-pyrrol-3-yl]-N-methylmethanamin, ist,
wobei die organische Säure eine Verbindung ist, die durch die Formel (IV) dargestellt wird: 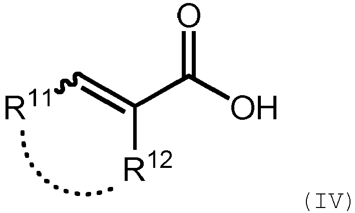 wobei R¹¹ und R¹² gleich oder verschieden sind und jeweils ein Wasserstoffatom, eine gegebenenfalls substituierte Kohlenwasserstoffgruppe, eine Carboxylgruppe, ein Halogenatom, eine C₁₋₆-Alkoxycarbonylgruppe oder eine C₁₋₆-Alkoxygruppe sind, oder R¹¹ und R¹² zusammen einen gegebenenfalls substituierten Ring bilden, oder Ascorbinsäure, und
wobei das Salz eine oder mehrere Arten ist, ausgewählt aus der Gruppe bestehend aus Natriumchlorid, Calciumchlorid, Magnesiumchlorid, Natriumbromid und Calciumbromid,
wobei das Reaktionsprodukt eine Verbindung ist, die durch die Formel (V) oder (V') dargestellt wird: 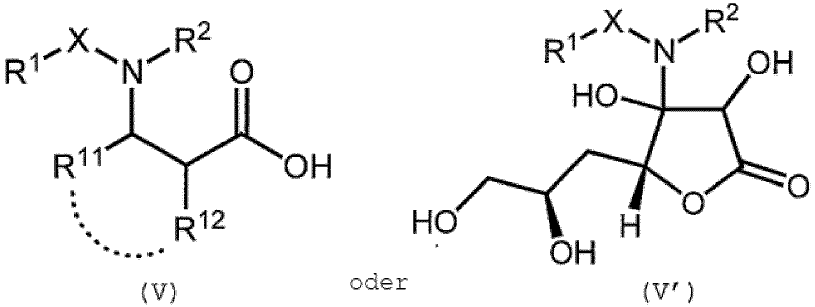 wobei R¹¹ und R¹² wie oben definiert sind; R¹ 5-(2-Fluorphenyl)-1-[(6-methylpyridin-3-yl)sulfonyl]-1H-pyrrol-3-yl, 4-Fluor-5-phenyl-1-(pyridin-3-ylsulfonyl)-1H-pyrrol-3-yl, 5-(4-Methyl-3-thienyl)-1-(pyridin-3-ylsulfonyl)-1H-pyrrol-3-yl, 5-(2-Fluorphenyl)-1-(pyridin-3-ylsulfonyl)-1H-pyrrol-3-yl, 5-(2-Methylphenyl)-1-(pyridin-3-ylsulfonyl)-1H-pyrrol-3-yl, 4-Fluor-5-(2-fluorpyridin-3-yl)-1-[(4-methylpyridin-2-yl)sulfonyl]-1H-pyrrol-3-yl, oder 4-Fluor-5-(2-fluorpyridin-3-yl)-1-(pyridin-3-ylsulfonyl)-1H-pyrrol-3-yl ist; R² ist Methyl; und X ist -CH₂-.

## Revendications

1. Préparation liquide comprenant un principe pharmaceutiquement actif, un acide organique et un sel, dans laquelle une teneur en un produit de réaction du principe pharmaceutiquement actif et de l'acide organique dans la préparation n'est pas supérieure à 5 %,
dans laquelle le principe pharmaceutiquement actif est la 1-{5-(2-fluorophényl)-1-[(6-méthylpyridin-3-yl)sulfonyl]-1H-pyrrol-3-yl}-N-méthylméthanamine,
la 1-[4-fluoro-5-phényl-1-(pyridin-3-ylsulfonyl)-1H-pyrrol-3-yl]-N-méthylméthanamine,
la N-méthyl-1-[5-(4-méthyl-3-thiényl)-1-(pyridin-3-ylsulfonyl)-1H-pyrrol-3-yl]méthanamine,
la 1-[5-(2-fluorophényl)-1-(pyridin-3-ylsulfonyl)-1H-pyrrol-3-yl]-N-méthylméthanamine,
la N-méthyl-1-[5-(2-méthylphényl)-1-(pyridin-3-ylsulfonyl)-1H-pyrrol-3-yl]méthanamine,
la 1-{4-fluoro-5-(2-fluoropyridin-3-yl)-1-[(4-méthylpyridin-2-yl)sulfonyl]-1H-pyrrol-3-yl}-N-méthylméthanamine, ou
la 1-[4-fluoro-5-(2-fluoropyridin-3-yl)-1-(pyridin-3-ylsulfonyl)-1H-pyrrol-3-yl]-N-méthylméthanamine,
dans laquelle l'acide organique est un composé représenté par la formule (IV) : 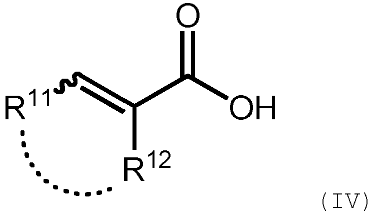 dans laquelle R¹¹ et R¹² sont identiques ou différents et sont chacun un atome d'hydrogène, un groupe hydrocarbure facultativement substitué, un groupe carboxyle, un atome d'halogène, un groupe (alcoxy en C₁₋₆)-carbonyle ou un groupe alcoxy en C₁₋₆, ou R¹¹ et R¹² forment ensemble un cycle facultativement substitué, ou un acide ascorbique, et
dans laquelle le sel est une ou plusieurs sortes sélectionnées dans le groupe consistant en le chlorure de sodium, le chlorure de calcium, le chlorure de magnésium, le bromure de sodium et le bromure de calcium,
dans laquelle le produit de réaction est un composé représenté par la formule (V) ou (V') : 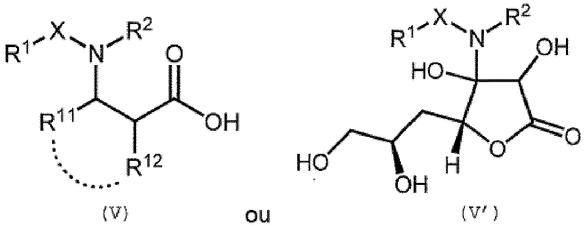 dans lesquelles R¹¹ et R¹² sont tels que définis ci-dessus ; R¹ est le 5-(2-fluorophényl)-1-[(6-méthylpyridin-3-yl)sulfonyl]-1H-pyrrol-3-yl, le 4-fluoro-5-phényl-1-(pyridin-3-ylsulfonyl)-1H-pyrrol-3-yl, le 5-(4-méthyl-3-thiényl)-1-(pyridin-3-ylsulfonyl)-1H-pyrrol-3-yl, le 5-(2-fluorophényl)-1-(pyridin-3-ylsulfonyl)-1H-pyrrol-3-yl, le 5-(2-méthylphényl)-1-(pyridin-3-ylsulfonyl)-1H-pyrrol-3-yl, le 4-fluoro-5-(2-fluoropyridin-3-yl)-1-[(4-méthylpyridin-2-yl)sulfonyl]-1H-pyrrol-3-yl, ou le 4-fluoro-5-(2-fluoropyridin-3-yl)-1-(pyridin-3-ylsulfonyl)-1H-pyrrol-3-yl ; R² est un méthyle ; et X est -CH₂-.

2. Préparation liquide selon la revendication 1, qui est une solution pour injection.

3. Préparation liquide selon la revendication 1, comprenant le produit de réaction du principe pharmaceutiquement actif et de l'acide organique à un facteur pas supérieur à 1,8 % après le stockage à 70 °C pendant une semaine par rapport à avant le stockage.

4. Préparation liquide selon la revendication 1, comprenant le produit de réaction du principe pharmaceutiquement actif et de l'acide organique à un facteur pas supérieur à 1,3 % après le stockage à 60 °C pendant une semaine par rapport à avant le stockage.

5. Procédé de production de la préparation liquide selon la revendication 1, comprenant une étape de dissolution ou de mise en suspension d'un sel d'acide organique du principe pharmaceutiquement actif, et du sel dans un solvant.

6. Préparation liquide selon la revendication 1, dans laquelle l'acide organique est une ou plusieurs sortes sélectionnées dans le groupe consistant en l'acide ascorbique, l'acide benzoïque, l'acide sorbique, l'acide fumarique et l'acide maléique.

7. Préparation liquide selon la revendication 1, dans laquelle le pH est un pH physiologiquement acceptable.

8. Préparation liquide selon la revendication 1, dans laquelle le pH est d'environ 3,0 à environ 5,0.

9. Préparation liquide selon la revendication 1, dans laquelle le principe pharmaceutiquement actif et l'acide organique sont contenus en un rapport molaire de 1:0,001 à 1:1 000.

10. Préparation liquide selon la revendication 1, dans laquelle le principe pharmaceutiquement actif et le sel sont contenus en un rapport molaire de 1:0,001 à 1:10 000.

11. Préparation liquide selon la revendication 1, dans laquelle le principe pharmaceutiquement actif a une concentration de 0,1 à 100 mg/ml.

12. Préparation liquide selon la revendication 1, pour l'utilisation dans la prophylaxie ou le traitement d'un ulcère gastrique accompagné d'une hémorragie, d'un ulcère duodénal, d'un ulcère de stress aigu ou d'une lésion aiguë de la muqueuse gastrique.

13. Préparation lyophilisée obtenue par lyophilisation de la préparation liquide selon la revendication 1.

14. Kit d'injection comprenant la solution pour injection selon la revendication 2 et une perfusion en combinaison.

15. Kit d'injection comprenant la préparation lyophilisée selon la revendication 13 et une perfusion en combinaison.

16. Procédé de stabilisation d'une préparation liquide, comprenant l'ajout d'un sel à une composition contenant un principe pharmaceutiquement actif et un acide organique,
dans laquelle le principe pharmaceutiquement actif est la 1-{5-(2-fluorophényl)-1-[(6-méthylpyridin-3-yl)sulfonyl]-1H-pyrrol-3-yl}-N-méthylméthanamine,
la 1-[4-fluoro-5-phényl-1-(pyridin-3-ylsulfonyl)-1H-pyrrol-3-yl]-N-méthylméthanamine,
la N-méthyl-1-[5-(4-méthyl-3-thiényl)-1-(pyridin-3-ylsulfonyl)-1H-pyrrol-3-yl]méthanamine,
la 1-[5-(2-fluorophényl)-1-(pyridin-3-ylsulfonyl)-1H-pyrrol-3-yl]-N-méthylméthanamine,
la N-méthyl-1-[5-(2-méthylphényl)-1-(pyridin-3-ylsulfonyl)-1H-pyrrol-3-yl]méthanamine,
la 1-{4-fluoro-5-(2-fluoropyridin-3-yl)-1-[(4-méthylpyridin-2-yl)sulfonyl]-1H-pyrrol-3-yl}-N-méthylméthanamine, ou la 1-[4-fluoro-5-(2-fluoropyridin-3-yl)-1-(pyridin-3-ylsulfonyl)-1H-pyrrol-3-yl]-N-méthylméthanamine,
dans laquelle l'acide organique est un composé représenté par la formule (IV) : 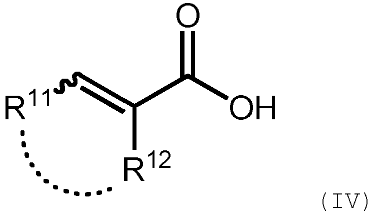 dans laquelle R¹¹ et R¹² sont identiques ou différents et sont chacun un atome d'hydrogène, un groupe hydrocarbure facultativement substitué, un groupe carboxyle, un atome d'halogène, un groupe (alcoxy en C₁₋₆)-carbonyle ou un groupe alcoxy en C₁₋₆, ou R¹¹ et R¹² forment ensemble un cycle facultativement substitué, ou un acide ascorbique, et
dans laquelle le sel est une ou plusieurs sortes sélectionnées dans le groupe consistant en le chlorure de sodium, le chlorure de calcium, le chlorure de magnésium, le bromure de sodium et le bromure de calcium.

17. Procédé de suppression de la production d'un produit de réaction d'un principe pharmaceutiquement actif et d'un acide organique, comprenant l'ajout d'un sel à une composition contenant le principe pharmaceutiquement actif et l'acide organique,
dans laquelle le principe pharmaceutiquement actif est la 1-{5-(2-fluorophényl)-1-[(6-méthylpyridin-3-yl)sulfonyl]-1H-pyrrol-3-yl}-N-méthylméthanamine,
la 1-[4-fluoro-5-phényl-1-(pyridin-3-ylsulfonyl)-1H-pyrrol-3-yl]-N-méthylméthanamine,
la N-méthyl-1-[5-(4-méthyl-3-thiényl)-1-(pyridin-3-ylsulfonyl)-1H-pyrrol-3-yl]méthanamine,
la 1-[5-(2-fluorophényl)-1-(pyridin-3-ylsulfonyl)-1H-pyrrol-3-yl]-N-méthylméthanamine,
la N-méthyl-1-[5-(2-méthylphényl)-1-(pyridin-3-ylsulfonyl)-1H-pyrrol-3-yl]méthanamine,
la 1-{4-fluoro-5-(2-fluoropyridin-3-yl)-1-[(4-méthylpyridin-2-yl)sulfonyl]-1H-pyrrol-3-yl}-N-méthylméthanamine, ou
la 1-[4-fluoro-5-(2-fluoropyridin-3-yl)-1-(pyridin-3-ylsulfonyl)-1H-pyrrol-3-yl]-N-méthylméthanamine,
dans laquelle l'acide organique est un composé représenté par la formule (IV) : 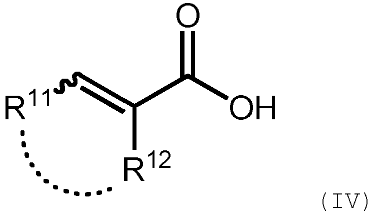 dans laquelle R¹¹ et R¹² sont identiques ou différents et sont chacun un atome d'hydrogène, un groupe hydrocarbure facultativement substitué, un groupe carboxyle, un atome d'halogène, un groupe (alcoxy en C₁₋₆)-carbonyle ou un groupe alcoxy en C₁₋₆, ou R¹¹ et R¹² forment ensemble un cycle facultativement substitué, ou un acide ascorbique, et
dans laquelle le sel est une ou plusieurs sortes sélectionnées dans le groupe consistant en le chlorure de sodium, le chlorure de calcium, le chlorure de magnésium, le bromure de sodium et le bromure de calcium,
dans laquelle le produit de réaction est un composé représenté par la formule (V) ou (V') : 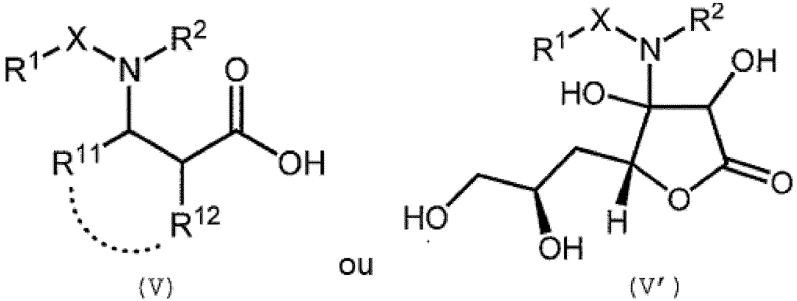 dans lesquelles R¹¹ et R¹² sont tels que définis ci-dessus ; R¹ est le 5-(2-fluorophényl)-1-[(6-méthylpyridin-3-yl)sulfonyl]-1H-pyrrol-3-yl, le 4-fluoro-5-phényl-1-(pyridin-3-ylsulfonyl)-1H-pyrrol-3-yl, le 5-(4-méthyl-3-thiényl)-1-(pyridin-3-ylsulfonyl)-1H-pyrrol-3-yl, le 5-(2-fluorophényl)-1-(pyridin-3-ylsulfonyl)-1H-pyrrol-3-yl, le 5-(2-méthylphényl)-1-(pyridin-3-ylsulfonyl)-1H-pyrrol-3-yl, le 4-fluoro-5-(2-fluoropyridin-3-yl)-1-[(4-méthylpyridin-2-yl)sulfonyl]-1H-pyrrol-3-yl, ou le 4-fluoro-5-(2-fluoropyridin-3-yl)-1-(pyridin-3-ylsulfonyl)-1H-pyrrol-3-yl ; R² est un groupe méthyle ; et X est -CH₂-.

18. Utilisation d'un sel en tant que stabilisant dans une préparation de liquide comprenant un principe pharmaceutiquement actif et un acide organique,
dans laquelle une teneur en un produit de réaction du principe pharmaceutiquement actif et de l'acide organique dans la préparation n'est pas supérieure à 5 %,
dans laquelle le principe pharmaceutiquement actif est la 1-{5-(2-fluorophényl)-1-[(6-méthylpyridin-3-yl)sulfonyl]-1H-pyrrol-3-yl}-N-méthylméthanamine,
la 1-[4-fluoro-5-phényl-1-(pyridin-3-ylsulfonyl)-1H-pyrrol-3-yl]-N-méthylméthanamine,
la N-méthyl-1-[5-(4-méthyl-3-thiényl)-1-(pyridin-3-ylsulfonyl)-1H-pyrrol-3-yl]méthanamine,
la 1-[5-(2-fluorophényl)-1-(pyridin-3-ylsulfonyl)-1H-pyrrol-3-yl]-N-méthylméthanamine,
la N-méthyl-1-[5-(2-méthylphényl)-1-(pyridin-3-ylsulfonyl)-1H-pyrrol-3-yl]méthanamine,
la 1-{4-fluoro-5-(2-fluoropyridin-3-yl)-1-[(4-méthylpyridin-2-yl)sulfonyl]-1H-pyrrol-3-yl}-N-méthylméthanamine, ou
la 1-[4-fluoro-5-(2-fluoropyridin-3-yl)-1-(pyridin-3-ylsulfonyl)-1H-pyrrol-3-yl]-N-méthylméthanamine,
dans laquelle l'acide organique est un composé représenté par la formule (IV) : 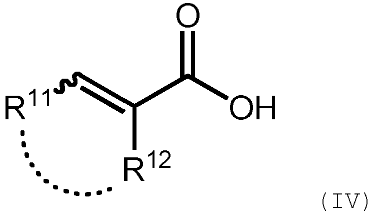 dans laquelle R¹¹ et R¹² sont identiques ou différents et sont chacun un atome d'hydrogène, un groupe hydrocarbure facultativement substitué, un groupe carboxyle, un atome d'halogène, un groupe (alcoxy en C₁₋₆)-carbonyle ou un groupe alcoxy en C₁₋₆, ou R¹¹ et R¹² forment ensemble un cycle facultativement substitué, ou un acide ascorbique, et
dans laquelle le sel est une ou plusieurs sortes sélectionnées dans le groupe consistant en le chlorure de sodium, le chlorure de calcium, le chlorure de magnésium, le bromure de sodium et le bromure de calcium,
dans laquelle le produit de réaction est un composé représenté par la formule (V) ou (V') : 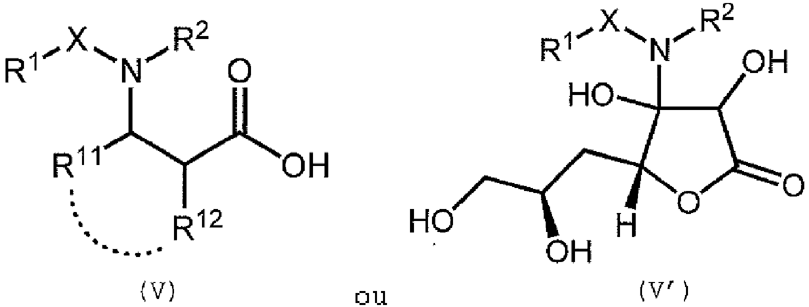 dans lesquelles R¹¹ et R¹² sont tels que définis ci-dessus ; R¹ est le 5-(2-fluorophényl)-1-[(6-méthylpyridin-3-yl)sulfonyl]-1H-pyrrol-3-yl, le 4-fluoro-5-phényl-1-(pyridin-3-ylsulfonyl)-1H-pyrrol-3-yl, le 5-(4-méthyl-3-thiényl)-1-(pyridin-3-ylsulfonyl)-1H-pyrrol-3-yl, le 5-(2-fluorophényl)-1-(pyridin-3-ylsulfonyl)-1H-pyrrol-3-yl, le 5-(2-méthylphényl)-1-(pyridin-3-ylsulfonyl)-1H-pyrrol-3-yl, le 4-fluoro-5-(2-fluoropyridin-3-yl)-1-[(4-méthylpyridin-2-yl)sulfonyl]-1H-pyrrol-3-yl, ou le 4-fluoro-5-(2-fluoropyridin-3-yl)-1-(pyridin-3-ylsulfonyl)-1H-pyrrol-3-yl ; R² est un méthyle ; et X est un -CH₂-.

19. Utilisation d'un sel pour la production d'une préparation liquide stabilisée comprenant un principe pharmaceutiquement actif et un acide organique,
dans laquelle une teneur en un produit de réaction du principe pharmaceutiquement actif et de l'acide organique dans la préparation n'est pas supérieure à 5 %,
dans laquelle le principe pharmaceutiquement actif est la 1-{5-(2-fluorophényl)-1-[(6-méthylpyridin-3-yl)sulfonyl]-1H-pyrrol-3-yl}-N-méthylméthanamine,
la 1-[4-fluoro-5-phényl-1-(pyridin-3-ylsulfonyl)-1H-pyrrol-3-yl]-N-méthylméthanamine,
la N-méthyl-1-[5-(4-méthyl-3-thiényl)-1-(pyridin-3-ylsulfonyl)-1H-pyrrol-3-yl]méthanamine,
la 1-[5-(2-fluorophényl)-1-(pyridin-3-ylsulfonyl)-1H-pyrrol-3-yl]-N-méthylméthanamine,
la N-méthyl-1-[5-(2-méthylphényl)-1-(pyridin-3-ylsulfonyl)-1H-pyrrol-3-yl]méthanamine,
la 1-{4-fluoro-5-(2-fluoropyridin-3-yl)-1-[(4-méthylpyridin-2-yl)sulfonyl]-1H-pyrrol-3-yl}-N-méthylméthanamine, ou
la 1-[4-fluoro-5-(2-fluoropyridin-3-yl)-1-(pyridin-3-ylsulfonyl)-1H-pyrrol-3-yl]-N-méthylméthanamine,
dans laquelle l'acide organique est un composé représenté par la formule (IV) : 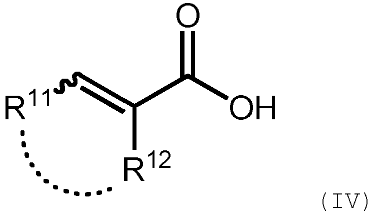 dans laquelle R¹¹ et R¹² sont identiques ou différents et sont chacun un atome d'hydrogène, un groupe hydrocarbure facultativement substitué, un groupe carboxyle, un atome d'halogène, un groupe (alcoxy en C₁₋₆)-carbonyle ou un groupe alcoxy en C₁₋₆, ou R¹¹ et R¹² forment ensemble un cycle facultativement substitué, ou un acide ascorbique, et
dans laquelle le sel est une ou plusieurs sortes sélectionnées dans le groupe consistant en le chlorure de sodium, le chlorure de calcium, le chlorure de magnésium, le bromure de sodium et le bromure de calcium,
dans laquelle le produit de réaction est un composé représenté par la formule (V) ou (V') : 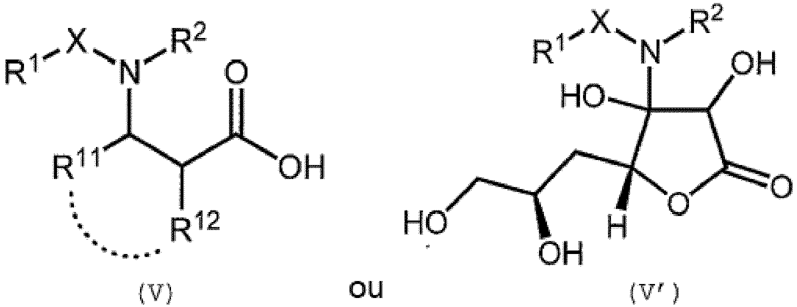 dans lesquelles R¹¹ et R¹² sont tels que définis ci-dessus ; R¹ est le 5-(2-fluorophényl)-1-[(6-méthylpyridin-3-yl)sulfonyl]-1H-pyrrol-3-yl, le 4-fluoro-5-phényl-1-(pyridin-3-ylsulfonyl)-1H-pyrrol-3-yl, le 5-(4-méthyl-3-thiényl)-1-(pyridin-3-ylsulfonyl)-1H-pyrrol-3-yl, le 5-(2-fluorophényl)-1-(pyridin-3-ylsulfonyl)-1H-pyrrol-3-yl, le 5-(2-méthylphényl)-1-(pyridin-3-ylsulfonyl)-1H-pyrrol-3-yl, le 4-fluoro-5-(2-fluoropyridin-3-yl)-1-[(4-méthylpyridin-2-yl)sulfonyl]-1H-pyrrol-3-yl, ou le 4-fluoro-5-(2-fluoropyridin-3-yl)-1-(pyridin-3-ylsulfonyl)-1H-pyrrol-3-yl ; R² est un méthyle ; et X est -CH₂-.

20. Préparation liquide produite à partir d'un composé de sel d'acide organique d'un principe pharmaceutiquement actif, et d'un sel en tant que matières premières, dans laquelle la quantité d'un produit de réaction du principe pharmaceutiquement actif et de l'acide organique libéré est supprimée par le sel,
dans laquelle le principe pharmaceutiquement actif est la 1-{5-(2-fluorophényl)-1-[(6-méthylpyridin-3-yl)sulfonyl]-1H-pyrrol-3-yl}-N-méthylméthanamine,
la 1-[4-fluoro-5-phényl-1-(pyridin-3-ylsulfonyl)-1H-pyrrol-3-yl]-N-méthylméthanamine,
la N-méthyl-1-[5-(4-méthyl-3-thiényl)-1-(pyridin-3-ylsulfonyl)-1H-pyrrol-3-yl]méthanamine,
la 1-[5-(2-fluorophényl)-1-(pyridin-3-ylsulfonyl)-1H-pyrrol-3-yl]-N-méthylméthanamine,
la N-méthyl-1-[5-(2-méthylphényl)-1-(pyridin-3-ylsulfonyl)-1H-pyrrol-3-yl]méthanamine,
la 1-{4-fluoro-5-(2-fluoropyridin-3-yl)-1-[(4-méthylpyridin-2-yl)sulfonyl]-1H-pyrrol-3-yl}-N-méthylméthanamine, ou
la 1-[4-fluoro-5-(2-fluoropyridin-3-yl)-1-(pyridin-3-ylsulfonyl)-1H-pyrrol-3-yl]-N-méthylméthanamine,
dans laquelle l'acide organique est un composé représenté par la formule (IV) : 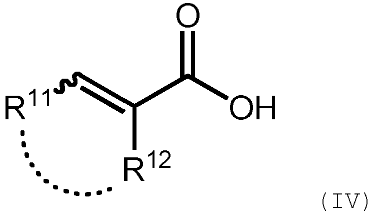 dans laquelle R¹¹ et R¹² sont identiques ou différents et sont chacun un atome d'hydrogène, un groupe hydrocarbure facultativement substitué, un groupe carboxyle, un atome d'halogène, un groupe (alcoxy en C₁₋₆)-carbonyle ou un groupe alcoxy en C₁₋₆, ou R¹¹ et R¹² forment ensemble un cycle facultativement substitué, ou un acide ascorbique, et
dans laquelle le sel est une ou plusieurs sortes sélectionnées dans le groupe consistant en le chlorure de sodium, le chlorure de calcium, le chlorure de magnésium, le bromure de sodium et le bromure de calcium,
dans laquelle le produit de réaction est un composé représenté par la formule (V) ou (V') : 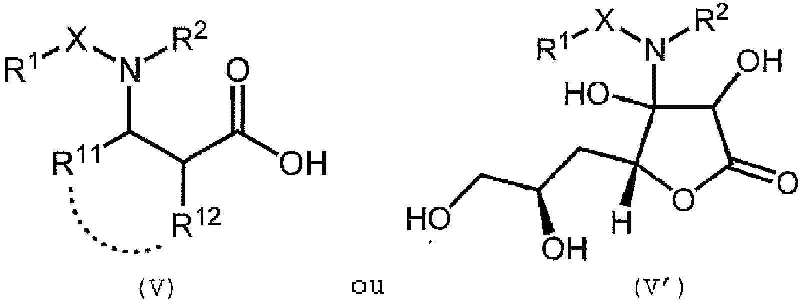 dans lesquelles R¹¹ et R¹² sont tels que définis ci-dessus ; R¹ est le 5-(2-fluorophényl)-1-[(6-méthylpyridin-3-yl)sulfonyl]-1H-pyrrol-3-yl, le 4-fluoro-5-phényl-1-(pyridin-3-ylsulfonyl)-1H-pyrrol-3-yl, le 5-(4-méthyl-3-thiényl)-1-(pyridin-3-ylsulfonyl)-1H-pyrrol-3-yl, le 5-(2-fluorophényl)-1-(pyridin-3-ylsulfonyl)-1H-pyrrol-3-yl, le 5-(2-méthylphényl)-1-(pyridin-3-ylsulfonyl)-1H-pyrrol-3-yl, 4-fluoro-5-(2-fluoropyridin-3-yl)-1-[(4-méthylpyridin-2-yl)sulfonyl]-1H-pyrrol-3-yl, ou le 4-fluoro-5-(2-fluoropyridin-3-yl)-1-(pyridin-3-ylsulfonyl)-1H-pyrrol-3-yl ; R² est un méthyle ; et X est -CH₂-.
